(19) Europäisches Patentamt
European Patent Office
Office européen des brevets

(11) EP 2 906 252 B1

(12) **EUROPEAN PATENT SPECIFICATION**

(45) Date of publication and mention of the grant of the patent:
**14.06.2017 Bulletin 2017/24**

(21) Application number: **13788691.7**

(22) Date of filing: **11.10.2013**

(51) Int Cl.:
***A61K 47/68*** (2017.01) ***A61P 35/00*** (2006.01)
*C07K 16/32* (2006.01) *A61K 39/395* (2006.01)
*A61K 31/551* (2006.01)

(86) International application number:
**PCT/EP2013/071343**

(87) International publication number:
**WO 2014/057115 (17.04.2014 Gazette 2014/16)**

(54) **PYRROLOBENZODIAZEPINE-ANTI-HER2 ANTIBODY CONJUGATES**

PYRROLOBENZODIAZEPIN-ANTI-HER2-ANTIKÖRPERKONJUGATE

CONJUGUÉS ANTICORPS ANTI-HER2 - PYRROLOBENZODIAZÉPINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.10.2012 US 201261712924 P**
**12.10.2012 US 201261712928 P**
**14.03.2013 US 201361784270 P**
**14.03.2013 US 201361784233 P**
**14.03.2013 US 201361784249 P**

(43) Date of publication of application:
**19.08.2015 Bulletin 2015/34**

(73) Proprietors:
- **ADC Therapeutics SA**
**1066 Epalinges (CH)**
- **Medimmune Limited**
**Cambridge, Cambridgeshire CB21 6GH (GB)**

(72) Inventors:
- **VAN BERKEL, Patricius Hendrikus Cornelis**
**1003 Lausanne (CH)**
- **HOWARD, Philip Wilson**
**London**
**Greater London E1 2AX (GB)**

(74) Representative: **Watson, Robert James et al**
**Mewburn Ellis LLP**
**City Tower**
**40 Basinghall Street**
**London EC2V 5DE (GB)**

(56) References cited:
**WO-A1-2010/151793** **WO-A1-2011/130598**
**WO-A1-2011/130613** **WO-A1-2011/130616**
**WO-A1-2013/055987** **WO-A1-2013/177481**
**WO-A2-2011/137245** **WO-A2-2012/153193**
**US-A1- 2008 112 961**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 906 252 B1

## Description

The present invention relates to pyrrolobenzodiazepines (PBDs) having a labile C2 or N10 protecting group in the form of a linker to an antibody.

## Background to the invention

*Pyrrolobenzodiazepines*

Some pyrrolobenzodiazepines (PBDs) have the ability to recognise and bond to specific sequences of DNA; the preferred sequence is PuGPu. The first PBD antitumour antibiotic, anthramycin, was discovered in 1965 (Leimgruber, et al., J. Am. Chem. Soc., 87, 5793-5795 (1965); Leimgruber, et al., J. Am. Chem. Soc., 87, 5791-5793 (1965)). Since then, a number of naturally occurring PBDs have been reported, and over 10 synthetic routes have been developed to a variety of analogues (Thurston, et al., Chem. Rev. 1994, 433-465 (1994); Antonow, D. and Thurston, D.E., Chem. Rev. 2011 111 (4), 2815-2864). Family members include abbeymycin (Hochlowski, et al., J. Antibiotics, 40, 145-148 (1987)), chicamycin (Konishi, et al., J. Antibiotics, 37, 200-206 (1984)), DC-81 (Japanese Patent 58-180 487; Thurston, et al., Chem. Brit., 26, 767-772 (1990); Bose, et al., Tetrahedron, 48, 751-758 (1992)), mazethramycin (Kuminoto, et al., J. Antibiotics, 33, 665-667 (1980)), neothramycins A and B (Takeuchi, et al., J. Antibiotics, 29, 93-96 (1976)), porothramycin (Tsunakawa, et al., J. Antibiotics, 41, 1366-1373 (1988)), prothracarcin (Shimizu, et al, J. Antibiotics, 29, 2492-2503 (1982); Langley and Thurston, J. Org. Chem., 52, 91-97 (1987)), sibanomicin (DC-102)(Hara, et al., J. Antibiotics, 41, 702-704 (1988); Itoh, et al., J. Antibiotics, 41, 1281-1284 (1988)), sibiromycin (Leber, et al., J. Am. Chem. Soc., 110, 2992-2993 (1988)) and tomamycin (Arima, et al., J. Antibiotics, 25, 437-444 (1972)). PBDs are of the general structure:

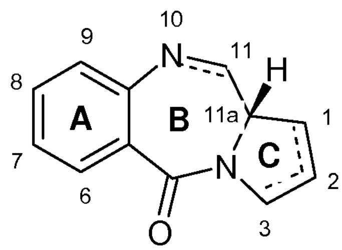

They differ in the number, type and position of substituents, in both their aromatic A rings and pyrrolo C rings, and in the degree of saturation of the C ring. In the B-ring there is either an imine (N=C), a carbinolamine(NH-CH(OH)), or a carbinolamine methyl ether (NH-CH(OMe)) at the N10-C11 position which is the electrophilic centre responsible for alkylating DNA. All of the known natural products have an (*S*)-configuration at the chiral C11a position which provides them with a right-handed twist when viewed from the C ring towards the A ring. This gives them the appropriate three-dimensional shape for isohelicity with the minor groove of B-form DNA, leading to a snug fit at the binding site (Kohn, In Antibiotics III. Springer-Verlag, New York, pp. 3-11 (1975); Hurley and Needham-VanDevanter, Acc. Chem. Res., 19, 230-237 (1986)). Their ability to form an adduct in the minor groove, enables them to interfere with DNA processing, hence their use as antitumour agents.

A particularly advantageous pyrrolobenzodiazepine compound is described by Gregson et al. (Chem. Commun. 1999, 797-798) as compound 1, and by Gregson et al. (J. Med. Chem. 2001, 44, 1161-1174) as compound **4a.** This compound, also known as SG2000, is shown below:

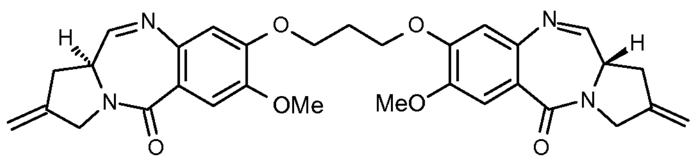

**SG2000**

.

WO 2007/085930 describes the preparation of dimer PBD compounds having linker groups for connection to a cell binding agent, such as an antibody. The linker is present in the bridge linking the monomer PBD units of the dimer.

The present inventors have described dimer PBD compounds having linker groups for connection to a cell binding agent, such as an antibody, in WO 2011/130613 and WO 2011/130616. The linker in these compounds is

attached to the PBD core via the C2 position, and are generally cleaved by action of an enzyme on the linker group. In WO 2011/130598, the linker in these compounds is attached to one of the available N10 positions on the PBD core, and are generally cleaved by action of an enzyme on the linker group.

*Antibody-drug conjugates*

Antibody therapy has been established for the targeted treatment of patients with cancer, immunological and angiogenic disorders (Carter, P. (2006) Nature Reviews Immunology 6:343-357). The use of antibody-drug conjugates (ADC), i.e. immunoconjugates, for the local delivery of cytotoxic or cytostatic agents, i.e. drugs to kill or inhibit tumor cells in the treatment of cancer, targets delivery of the drug moiety to tumors, and intracellular accumulation therein, whereas systemic administration of these unconjugated drug agents may result in unacceptable levels of toxicity to normal cells (Xie et al (2006) Expert. Opin. Biol. Ther. 6(3):281-291; Kovtun et al (2006) Cancer Res. 66(6):3214-3121; Law et al (2006) Cancer Res. 66(4):2328-2337; Wu et al (2005) Nature Biotech. 23(9):1137-1145; Lambert J. (2005) Current Opin. in Pharmacol. 5:543-549; Hamann P. (2005) Expert Opin. Ther. Patents 15(9):1087-1103; Payne, G. (2003) Cancer Cell 3:207-212; Trail et al (2003) Cancer Immunol. Immunother. 52:328-337; Syrigos and Epenetos (1999) Anticancer Research 19:605-614).

Maximal efficacy with minimal toxicity is sought thereby. Efforts to design and refine ADC have focused on the selectivity of monoclonal antibodies (mAbs) as well as drug mechanism of action, drug-linking, drug/antibody ratio (loading), and drug-releasing properties (Junutula, et al., 2008b Nature Biotech., 26(8):925-932; Dornan et al (2009) Blood 114(13):2721-2729; US 7521541; US 7723485; WO2009/052249; McDonagh (2006) Protein Eng. Design & Sel. 19(7): 299-307; Doronina et al (2006) Bioconj. Chem. 17:114-124; Erickson et al (2006) Cancer Res. 66(8):1-8; Sanderson et al (2005) Clin. Cancer Res. 11:843-852; Jeffrey et al (2005) J. Med. Chem. 48:1344-1358; Hamblett et al (2004) Clin. Cancer Res. 10:7063-7070). Drug moieties may impart their cytotoxic and cytostatic effects by mechanisms including tubulin binding, DNA binding, proteasome and/or topoisomerase inhibition. Some cytotoxic drugs tend to be inactive or less active when conjugated to large antibodies or protein receptor ligands.

The present inventors have developed particular PBD dimer antibody conjugates.

**Disclosure of the Invention**

A first aspect of the present invention comprises a conjugate of formula

ConjA

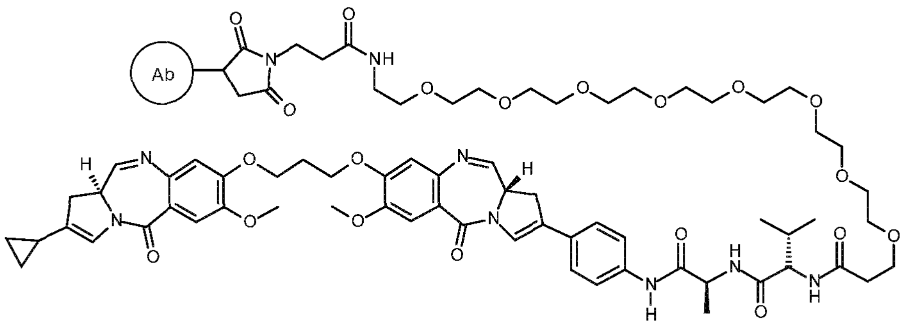

ConjA ;

ConjB

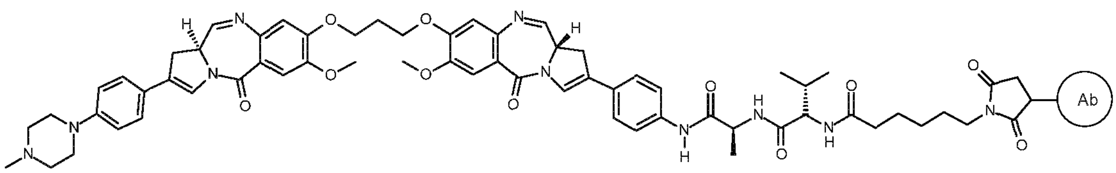

ConjB ;

ConjC:

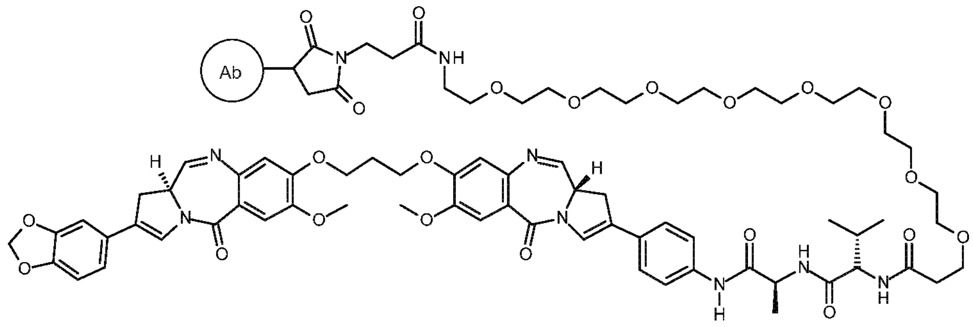

ConjC ;

ConjD

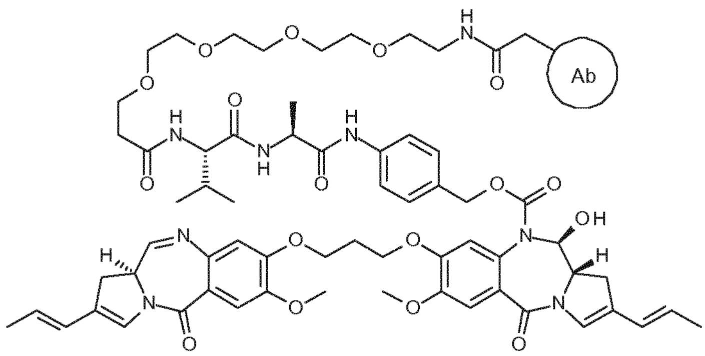

ConjD ;

or
ConjE:

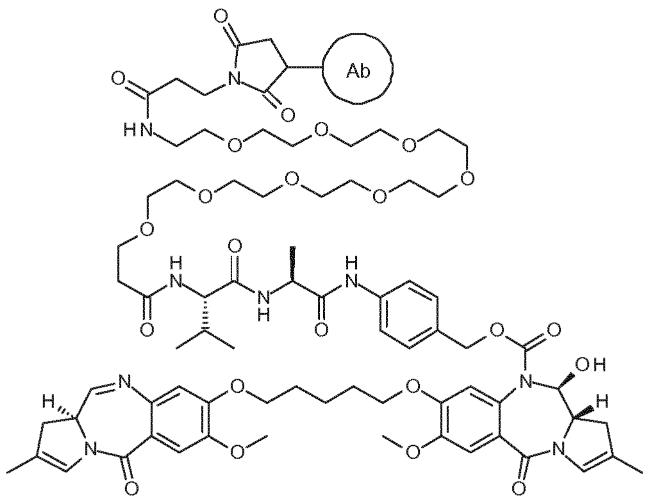

ConjE .

where Ab is an antibody that binds to HER2, the antibody comprising a VH domain having the sequence according to SEQ ID NO. 1; and
wherein the drug loading (p) of drugs (D) to antibody (Ab) is an integer from 1 to about 8

There is provided a method of making a conjugate selected from the group consisting of ConjA, ConjB, ConjC, ConjD and ConjE comprising conjugating a compound which is selected respectively from A:

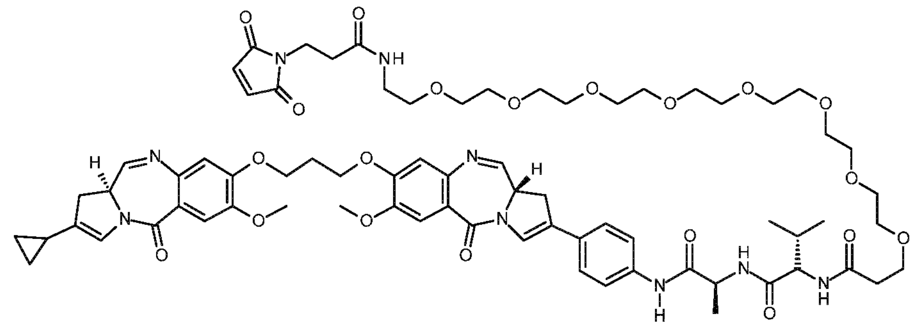

A

B:

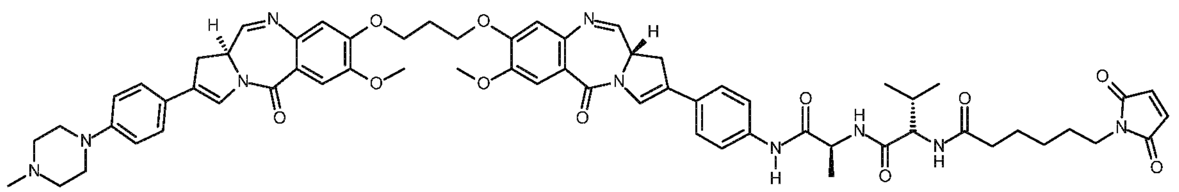

B

C:

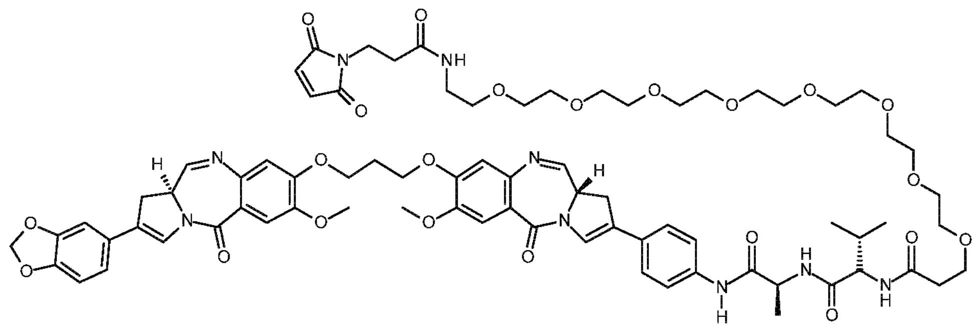

C

D:

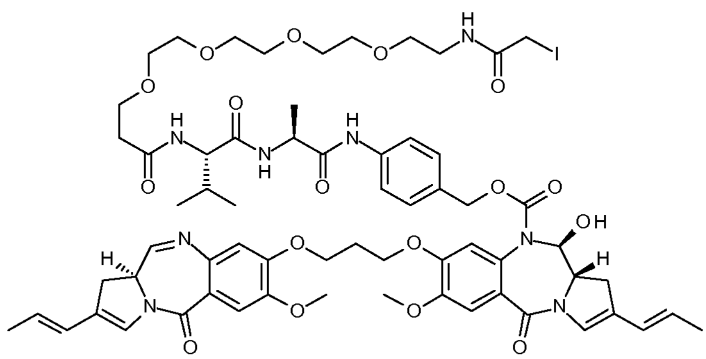

D

and E:

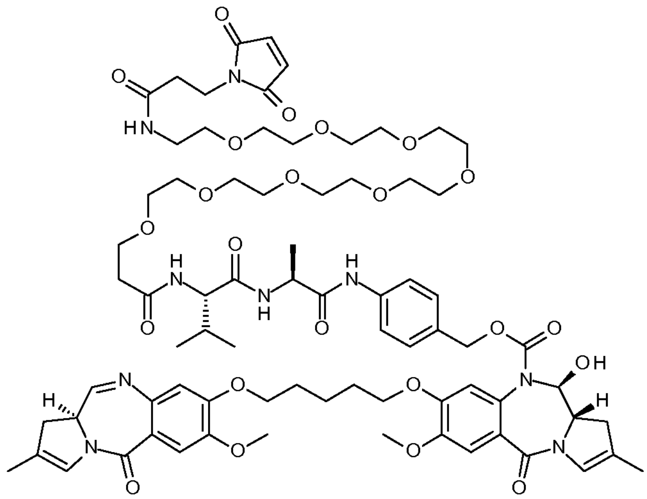

E

with an antibody as defined below.

WO 2011/130615 discloses compound 26:

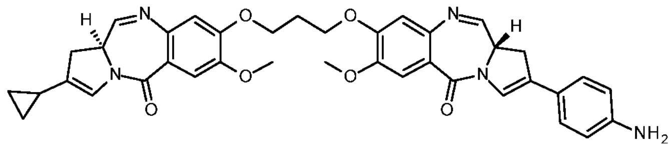

26

which is the parent compound of A. Compound A comprises this PBD with a linker for attachment to a cell binding agent. The cell binding agent provides a number of ethylene glycol moieties to provide solubility which is useful in the synthesis of conjugates.

WO 2010/043380 and WO 2011/130613 disclose compound 30:

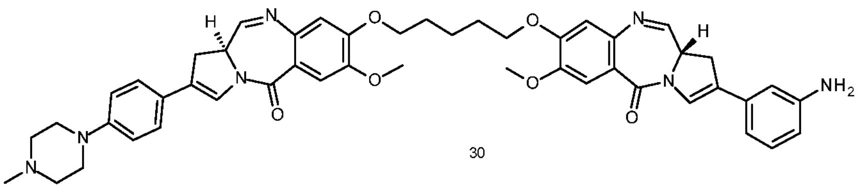

30

WO 2011/130613 also discloses compound 51:

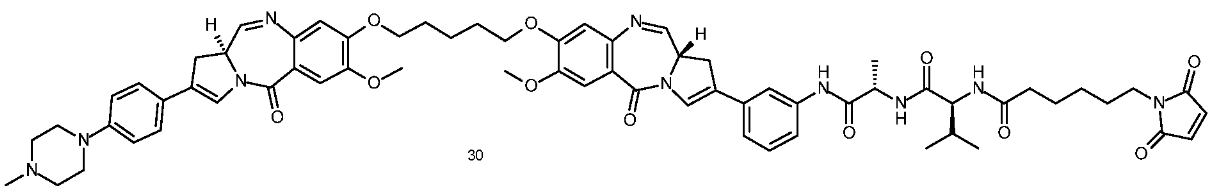

30

Compound B differs from compound 30 by only having a $(CH_2)_3$ tether between the PBD moieties, instead of a $(CH_2)_5$ tether, which reduces the lipophilicity of the released PBD dimer. The linking group is attached to the C2-phenyl group in the para rather than meta position.

WO 2011/130613 discloses compound 93:

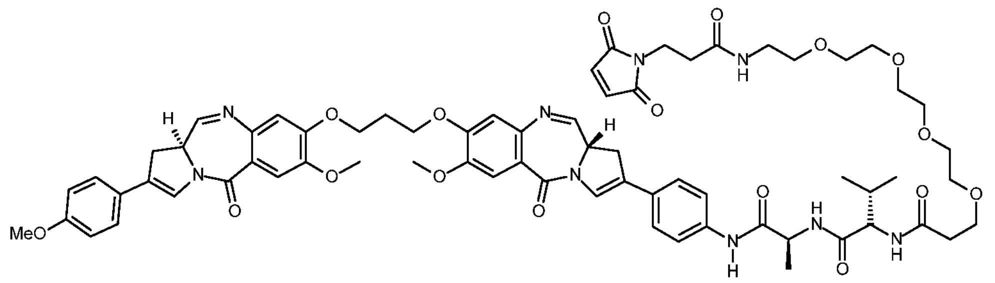

93

Compound C differs from this in two respects. The cell binding agent provides an increased number of ethylene glycol moieties to provide solubility which is useful in the synthesis of conjugates, and the phenyl substiuent provide two rather than one oxygen atom, which also aids solubility. Compound C's strucutre may also mean it binds more strongly in the minor groove.

Compounds A, B and C have two $sp^2$ centres in each C-ring, which may allow for stronger binding in the minor groove of DNA, than for compounds with only one $sp^2$ centre in each C-ring.

WO 2011/130598 discloses compound 80:

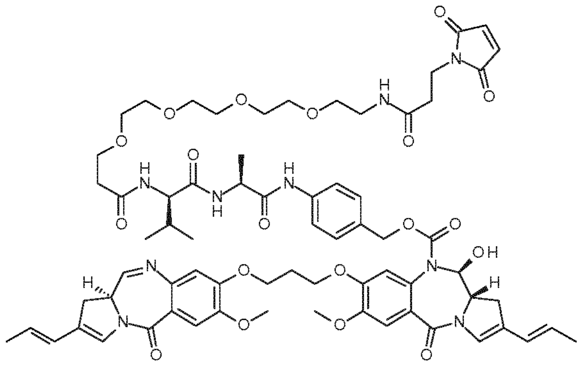

80

.

Compound D differs from this by comprising an iodoacetamide group for linking to the cell binding agent. This group may offer advantages over compound 80 with regards to its stability when bound to a cell binding agent (see below). The malemide group in compound 80 can undergo a retro-Michael reaction, becoming unconjugated from the cell binding agent, and thus vunerable to scavenging by other thiol containing biological molecules, such as albumin and glutathione. Such unconjugation cannot occur with compound A. Also, the iodoacetamide group may avoid other unwanted side reactions.

Compound E differs from previously disclosed PBD dimers with a drug linker having a C2-3 endo-double bond, by having a smaller, less lipophilic C2 substituent, e.g. 4F-phenyl, propylene. As such, the conjugates of compound B (see below) are less likely to aggregate once synthesised. Such aggregation of conjugates can be measured by Size exclusion chromatography (SEC).

Both compound D and E have two $sp^2$ centres in each C-ring, which may allow for stronger binding in the minor groove of DNA, than for compounds with only one $sp^2$ centre in each C-ring.

**Detailed description of the invention**

The present invention is suitable for use in providing a PBD compound to a preferred site in a subject. The conjugate allows the release of an active PBD compound that does not retain any part of the linker. There is no stub present that could affect the reactivity of the PBD compound. Thus ConjA would release the compound RelA:

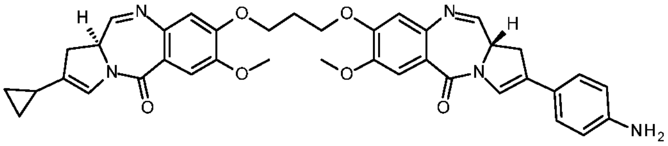

RelA

ConjB would release the compound RelB:

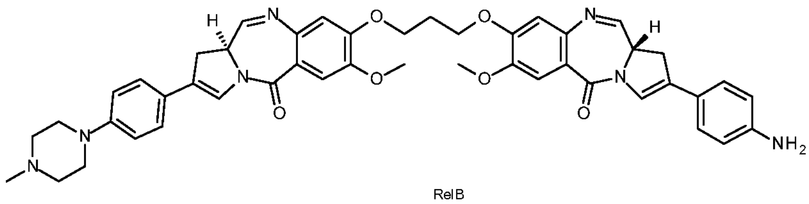

RelB

ConjC would release the compound RelC:

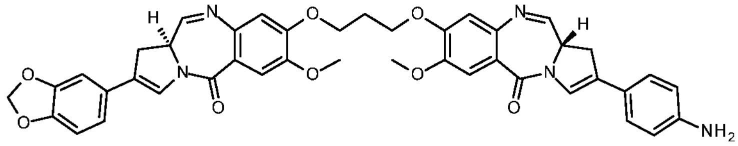

RelC

ConjD would release the compound RelD:

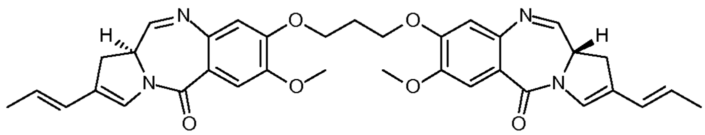

RelD

and ConjE would release the compound RelE:

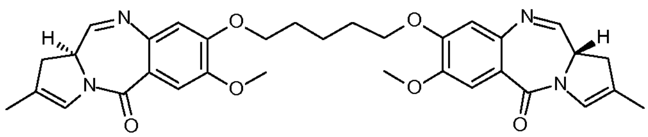

RelE

The speficied link between the PBD dimer and the antibody, in the present invention is preferably stable extracellularly. Before transport or delivery into a cell, the antibody-drug conjugate (ADC) is preferably stable and remains intact, i.e. the antibody remains linked to the drug moiety. The linkers are stable outside the target cell and may be cleaved at some efficacious rate inside the cell. An effective linker will: (i) maintain the specific binding properties of the antibody; (ii) allow intracellular delivery of the conjugate or drug moiety; (iii) remain stable and intact, i.e. not cleaved, until the conjugate has been delivered or transported to its targetted site; and (iv) maintain a cytotoxic, cell-killing effect or a cytostatic effect of the PBD drug moiety. Stability of the ADC may be measured by standard analytical techniques such as mass spectroscopy, HPLC, and the separation/analysis technique LC/MS.

Delivery of the compounds of formulae RelA, RelB, RelC, RelD or RelE is achieved at the desired activation site of the conjugates of formulae ConjA, ConjB, ConjC, ConjD or ConjE by the action of an enzyme, such as cathepsin, on the linking group, and in particular on the valine-alanine dipeptide moiety.

## Antibody

In one aspect the antibody is an antibody that binds to HER2, the antibody comprising a VH domain having the sequence according to SEQ ID NO. 1.

The antibody may further comprise a VL domain. In some embodiments the antibody further comprises a VL domain having the sequence according to SEQ ID NO. 2.

In some embodiments the antibody comprises a VH domain paired with a VL domain, the VH and VL domains having the sequences of SEQ ID NO. 1 paired with SEQ ID NO. 2.

The VH and VL domain(s) may pair so as to form an antibody antigen binding site that binds HER2.

In some embodiments the antibody is an intact antibody comprising a VH domain paired with a VL domain, the VH and VL domains having sequences of SEQ ID NO. 1 paired with SEQ ID NO. 2. In one embodiment the antibody comprises a heavy chain having the sequence of SEQ ID NO. 3 paired with a light chain having the sequence of SEQ ID NO. 4. In one embodiment the antibody is an intact antibody comprising two heavy chains having the sequence of SEQ ID NO. 3, each paired with a light chain having the sequence of SEQ ID NO. 4.

In some embodiments, the antibody competes with the antibody secreted by hybridoma ATCC accession No. CRL-10463 for binding to HER2. In one embodiment the antibody binds HER2 with an association constant ($K_a$) no less than 2, 5 or 10-fold less than the antibody secreted by the hybridoma.

In one aspect the antibody is the antibody secreted by a hydridoma. In one embodiment the hybridoma is ATCC accession No. CRL-10463.

In aspect the antibody is an antibody as described herein which has been modified (or further modified) as described below. In some embodiments the antibody is a humanised, deimmunised or resurfaced version of an antibody disclosed herein.

Terminology

The term "antibody" herein is used in the broadest sense and specifically covers monoclonal antibodies, polyclonal antibodies, dimers, multimers, multispecific antibodies (*e.g.*, bispecific antibodies), intact antibodies and antibody fragments, so long as they exhibit the desired biological activity, for example, the ability to bind HER2 (Miller et al (2003) Jour. of Immunology 170:4854-4861). Antibodies may be murine, human, humanized, chimeric, or derived from other species. An antibody is a protein generated by the immune system that is capable of recognizing and binding to a specific antigen. (Janeway, C., Travers, P., Walport, M., Shlomchik (2001) Immuno Biology, 5th Ed., Garland Publishing, New York). A target antigen generally has numerous binding sites, also called epitopes, recognized by CDRs on multiple antibodies. Each antibody that specifically binds to a different epitope has a different structure. Thus, one antigen may have more than one corresponding antibody. An antibody includes a full-length immunoglobulin molecule or an immunologically active portion of a full-length immunoglobulin molecule, *i.e.*, a molecule that contains an antigen binding site that immunospecifically binds an antigen of a target of interest or part thereof, such targets including but not limited to, cancer cell or cells that produce autoimmune antibodies associated with an autoimmune disease. The immunoglobulin can be of any type (e.g. IgG, IgE, IgM, IgD, and IgA), class (e.g. IgG1, IgG2, IgG3, IgG4, IgA1 and IgA2) or subclass, or allotype (e.g. human G1m1, G1m2, G1m3, non-G1m1 [that, is any allotype other than G1m1], G1m17, G2m23, G3m21, G3m28, G3m11, G3m5, G3m13, G3m14, G3m10, G3m15, G3m16, G3m6, G3m24, G3m26, G3m27, A2m1, A2m2, Km1, Km2 and Km3) of immunoglobulin molecule. The immunoglobulins can be derived from any species, including human, murine, or rabbit origin.

As used herein, "binds HER2" is used to mean the antibody binds HER2 with a higher affinity than a non-specific partner such as Bovine Serum Albumin (BSA, Genbank accession no. CAA76847, version no. CAA76847.1 GI:3336842, record update date: Jan 7, 2011 02:30 PM). In some embodiments the antibody binds HER2 with an association constant ($K_a$) at least 2, 3, 4, 5, 10, 20, 50, 100, 200, 500, 1000, 2000, 5000, $10^4$, $10^5$ or $10^6$-fold higher than the antibody's association constant for BSA, when measured at physiological conditions. The antibodies of the invention can bind HER2 with a high affinity. For example, in some embodiments the antibody can bind HER2 with a KD equal to or less than about 10-6 M, such as 1 x 10-6, 10-7, 10-8, 10-9,10-10, 10-11, 10-12, 10-13 or 10-14.

As used herein, HER2 refers to Human Epidermal Growth Factor Receptor 2. In one embodiment, HER2 polypeptide corresponds to Genbank accession no. AAA75493, version no. AAA75493.1 GI:306840, record update date: Jun 23, 2010 08:47 AM. In one embodiment, the nucleic acid encoding HER2 polypeptide corresponds to Genbank accession no. M11730, version no. M11730.1 GI:183986, record update date: Jun 23, 2010 08:47 AM.

"Antibody fragments" comprise a portion of a full length antibody, generally the antigen binding or variable region thereof. Examples of antibody fragments include Fab, Fab', $F(ab')_2$, and scFv fragments; diabodies; linear antibodies; fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

The term "monoclonal antibody" as used herein refers to an antibody obtained from a population of substantially homogeneous antibodies, i.e. the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as

requiring production of the antibody by any particular method. For example, the monoclonal antibodies to be used in accordance with the present invention may be made by the hybridoma method first described by Kohler et al (1975) Nature 256:495, or may be made by recombinant DNA methods (see, US 4816567). The monoclonal antibodies may also be isolated from phage antibody libraries using the techniques described in Clackson et al (1991) Nature, 352:624-628; Marks et al (1991) J. Mol. Biol., 222:581-597 or from transgenic mice carrying a fully human immunoglobulin system (Lonberg (2008) Curr. Opinion 20(4):450-459).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (US 4816567; and Morrison et al (1984) Proc. Natl. Acad. Sci. USA, 81:6851-6855). Chimeric antibodies include "primatized" antibodies comprising variable domain antigen-binding sequences derived from a non-human primate (e.g. Old World Monkey or Ape) and human constant region sequences.

An "intact antibody" herein is one comprising VL and VH domains, as well as a light chain constant domain (CL) and heavy chain constant domains, CH1, CH2 and CH3. The constant domains may be native sequence constant domains (e.g. human native sequence constant domains) or amino acid sequence variant thereof. The intact antibody may have one or more "effector functions" which refer to those biological activities attributable to the Fc region (a native sequence Fc region or amino acid sequence variant Fc region) of an antibody. Examples of antibody effector functions include C1 q binding; complement dependent cytotoxicity; Fc receptor binding; antibody-dependent cell-mediated cytotoxicity (ADCC); phagocytosis; and down regulation of cell surface receptors such as B cell receptor and BCR.

Depending on the amino acid sequence of the constant domain of their heavy chains, intact antibodies can be assigned to different "classes." There are five major classes of intact antibodies: IgA, IgD, IgE, IgG, and IgM, and several of these may be further divided into "subclasses" (isotypes), *e.g.*, IgG1, IgG2, IgG3, IgG4, IgA, and IgA2. The heavy-chain constant domains that correspond to the different classes of antibodies are called $\alpha$, $\delta$, $\varepsilon$, $\gamma$, and $\mu$, respectively. The subunit structures and three-dimensional configurations of different classes of immunoglobulins are well known.

**Modification of antibodies**

The antibodies disclosed herein may be modified. For example, to make them less immunogenic to a human subject. This may be achieved using any of a number of techniques familiar to the person skilled in the art. Some of these techniques are described in more detail below.

*Humanisation*

Techniques to reduce the *in vivo* immunogenicity of a non-human antibody or antibody fragment include those termed "humanisation".

A "humanized antibody" refers to a polypeptide comprising at least a portion of a modified variable region of a human antibody wherein a portion of the variable region, preferably a portion substantially less than the intact human variable domain, has been substituted by the corresponding sequence from a non-human species and wherein the modified variable region is linked to at least another part of another protein, preferably the constant region of a human antibody. The expression "humanized antibodies" includes human antibodies in which one or more complementarity determining region ("CDR") amino acid residues and/or one or more framework region ("FW" or "FR") amino acid residues are substituted by amino acid residues from analogous sites in rodent or other non-human antibodies. The expression "humanized antibody" also includes an immunoglobulin amino acid sequence variant or fragment thereof that comprises an FR having substantially the amino acid sequence of a human immunoglobulin and a CDR having substantially the amino acid sequence of a non-human immunoglobulin.

"Humanized" forms of non-human (e.g., murine) antibodies are chimeric antibodies that contain minimal sequence derived from non-human immunoglobulin. Or, looked at another way, a humanized antibody is a human antibody that also contains selected sequences from non-human (e.g. murine) antibodies in place of the human sequences. A humanized antibody can include conservative amino acid substitutions or non-natural residues from the same or different species that do not significantly alter its binding and/or biologic activity. Such antibodies are chimeric antibodies that contain minimal sequence derived from non-human immunoglobulins.

There are a range of humanisation techniques, including 'CDR grafting', 'guided selection', 'deimmunization', 'resurfacing' (also known as 'veneering'), 'composite antibodies', 'Human String Content Optimisation' and framework shuffling.

CDR grafting

In this technique, the humanized antibodies are human immunoglobulins (recipient antibody) in which residues from a complementary-determining region (CDR) of the recipient antibody are replaced by residues from a CDR of a non-human species (donor antibody) such as mouse, rat, camel, bovine, goat, or rabbit having the desired properties (in effect, the non-human CDRs are 'grafted' onto the human framework). In some instances, framework region (FR) residues of the human immunoglobulin are replaced by corresponding non-human residues (this may happen when, for example, a particular FR residue has significant effect on antigen binding).

Furthermore, humanized antibodies can comprise residues that are found neither in the recipient antibody nor in the imported CDR or framework sequences. These modifications are made to further refine and maximize antibody performance. Thus, in general, a humanized antibody will comprise all of at least one, and in one aspect two, variable domains, in which all or all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), or that of a human immunoglobulin.

Guided selection

The method consists of combining the $V_H$ or $V_L$ domain of a given non-human antibody specific for a particular epitope with a human $V_H$ or $V_L$ library and specific human V domains are selected against the antigen of interest. This selected human VH is then combined with a VL library to generate a completely human VHxVL combination. The method is described in Nature Biotechnology (N.Y.) 12, (1994) 899-903.

Composite antibodies

In this method, two or more segments of amino acid sequence from a human antibody are combined within the final antibody molecule. They are constructed by combining multiple human VH and VL sequence segments in combinations which limit or avoid human T cell epitopes in the final composite antibody V regions. Where required, T cell epitopes are limited or avoided by, exchanging V region segments contributing to or encoding a T cell epitope with alternative segments which avoid T cell epitopes. This method is described in US 2008/0206239 A1.

Deimmunization

This method involves the removal of human (or other second species) T-cell epitopes from the V regions of the therapeutic antibody (or other molecule). The therapeutic antibodies V-region sequence is analysed for the presence of MHC class II- binding motifs by, for example, comparison with databases of MHC-binding motifs (such as the "motifs" database hosted at www.wehi.edu.au). Alternatively, MHC class II- binding motifs may be identified using computational threading methods such as those devised by Altuvia et al. (J. Mol. Biol. 249 244-250 (1995)); in these methods, consecutive overlapping peptides from the V-region sequences are testing for their binding energies to MHC class II proteins. This data can then be combined with information on other sequence features which relate to successfully presented peptides, such as amphipathicity, Rothbard motifs, and cleavage sites for cathepsin B and other processing enzymes.

Once potential second species (e.g. human) T-cell epitopes have been identified, they are eliminated by the alteration of one or more amino acids. The modified amino acids are usually within the T-cell epitope itself, but may also be adjacent to the epitope in terms of the primary or secondary structure of the protein (and therefore, may not be adjacent in the primary structure). Most typically, the alteration is by way of substitution but, in some circumstances amino acid addition or deletion will be more appropriate.

All alterations can be accomplished by recombinant DNA technology, so that the final molecule may be prepared by expression from a recombinant host using well established methods such as Site Directed Mutagenesis. However, the use of protein chemistry or any other means of molecular alteration is also possible.

Resurfacing

This method involves:

(a) determining the conformational structure of the variable region of the non-human (e.g. rodent) antibody (or fragment thereof) by constructing a three-dimensional model of the non-human antibody variable region;

(b) generating sequence alignments using relative accessibility distributions from x-ray crystallographic structures of a sufficient number of non-human and human antibody variable region heavy and light chains to give a set of heavy and light chain framework positions wherein the alignment positions are identical in 98% of the sufficient

number of non-human antibody heavy and light chains;

(c) defining for the non-human antibody to be humanized, a set of heavy and light chain surface exposed amino acid residues using the set of framework positions generated in step (b);

(d) identifying from human antibody amino acid sequences a set of heavy and light chain surface exposed amino acid residues that is most closely identical to the set of surface exposed amino acid residues defined in step (c), wherein the heavy and light chain from the human antibody are or are not naturally paired;

(e) substituting, in the amino acid sequence of the non-human antibody to be humanized, the set of heavy and light chain surface exposed amino acid residues defined in step (c) with the set of heavy and light chain surface exposed amino acid residues identified in step (d);

(f) constructing a three-dimensional model of the variable region of the non-human antibody resulting from the substituting specified in step (e);

(g) identifying, by comparing the three-dimensional models constructed in steps (a) and (f), any amino acid residues from the sets identified in steps (c) or (d), that are within 5 Angstroms of any atom of any residue of the complementarity determining regions of the non-human antibodt to be humanized; and

(h) changing any residues identified in step (g) from the human to the original non-human amino acid residue to thereby define a non-human antibody humanizing set of surface exposed amino acid residues; with the proviso that step (a) need not be conducted first, but must be conducted prior to step (g).

Superhumanization

The method compares the non-human sequence with the functional human germline gene repertoire. Those human genes encoding canonical structures identical or closely related to the non-human sequences are selected. Those selected human genes with highest homology within the CDRs are chosen as FR donors. Finally, the non-human CDRs are grafted onto these human FRs. This method is described in patent WO 2005/079479 A2.

Human String Content Optimization

This method compares the non-human (e.g. mouse) sequence with the repertoire of human germline genes and the differences are scored as Human String Content (HSC) that quantifies a sequence at the level of potential MHC/T-cell epitopes. The target sequence is then humanized by maximizing its HSC rather than using a global identity measure to generate multiple diverse humanized variants (described in Molecular Immunology, 44, (2007) 1986-1998).

Framework Shuffling

The CDRs of the non-human antibody are fused in-frame to cDNA pools encompassing all known heavy and light chain human germline gene frameworks. Humanised antibodies are then selected by e.g. panning of the phage displayed antibody library. This is described in Methods 36, 43-60 (2005).

**Conjugates**

The present invention provides a conjugate comprising a PBD compound connected to the antibody via a Linker Unit.

The present invention is suitable for use in providing a PBD compound to a preferred site in a subject. In the preferred embodiments, the conjugate allows the release of an active PBD compound that does not retain any part of the linker. There is no stub present that could affect the reactivity of the PBD compound.

The linkers of the ADC preferably prevent aggregation of ADC molecules and keep the ADC freely soluble in aqueous media and in a monomeric state.

The linkers of the ADC are preferably stable extracellularly. Before transport or delivery into a cell, the antibody-drug conjugate (ADC) is preferably stable and remains intact, i.e. the antibody remains linked to the drug moiety. The linkers are stable outside the target cell and may be cleaved at some efficacious rate inside the cell. An effective linker will: (i) maintain the specific binding properties of the antibody; (ii) allow intracellular delivery of the conjugate or drug moiety; (iii) remain stable and intact, i.e. not cleaved, until the conjugate has been delivered or transported to its targetted site; and (iv) maintain a cytotoxic, cell-killing effect or a cytostatic effect of the PBD drug moiety. Stability of the ADC may be measured by standard analytical techniques such as mass spectroscopy, HPLC, and the separation/analysis technique LC/MS.

*Embodiments*

Embodiments of the present invention include ConjA wherein the antibody is as defined above.
Embodiments of the present invention include ConjB wherein the antibody is as defined above.
Embodiments of the present invention include ConjC wherein the antibody is as defined above.
Embodiments of the present invention include ConjD wherein the antibody is as defined above.
Embodiments of the present invention include ConjE wherein the antibody is as defined above.

*Drug loading*

The drug loading is the average number of PBD drugs per antibody, e.g. antibody. Where the compounds of the invention are bound to cysteines, drug loading may range from 1 to 8 drugs ($D^L$) per antibody, i.e. where 1, 2, 3, 4, 5, 6, 7, and 8 drug moieties are covalently attached to the antibody. Compositions of conjgates include collections of antibodies, conjugated with a range of drugs, from 1 to 8.
The average number of drugs per antibody in preparations of ADC from conjugation reactions may be characterized by conventional means such as UV, reverse phase HPLC, HIC, mass spectroscopy, ELISA assay, and electrophoresis. The quantitative distribution of ADC in terms of p may also be determined. By ELISA, the averaged value of p in a particular preparation of ADC may be determined (Hamblett et al (2004) Clin. Cancer Res. 10:7063-7070; Sanderson et al (2005) Clin. Cancer Res. 11:843-852). However, the distribution of p (drug) values is not discernible by the antibody-antigen binding and detection limitation of ELISA. Also, ELISA assay for detection of antibody-drug conjugates does not determine where the drug moieties are attached to the antibody, such as the heavy chain or light chain fragments, or the particular amino acid residues. In some instances, separation, purification, and characterization of homogeneous ADC where p is a certain value from ADC with other drug loadings may be achieved by means such as reverse phase HPLC or electrophoresis. Such techniques are also applicable to other types of conjugates.
For some antibody-drug conjugates, p may be limited by the number of attachment sites on the antibody. For example, an antibody may have only one or several cysteine thiol groups, or may have only one or several sufficiently reactive thiol groups through which a linker may be attached. Higher drug loading, e.g. p >5, may cause aggregation, insolubility, toxicity, or loss of cellular permeability of certain antibody-drug conjugates.
Typically, fewer than the theoretical maximum of drug moieties are conjugated to an antibody during a conjugation reaction. An antibody may contain, for example, many lysine residues that do not react with the drug-linker intermediate (D-L) or linker reagent. Only the most reactive lysine groups may react with an amine-reactive linker reagent. Also, only the most reactive cysteine thiol groups may react with a thiol-reactive linker reagent. Generally, antibodies do not contain many, if any, free and reactive cysteine thiol groups which may be linked to a drug moiety. Most cysteine thiol residues in the antibodies of the compounds exist as disulfide bridges and must be reduced with a reducing agent such as dithiothreitol (DTT) or TCEP, under partial or total reducing conditions. The loading (drug/antibody ratio) of an ADC may be controlled in several different manners, including: (i) limiting the molar excess of drug-linker intermediate (D-L) or linker reagent relative to antibody, (ii) limiting the conjugation reaction time or temperature, and (iii) partial or limiting reductive conditions for cysteine thiol modification.
Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol. Reactive thiol groups may be introduced into the antibody (or fragment thereof) by engineering one, two, three, four, or more cysteine residues (e.g., preparing mutant antibodies comprising one or more non-native cysteine amino acid residues). US 7521541 teaches engineering antibodies by introduction of reactive cysteine amino acids.
Cysteine amino acids may be engineered at reactive sites in an antibody and which do not form intrachain or intermolecular disulfide linkages (Junutula, et al., 2008b Nature Biotech., 26(8):925-932; Dornan et al (2009) Blood 114(13):2721-2729; US 7521541; US 7723485; WO2009/052249). The engineered cysteine thiols may react with linker reagents or the drug-linker reagents of the present invention which have thiol-reactive, electrophilic groups such as maleimide or alpha-halo amides to form ADC with cysteine engineered antibodies and the PBD drug moieties. The location of the drug moiety can thus be designed, controlled, and known. The drug loading can be controlled since the engineered cysteine thiol groups typically react with thiol-reactive linker reagents or drug-linker reagents in high yield. Engineering an IgG antibody to introduce a cysteine amino acid by substitution at a single site on the heavy or light chain gives two new cysteines on the symmetrical antibody. A drug loading near 2 can be achieved with near homogeneity of the conjugation product ADC.
Alternatively, site-specific conjugation can be achieved by engineering antibodies to contain unnatural amino acids in their heavy and/or light chains as described by Axup et al. ((2012), Proc Natl Acad Sci USA. 109(40):16101-16116). The unnatural amino acids provide the additional advantage that orthogonal chemistry can be

designed to attach the linker reagent and drug.

Where more than one nucleophilic or electrophilic group of the antibody reacts with a drug-linker intermediate, or linker reagent followed by drug moiety reagent, then the resulting product is a mixture of ADC compounds with a distribution of drug moieties attached to an antibody, e.g. 1, 2, 3, etc. Liquid chromatography methods such as polymeric reverse phase (PLRP) and hydrophobic interaction (HIC) may separate compounds in the mixture by drug loading value. Preparations of ADC with a single drug loading value (p) may be isolated, however, these single loading value ADCs may still be heterogeneous mixtures because the drug moieties may be attached, via the linker, at different sites on the antibody.

Thus the antibody-drug conjugate compositions of the invention include mixtures of antibody-drug conjugate compounds where the antibody has one or more PBD drug moieties and where the drug moieties may be attached to the antibody at various amino acid residues.

In one embodiment, the average number of dimer pyrrolobenzodiazepine groups per antibody is in the range selected from 1 to 8, 2 to 8, 2 to 6, 2 to 4, and 4 to 8.

In some embodiments, there is one dimer pyrrolobenzodiazepine group per antibody.

*Includes Other Forms*

Unless otherwise specified, included in the above are the well known ionic, salt, solvate, and protected forms of these substituents. For example, a reference to carboxylic acid (-COOH) also includes the anionic (carboxylate) form ($-COO^-$), a salt or solvate thereof, as well as conventional protected forms. Similarly, a reference to an amino group includes the protonated form ($-N^+HR^1R^2$), a salt or solvate of the amino group, for example, a hydrochloride salt, as well as conventional protected forms of an amino group. Similarly, a reference to a hydroxyl group also includes the anionic form ($-O^-$), a salt or solvate thereof, as well as conventional protected forms.

*Salts*

It may be convenient or desirable to prepare, purify, and/or handle a corresponding salt of the active compound, for example, a pharmaceutically-acceptable salt. Examples of pharmaceutically acceptable salts are discussed in Berge, et al., J. Pharm. Sci., 66, 1-19 (1977).

For example, if the compound is anionic, or has a functional group which may be anionic (e.g. -COOH may be $-COO^-$), then a salt may be formed with a suitable cation. Examples of suitable inorganic cations include, but are not limited to, alkali metal ions such as $Na^+$ and $K^+$, alkaline earth cations such as $Ca^{2+}$ and $Mg^{2+}$, and other cations such as $Al^{+3}$. Examples of suitable organic cations include, but are not limited to, ammonium ion (i.e. $NH_4^+$) and substituted ammonium ions (e.g. $NH_3R^+$, $NH_2R_2^+$, $NHR_3^+$, $NR_4^+$). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is $N(CH_3)_4^+$.

If the compound is cationic, or has a functional group which may be cationic (e.g. $-NH_2$ may be $-NH_3^+$), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, trifluoroacetic acid and valeric. Examples of suitable polymeric organic anions include, but are not limited to, those derived from the following polymeric acids: tannic acid, carboxymethyl cellulose.

*Solvates*

It may be convenient or desirable to prepare, purify, and/or handle a corresponding solvate of the active compound. The term "solvate" is used herein in the conventional sense to refer to a complex of solute (e.g. active compound, salt of active compound) and solvent. If the solvent is water, the solvate may be conveniently referred to as a hydrate, for example, a mono-hydrate, a di-hydrate, a tri-hydrate, etc.

The invention includes compounds where a solvent adds across the imine bond of the PBD moiety, which is illustrated below where the solvent is water or an alcohol ($R^AOH$, where $R^A$ is $C_{1-4}$ alkyl):

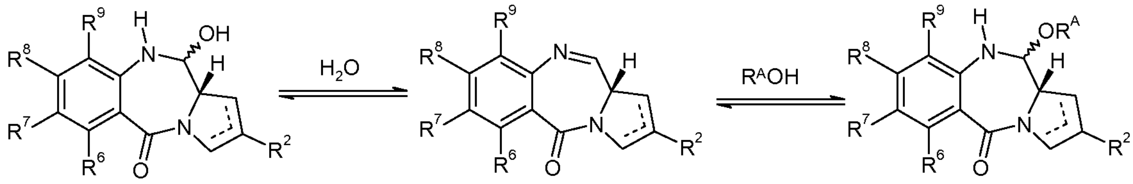


These forms can be called the carbinolamine and carbinolamine ether forms of the PBD (as described in the section relating to $R^{10}$ above). The balance of these equilibria depend on the conditions in which the compounds are found, as well as the nature of the moiety itself.

These particular compounds may be isolated in solid form, for example, by lyophilisation.

*Isomers*

Certain compounds of the invention may exist in one or more particular geometric, optical, enantiomeric, diasteriomeric, epimeric, atropic, stereoisomeric, tautomeric, conformational, or anomeric forms, including but not limited to, cis- and trans-forms; E- and Z-forms; c-, t-, and r- forms; endo- and exo-forms; R-, S-, and meso-forms; D- and L-forms; d- and l-forms; (+) and (-) forms; keto-, enol-, and enolate-forms; syn- and anti-forms; synclinal- and anticlinal-forms; α- and β-forms; axial and equatorial forms; boat-, chair-, twist-, envelope-, and halfchair-forms; and combinations thereof, hereinafter collectively referred to as "isomers" (or "isomeric forms").

The term "chiral" refers to molecules which have the property of non-superimposability of the mirror image partner, while the term "achiral" refers to molecules which are superimposable on their mirror image partner.

The term "stereoisomers" refers to compounds which have identical chemical constitution, but differ with regard to the arrangement of the atoms or groups in space.

"Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers may separate under high resolution analytical procedures such as electrophoresis and chromatography.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. The compounds of the invention may contain asymmetric or chiral centers, and therefore exist in different stereoisomeric forms. It is intended that all stereoisomeric forms of the compounds of the invention, including but not limited to, diastereomers, enantiomers and atropisomers, as well as mixtures thereof such as racemic mixtures, form part of the present invention. Many organic compounds exist in optically active forms, i.e., they have the ability to rotate the plane of plane-polarized light. In describing an optically active compound, the prefixes D and L, or *R* and *S*, are used to denote the absolute configuration of the molecule about its chiral center(s). The prefixes d and l or (+) and (-) are employed to designate the sign of rotation of plane-polarized light by the compound, with (-) or l meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

Note that, except as discussed below for tautomeric forms, specifically excluded from the term "isomers", as used herein, are structural (or constitutional) isomers (i.e. isomers which differ in the connections between atoms rather than merely by the position of atoms in space). For example, a reference to a methoxy group, $-OCH_3$, is not to be construed as a reference to its structural isomer, a hydroxymethyl group, $-CH_2OH$. Similarly, a reference to ortho-chlorophenyl is not to be construed as a reference to its structural isomer, meta-chlorophenyl. However, a reference to a class of structures may well include structurally isomeric forms falling within that class (e.g. $C_{1-7}$ alkyl includes n-propyl and iso-propyl; butyl includes n-, iso-, sec-, and tert-butyl; methoxyphenyl includes ortho-, meta-, and para-methoxyphenyl).

The above exclusion does not pertain to tautomeric forms, for example, keto-, enol-, and enolate-forms, as in, for example, the following tautomeric pairs: keto/enol (illustrated below), imine/enamine, amide/imino alcohol, amidine/amidine, nitroso/oxime, thioketone/enethiol, N-nitroso/hyroxyazo, and nitro/aci-nitro.

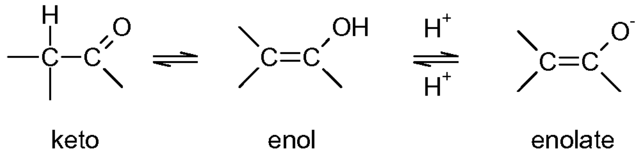

The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

Note that specifically included in the term "isomer" are compounds with one or more isotopic substitutions. For example, H may be in any isotopic form, including $^{1}H$, $^{2}H$ (D), and $^{3}H$ (T); C may be in any isotopic form, including $^{12}C$, $^{13}C$, and $^{14}C$; O may be in any isotopic form, including $^{16}O$ and $^{18}O$; and the like.

Examples of isotopes that can be incorporated into compounds of the invention include isotopes of hydrogen, carbon, nitrogen, oxygen, phosphorous, fluorine, and chlorine, such as, but not limited to $^{2}H$ (deuterium, D), $^{3}H$ (tritium), $^{11}C$, $^{13}C$, $^{14}C$, $^{15}N$, $^{18}F$, $^{31}P$, $^{32}P$, $^{35}S$, $^{36}Cl$, and $^{125}I$. Various isotopically labeled compounds of the present invention, for example those into which radioactive isotopes such as 3H, 13C, and 14C are incorporated. Such isotopically labelled compounds may be useful in metabolic studies, reaction kinetic studies, detection or imaging techniques, such as positron emission tomography (PET) or single-photon emission computed tomography (SPECT) including drug or substrate tissue distribution assays, or in radioactive treatment of patients. Deuterium labelled or substituted therapeutic compounds of the invention may have improved DMPK (drug metabolism and pharmacokinetics) properties, relating to distribution, metabolism, and excretion (ADME). Substitution with heavier isotopes such as deuterium may afford certain therapeutic advantages resulting from greater metabolic stability, for example increased in vivo half-life or reduced dosage requirements. An 18F labeled compound may be useful for PET or SPECT studies. Isotopically labeled compounds of this invention and prodrugs thereof can generally be prepared by carrying out the procedures disclosed in the schemes or in the examples and preparations described below by substituting a readily available isotopically labeled reagent for a non-isotopically labeled reagent. Further, substitution with heavier isotopes, particularly deuterium (i.e., 2H or D) may afford certain therapeutic advantages resulting from greater metabolic stability, for example increased in vivo half-life or reduced dosage requirements or an improvement in therapeutic index. It is understood that deuterium in this context is regarded as a substituent. The concentration of such a heavier isotope, specifically deuterium, may be defined by an isotopic enrichment factor. In the compounds of this invention any atom not specifically designated as a particular isotope is meant to represent any stable isotope of that atom.

Unless otherwise specified, a reference to a particular compound includes all such isomeric forms, including (wholly or partially) racemic and other mixtures thereof. Methods for the preparation (e.g. asymmetric synthesis) and separation (e.g. fractional crystallisation and chromatographic means) of such isomeric forms are either known in the art or are readily obtained by adapting the methods taught herein, or known methods, in a known manner.

### *Biological Activity*

In vitro cell proliferation assays

Generally, the cytotoxic or cytostatic activity of an antibody-drug conjugate (ADC) is measured by: exposing mammalian cells having receptor proteins to the antibody of the ADC in a cell culture medium; culturing the cells for a period from about 6 hours to about 5 days; and measuring cell viability. Cell-based *in vitro* assays are used to measure viability (proliferation), cytotoxicity, and induction of apoptosis (caspase activation) of an ADC of the invention.

The *in vitro* potency of antibody-drug conjugates can be measured by a cell proliferation assay. The CellTiter-Glo® Luminescent Cell Viability Assay is a commercially available (Promega Corp., Madison, WI), homogeneous assay method based on the recombinant expression of *Coleoptera* luciferase (US Patent Nos. 5583024; 5674713 and 5700670). This cell proliferation assay determines the number of viable cells in culture based on quantitation of the ATP present, an indicator of metabolically active cells (Crouch et al (1993) J. Immunol. Meth. 160:81-88; US 6602677). The CellTiter-Glo® Assay is conducted in 96 well format, making it amenable to automated high-throughput screening (HTS) (Cree et al (1995) AntiCancer Drugs 6:398-404). The homogeneous assay procedure involves adding the single reagent (CellTiter-Glo® Reagent) directly to cells cultured in serum-supplemented medium. Cell washing, removal of medium and multiple pipetting steps are not required. The system detects as few as 15 cells/well in a 384-well format in 10 minutes after adding reagent and mixing. The cells may be treated continuously with ADC, or they may be treated and separated from ADC. Generally, cells treated briefly, i.e. 3 hours, showed the same potency effects as continuously treated cells.

The homogeneous "add-mix-measure" format results in cell lysis and generation of a luminescent signal proportional to the amount of ATP present. The amount of ATP is directly proportional to the number of cells present in culture. The CellTiter-Glo® Assay generates a "glow-type" luminescent signal, produced by the luciferase reaction, which has a half-life generally greater than five hours, depending on cell type and medium used. Viable cells are reflected in relative luminescence units (RLU). The substrate, Beetle Luciferin, is oxidatively decarboxylated by recombinant firefly luciferase with concomitant conversion of ATP to AMP and generation of photons.

The *in vitro* potency of antibody-drug conjugates can also be measured by a cytotoxicity assay. Cultured adherent cells are washed with PBS, detached with trypsin, diluted in complete medium, containing 10% FCS, centrifuged, re-suspended in fresh medium and counted with a haemocytometer. Suspension cultures are counted directly. Monodisperse cell suspensions suitable for counting may require agitation of the suspension by repeated aspiration to break up cell clumps.

The cell suspension is diluted to the desired seeding density and dispensed (100μl per well) into black 96 well plates. Plates of adherent cell lines are incubated overnight to allow adherence. Suspension cell cultures can be used on the day of seeding.

A stock solution (1ml) of ADC (20μg/ml) is made in the appropriate cell culture medium. Serial 10-fold dilutions of stock ADC are made in 15ml centrifuge tubes by serially transferring 100μl to 900μl of cell culture medium.

Four replicate wells of each ADC dilution (100μl) are dispensed in 96-well black plates, previously plated with cell suspension (100μl), resulting in a final volume of 200 μl. Control wells receive cell culture medium (100μl).

If the doubling time of the cell line is greater than 30 hours, ADC incubation is for 5 days, otherwise a four day incubation is done.

At the end of the incubation period, cell viability is assessed with the Alamar blue assay. AlamarBlue (Invitrogen) is dispensed over the whole plate (20μl per well) and incubated for 4 hours. Alamar blue fluorescence is measured at excitation 570nm, emission 585nm on the Varioskan flash plate reader. Percentage cell survival is calculated from the mean fluorescence in the ADC treated wells compared to the mean fluorescence in the control wells.

***Use***

The conjugates of the invention may be used to provide a PBD compound at a target location.

The target location is preferably a proliferative cell population. The antibody is an antibody for an antigen present on a proliferative cell population.

In one embodiment the antigen is absent or present at a reduced level in a non-proliferative cell population compared to the amount of antigen present in the proliferative cell population, for example a tumour cell population.

At the target location the linker may be cleaved so as to release a compound RelA, RelB, RelC, RelD or RelE. Thus, the conjugate may be used to selectively provide a compound RelA, RelB, Rel C, RelD or RelE to the target location.

The linker may be cleaved by an enzyme present at the target location.

The target location may be *in vitro*, *in vivo* or *ex vivo.*

The antibody-drug conjugate (ADC) compounds of the invention include those with utility for anticancer activity. In particular, the compounds include an antibody conjugated, i.e. covalently attached by a linker, to a PBD drug moiety, i.e. toxin. When the drug is not conjugated to an antibody, the PBD drug has a cytotoxic effect. The biological activity of the PBD drug moiety is thus modulated by conjugation to an antibody. The antibody-drug conjugates (ADC) of the invention selectively deliver an effective dose of a cytotoxic agent to tumor tissue whereby greater selectivity, i.e. a lower efficacious dose, may be achieved.

Thus, in one aspect, the present invention provides a conjugate compound as described herein for use in therapy.

In a further aspect there is also provides a conjugate compound as described herein for use in the treatment of a proliferative disease. A second aspect of the present invention provides the use of a conjugate compound in the manufacture of a medicament for treating a proliferative disease.

One of ordinary skill in the art is readily able to determine whether or not a candidate conjugate treats a proliferative condition for any particular cell type. For example, assays which may conveniently be used to assess the activity offered by a particular compound are described in the examples below.

The term "proliferative disease" pertains to an unwanted or uncontrolled cellular proliferation of excessive or abnormal cells which is undesired, such as, neoplastic or hyperplastic growth, whether *in vitro* or *in vivo.*

Examples of proliferative conditions include, but are not limited to, benign, pre-malignant, and malignant cellular proliferation, including but not limited to, neoplasms and tumours (e.g. histocytoma, glioma, astrocyoma, osteoma), cancers (e.g. lung cancer, small cell lung cancer, gastrointestinal cancer, bowel cancer, colon cancer, breast carinoma, ovarian carcinoma, prostate cancer, testicular cancer, liver cancer, kidney cancer, bladder cancer, pancreas cancer, brain cancer, sarcoma, osteosarcoma, Kaposi's sarcoma, melanoma), lymphomas, leukemias, psoriasis, bone diseases, fibroproliferative disorders (e.g. of connective tissues), and atherosclerosis. Cancers of particular interest include, but are not limited to, leukemias and ovarian cancers.

Any type of cell may be treated, including but not limited to, lung, gastrointestinal (including, e.g. bowel, colon),

breast (mammary), ovarian, prostate, liver (hepatic), kidney (renal), bladder, pancreas, brain, and skin.

Cancers of particular interest include, but are not limited to, breast, gastric or bladder cancers.

It is contemplated that the antibody-drug conjugates (ADC) of the present invention may be used to treat various diseases or disorders, e.g. characterized by the overexpression of a tumor antigen. Exemplary conditions or hyperproliferative disorders include benign or malignant tumors; leukemia, haematological, and lymphoid malignancies. Others include neuronal, glial, astrocytal, hypothalamic, glandular, macrophagal, epithelial, stromal, blastocoelic, inflammatory, angiogenic and immunologic, including autoimmune, disorders.

Generally, the disease or disorder to be treated is a hyperproliferative disease such as cancer. Examples of cancer to be treated herein include, but are not limited to, carcinoma, lymphoma, blastoma, sarcoma, and leukemia or lymphoid malignancies. More particular examples of such cancers include squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

Autoimmune diseases for which the ADC compounds may be used in treatment include rheumatologic disorders (such as, for example, rheumatoid arthritis, Sjögren's syndrome, scleroderma, lupus such as SLE and lupus nephritis, polymyositis/dermatomyositis, cryoglobulinemia, anti-phospholipid antibody syndrome, and psoriatic arthritis), osteoarthritis, autoimmune gastrointestinal and liver disorders (such as, for example, inflammatory bowel diseases (e.g. ulcerative colitis and Crohn's disease), autoimmune gastritis and pernicious anemia, autoimmune hepatitis, primary biliary cirrhosis, primary sclerosing cholangitis, and celiac disease), vasculitis (such as, for example, ANCA-associated vasculitis, including Churg-Strauss vasculitis, Wegener's granulomatosis, and polyarteriitis), autoimmune neurological disorders (such as, for example, multiple sclerosis, opsoclonus myoclonus syndrome, myasthenia gravis, neuromyelitis optica, Parkinson's disease, Alzheimer's disease, and autoimmune polyneuropathies), renal disorders (such as, for example, glomerulonephritis, Goodpasture's syndrome, and Berger's disease), autoimmune dermatologic disorders (such as, for example, psoriasis, urticaria, hives, pemphigus vulgaris, bullous pemphigoid, and cutaneous lupus erythematosus), hematologic disorders (such as, for example, thrombocytopenic purpura, thrombotic thrombocytopenic purpura, post-transfusion purpura, and autoimmune hemolytic anemia), atherosclerosis, uveitis, autoimmune hearing diseases (such as, for example, inner ear disease and hearing loss), Behcet's disease, Raynaud's syndrome, organ transplant, and autoimmune endocrine disorders (such as, for example, diabetic-related autoimmune diseases such as insulin-dependent diabetes mellitus (IDDM), Addison's disease, and autoimmune thyroid disease (e.g. Graves' disease and thyroiditis)). More preferred such diseases include, for example, rheumatoid arthritis, ulcerative colitis, ANCA-associated vasculitis, lupus, multiple sclerosis, Sjögren's syndrome, Graves' disease, IDDM, pernicious anemia, thyroiditis, and glomerulonephritis.

***Methods of Treatment***

The conjugates of the present invention may be used in a method of therapy

The actual amount administered, and rate and time-course of administration, will depend on the nature and severity of what is being treated. Prescription of treatment, e.g. decisions on dosage, is within the responsibility of general practitioners and other medical doctors.

A compound of the invention may be administered alone or in combination with other treatments, either simultaneously or sequentially dependent upon the condition to be treated. Examples of treatments and therapies include, but are not limited to, chemotherapy (the administration of active agents, including, e.g. drugs, such as chemotherapeutics); surgery; and radiation therapy.

A "chemotherapeutic agent" is a chemical compound useful in the treatment of cancer, regardless of mechanism of action. Classes of chemotherapeutic agents include, but are not limited to: alkylating agents, antimetabolites, spindle poison plant alkaloids, cytotoxic/antitumor antibiotics, topoisomerase inhibitors, antibodies, photosensitizers, and kinase inhibitors. Chemotherapeutic agents include compounds used in "targeted therapy" and conventional chemotherapy.

Examples of chemotherapeutic agents include: erlotinib (TARCEVA®, Genentech/OSI Pharm.), docetaxel (TAXOTERE®, Sanofi-Aventis), 5-FU (fluorouracil, 5-fluorouracil, CAS No. 51-21-8), gemcitabine (GEMZAR®, Lilly), PD-0325901 (CAS No. 391210-10-9, Pfizer), cisplatin (cis-diamine, dichloroplatinum(II), CAS No. 15663-27-1), carboplatin (CAS No. 41575-94-4), paclitaxel (TAXOL®, Bristol-Myers Squibb Oncology, Princeton, N.J.), trastuzumab (HERCEPTIN®, Genentech), temozolomide (4-methyl-5-oxo- 2,3,4,6,8-pentazabicyclo [4.3.0] nona-2,7,9-triene- 9-carboxamide, CAS No. 85622-93-1, TEMODAR®, TEMODAL®, Schering Plough), tamoxifen ((Z)-2-[4-(1,2-diphenylbut-1-enyl)phenoxy]-N,N-dimethylethanamine, NOLVADEX®, ISTUBAL®, VALODEX®), and doxorubicin (ADRIAMYCIN®), Akti-1/2, HPPD, and rapamycin.

More examples of chemotherapeutic agents include: oxaliplatin (ELOXATIN®, Sanofi), bortezomib (VELCADE®, Millennium Pharm.), sutent (SUNITINIB®, SU11248, Pfizer), letrozole (FEMARA®, Novartis), imatinib mesylate (GLEEVEC®, Novartis), XL-518 (Mek inhibitor, Exelixis, WO 2007/044515), ARRY-886 (Mek inhibitor, AZD6244, Array BioPharma, Astra Zeneca), SF-1126 (PI3K inhibitor, Semafore Pharmaceuticals), BEZ-235 (PI3K inhibitor, Novartis), XL-147 (PI3K inhibitor, Exelixis), PTK787/ZK 222584 (Novartis), fulvestrant (FASLODEX®, AstraZeneca), leucovorin (folinic acid), rapamycin (sirolimus, RAPAMUNE®, Wyeth), lapatinib (TYKERB®, GSK572016, Glaxo Smith Kline), lonafarnib (SARASAR™, SCH 66336, Schering Plough), sorafenib (NEXAVAR®, BAY43-9006, Bayer Labs), gefitinib (IRESSA®, AstraZeneca), irinotecan (CAMPTOSAR®, CPT-11, Pfizer), tipifarnib (ZARNESTRA™, Johnson & Johnson), ABRAXANE™ (Cremophor-free), albumin-engineered nanoparticle formulations of paclitaxel (American Pharmaceutical Partners, Schaumberg, Il), vandetanib (rINN, ZD6474, ZACTIMA®, AstraZeneca), chloranmbucil, AG1478, AG1571 (SU 5271; Sugen), temsirolimus (TORISEL®, Wyeth), pazopanib (GlaxoSmithKline), canfosfamide (TELCYTA®, Telik), thiotepa and cyclosphosphamide (CYTOXAN®, NEOSAR®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including the synthetic analog topotecan); bryostatin; callystatin; CC-1065 (including its adozelesin, carzelesin and bizelesin synthetic analogs); cryptophycins (particularly cryptophycin 1 and cryptophycin 8); dolastatin; duocarmycin (including the synthetic analogs, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g. calicheamicin, calicheamicin gamma1I, calicheamicin omegaI1 (Angew Chem. Intl. Ed. Engl. (1994) 33:183-186); dynemicin, dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, morpholino-doxorubicin, cyanomorpholino-doxorubicin, 2-pyrrolino-doxorubicin and deoxydoxorubicin), epirubicin, esorubicin, idarubicin, nemorubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; 6-thioguanine; mercaptopurine; methotrexate; platinum analogs such as cisplatin and carboplatin; vinblastine; etoposide (VP-16); ifosfamide; mitoxantrone; vincristine; vinorelbine (NAVELBINE®); novantrone; teniposide; edatrexate; daunomycin; aminopterin; capecitabine (XELODA®, Roche); ibandronate; CPT-11; topoisomerase inhibitor RFS 2000; difluoromethylornithine (DMFO); retinoids such as retinoic acid; and pharmaceutically acceptable salts, acids and derivatives of any of the above.

Also included in the definition of "chemotherapeutic agent" are: (i) anti-hormonal agents that act to regulate or inhibit hormone action on tumors such as anti-estrogens and selective estrogen receptor modulators (SERMs), including, for example, tamoxifen (including NOLVADEX®; tamoxifen citrate), raloxifene, droloxifene, 4-hydroxytamoxifen, trioxifene, keoxifene, LY117018, onapristone, and FARESTON® (toremifine citrate); (ii) aromatase inhibitors that inhibit the enzyme aromatase, which regulates estrogen production in the adrenal glands, such as, for example, 4(5)-imidazoles, aminoglutethimide, MEGASE® (megestrol acetate), AROMASIN® (exemestane; Pfizer), formestanie, fadrozole, RIVISOR® (vorozole), FEMARA® (letrozole; Novartis), and ARIMIDEX® (anastrozole; AstraZeneca); (iii) anti-androgens such as flutamide, nilutamide, bicalutamide, leuprolide, and goserelin; as well as troxacitabine (a 1,3-dioxolane nucleoside cytosine analog); (iv) protein kinase inhibitors such as MEK inhibitors (WO 2007/044515); (v) lipid kinase inhibitors; (vi) antisense oligonucleotides, particularly those which inhibit expression of genes in signaling pathways implicated in aberrant cell proliferation, for example, PKC-alpha, Raf and H-Ras, such as oblimersen (GENASENSE®, Genta Inc.); (vii) ribozymes such as VEGF expression inhibitors (e.g., ANGIOZYME®) and HER2 expression inhibitors; (viii) vaccines such as gene therapy vaccines, for example, ALLOVECTIN®, LEUVECTIN®, and VAXID®; PROLEUKIN® rIL-2; topoisomerase 1 inhibitors such as LURTOTECAN®; ABARELIX® rmRH; (ix) anti-angiogenic agents such as bevacizumab (AVASTIN®, Genentech); and pharmaceutically acceptable salts, acids and derivatives of any of the above.
Also included in the definition of "chemotherapeutic agent" are therapeutic antibodies such as alemtuzumab (Campath), bevacizumab (AVASTIN®, Genentech); cetuximab (ERBITUX®, Imclone); panitumumab (VECTIBIX®, Amgen), rituximab (RITUXAN®, Genentech/Biogen Idec), ofatumumab (ARZERRA®, GSK), pertuzumab (PERJETA™, OMNITARG™, 2C4, Genentech), trastuzumab (HERCEPTIN®, Genentech), tositumomab (Bexxar, Corixia), and the antibody drug conjugate, gemtuzumab ozogamicin (MYLOTARG®, Wyeth).

Humanized monoclonal antibodies with therapeutic potential as chemotherapeutic agents in combination with the conjugates of the invention include: alemtuzumab, apolizumab, aselizumab, atlizumab, bapineuzumab, bevacizumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pertuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, trastuzumab, tucotuzumab celmoleukin, tucusituzumab, umavizumab, urtoxazumab, and visilizumab.

Pharmaceutical compositions according to the present invention, and for use in accordance with the present invention, may comprise, in addition to the active ingredient, i.e. a conjugate compound, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. cutaneous, subcutaneous, or intravenous.

Pharmaceutical compositions for oral administration may be in tablet, capsule, powder or liquid form. A tablet may comprise a solid carrier or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included. A capsule may comprise a solid carrier such a gelatin.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

***Formulations***

While it is possible for the conjugate compound to be used (e.g., administered) alone, it is often preferable to present it as a composition or formulation.

In one embodiment, the composition is a pharmaceutical composition (e.g., formulation, preparation, medicament) comprising a conjugate compound, as described herein, and a pharmaceutically acceptable carrier, diluent, or excipient.

In one embodiment, the composition is a pharmaceutical composition comprising at least one conjugate compound, as described herein, together with one or more other pharmaceutically acceptable ingredients well known to those skilled in the art, including, but not limited to, pharmaceutically acceptable carriers, diluents, excipients, adjuvants, fillers, buffers, preservatives, anti-oxidants, lubricants, stabilisers, solubilisers, surfactants (e.g., wetting agents), masking agents, colouring agents, flavouring agents, and sweetening agents.

In one embodiment, the composition further comprises other active agents, for example, other therapeutic or prophylactic agents.

Suitable carriers, diluents, excipients, etc. can be found in standard pharmaceutical texts. See, for example, Handbook of Pharmaceutical Additives, 2nd Edition (eds. M. Ash and I. Ash), 2001 (Synapse Information Resources, Inc., Endicott, New York, USA), Remington's Pharmaceutical Sciences, 20th edition, pub. Lippincott, Williams & Wilkins, 2000; and Handbook of Pharmaceutical Excipients, 2nd edition, 1994.

Another aspect of the present invention pertains to methods of making a pharmaceutical composition comprising admixing at least one [$^{11}C$]-radiolabelled conjugate or conjugate-like compound, as defined herein, together with one or more other pharmaceutically acceptable ingredients well known to those skilled in the art, e.g., carriers, diluents, excipients, etc. If formulated as discrete units (e.g., tablets, etc.), each unit contains a predetermined amount (dosage) of the active compound.

The term "pharmaceutically acceptable," as used herein, pertains to compounds, ingredients, materials, compositions, dosage forms, etc., which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each carrier, diluent, excipient, etc. must also be "acceptable" in the sense of being compatible with the other ingredients of the formulation.

The formulations may be prepared by any methods well known in the art of pharmacy. Such methods include the step of bringing into association the active compound with a carrier which constitutes one or more accessory ingredients. In general, the formulations are prepared by uniformly and intimately bringing into association the active compound with carriers (e.g., liquid carriers, finely divided solid carrier, etc.), and then shaping the product, if necessary.

The formulation may be prepared to provide for rapid or slow release; immediate, delayed, timed, or sustained release; or a combination thereof.

Formulations suitable for parenteral administration (e.g., by injection), include aqueous or non-aqueous, isotonic, pyrogen-free, sterile liquids (e.g., solutions, suspensions), in which the active ingredient is dissolved, suspended, or otherwise provided (e.g., in a liposome or other microparticulate). Such liquids may additional contain other pharmaceutically acceptable ingredients, such as anti-oxidants, buffers, preservatives, stabilisers, bacteriostats, suspending agents, thickening agents, and solutes which render the formulation isotonic with the blood (or other relevant bodily fluid) of the intended recipient. Examples of excipients include, for example, water, alcohols, polyols, glycerol, vegetable oils, and the like. Examples of suitable isotonic carriers for use in such formulations include Sodium Chloride Injection, Ringer's Solution, or Lactated Ringer's Injection. Typically, the concentration of the active ingredient in the liquid is from about 1 ng/ml to about 10 μg/ml, for example from about 10 ng/ml to about 1 μg/ml. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets.

***Dosage***

It will be appreciated by one of skill in the art that appropriate dosages of the conjugate compound, and compositions comprising the conjugate compound, can vary from patient to patient. Determining the optimal dosage will generally involve the balancing of the level of therapeutic benefit against any risk or deleterious side effects. The selected dosage level will depend on a variety of factors including, but not limited to, the activity of the particular compound, the route of administration, the time of administration, the rate of excretion of the compound, the duration of the treatment, other drugs, compounds, and/or materials used in combination, the severity of the condition, and the species, sex, age, weight, condition, general health, and prior medical history of the patient. The amount of compound and route of administration will ultimately be at the discretion of the physician, veterinarian, or clinician, although generally the dosage will be selected to achieve local concentrations at the site of action which achieve the desired effect without causing substantial harmful or deleterious side-effects.

Administration can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell(s) being treated, and the subject being treated. Single or multiple administrations can be carried out with the dose level and pattern being selected by the treating physician, veterinarian, or clinician.

In general, a suitable dose of the active compound is in the range of about 100 ng to about 25 mg (more typically about 1 μg to about 10 mg) per kilogram body weight of the subject per day. Where the active compound is a salt, an ester, an amide, a prodrug, or the like, the amount administered is calculated on the basis of the parent compound and so the actual weight to be used is increased proportionately.

In one embodiment, the active compound is administered to a human patient according to the following dosage regime: about 100 mg, 3 times daily.

In one embodiment, the active compound is administered to a human patient according to the following dosage regime: about 150 mg, 2 times daily.

In one embodiment, the active compound is administered to a human patient according to the following dosage regime: about 200 mg, 2 times daily.

However in one embodiment, the conjugate compound is administered to a human patient according to the following dosage regime: about 50 or about 75 mg, 3 or 4 times daily.

In one embodiment, the conjugate compound is administered to a human patient according to the following dosage regime: about 100 or about 125 mg, 2 times daily.

The dosage amounts described above may apply to the conjugate (including the PBD moiety and the linker to the antibody) or to the effective amount of PBD compound provided, for example the amount of compound that is releasable after cleavage of the linker.

For the prevention or treatment of disease, the appropriate dosage of an ADC of the invention will depend on the type of disease to be treated, as defined above, the severity and course of the disease, whether the molecule is administered for preventive or therapeutic purposes, previous therapy, the patient's clinical history and response to the antibody, and the discretion of the attending physician. The molecule is suitably administered to the patient at one time or over a series of treatments. Depending on the type and severity of the disease, about 1 μg/kg to 15 mg/kg (e.g. 0.1-20 mg/kg) of molecule is an initial candidate dosage for administration to the patient, whether, for example, by one or more separate administrations, or by continuous infusion. A typical daily dosage might range from about 1 μg/kg to 100 mg/kg or more, depending on the factors mentioned above. An exemplary dosage of ADC to be administered to a patient is in the range of about 0.1 to about 10 mg/kg of patient weight. For repeated administrations over several days or longer, depending on the condition, the treatment is sustained until a desired suppression of disease symptoms occurs. An exemplary dosing regimen comprises a course of administering an initial loading dose of about 4 mg/kg, followed by additional doses every week, two weeks, or three weeks of an ADC. Other dosage regimens may be useful. The progress of this therapy is easily monitored by conventional techniques and assays.

***Treatment***

The term "treatment," as used herein in the context of treating a condition, pertains generally to treatment and therapy, whether of a human or an animal (e.g., in veterinary applications), in which some desired therapeutic effect is achieved, for example, the inhibition of the progress of the condition, and includes a reduction in the rate of progress, a halt in the rate of progress, regression of the condition, amelioration of the condition, and cure of the condition. Treatment as a prophylactic measure (i.e., prophylaxis, prevention) is also included.

The term "therapeutically-effective amount," as used herein, pertains to that amount of an active compound, or a material, composition or dosage from comprising an active compound, which is effective for producing some desired therapeutic effect, commensurate with a reasonable benefit/risk ratio, when administered in accordance with a desired treatment regimen.

Similarly, the term "prophylactically-effective amount," as used herein, pertains to that amount of an active compound, or a material, composition or dosage from comprising an active compound, which is effective for producing some desired prophylactic effect, commensurate with a reasonable benefit/risk ratio, when administered in accordance with a desired treatment regimen.

*Preparation of Drug conjugates*

Antibody drug conjugates may be prepared by several routes, employing organic chemistry reactions, conditions, and reagents known to those skilled in the art, including reaction of a nucleophilic group of an antibody with a drug-linker reagent. This method may be employed to prepare the antibody-drug conjugates of the invention.

Nucleophilic groups on antibodies include, but are not limited to side chain thiol groups, e.g. cysteine. Thiol groups are nucleophilic and capable of reacting to form covalent bonds with electrophilic groups on linker moieties such as those of the present invention. Certain antibodies have reducible interchain disulfides, i.e. cysteine bridges. Antibodies may be made reactive for conjugation with linker reagents by treatment with a reducing agent such as DTT (Cleland's reagent, dithiothreitol) or TCEP (tris(2-carboxyethyl)phosphine hydrochloride; Getz et al (1999) Anal. Biochem. Vol 273:73-80; Soltec Ventures, Beverly, MA). Each cysteine disulfide bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol.

***The Subject/Patient***

The subject/patient may be an animal, mammal, a placental mammal, a marsupial (e.g., kangaroo, wombat), a monotreme (e.g., duckbilled platypus), a rodent (e.g., a guinea pig, a hamster, a rat, a mouse), murine (e.g., a mouse), a lagomorph (e.g., a rabbit), avian (e.g., a bird), canine (e.g., a dog), feline (e.g., a cat), equine (e.g., a horse), porcine (e.g., a pig), ovine (e.g., a sheep), bovine (e.g., a cow), a primate, simian (e.g., a monkey or ape), a monkey (e.g., marmoset, baboon), an ape (e.g., gorilla, chimpanzee, orangutang, gibbon), or a human.

Furthermore, the subject/patient may be any of its forms of development, for example, a foetus. In one preferred embodiment, the subject/patient is a human.

## Examples

### General Experimental Methods

Optical rotations were measured on an ADP 220 polarimeter (Bellingham Stanley Ltd.) and concentrations (*c*) are given in g/100mL. Melting points were measured using a digital melting point apparatus (Electrothermal). IR spectra were recorded on a Perkin-Elmer Spectrum 1000 FT IR Spectrometer. $^1H$ and $^{13}C$ NMR spectra were acquired at 300 K using a Bruker Avance NMR spectrometer at 400 and 100 MHz, respectively. Chemical shifts are reported relative to TMS ($\delta$ = 0.0 ppm), and signals are designated as s (singlet), d (doublet), t (triplet), dt (double triplet), dd (doublet of doublets), ddd (double doublet of doublets) or m (multiplet), with coupling constants given in Hertz (Hz). Mass spectroscopy (MS) data were collected using a Waters Micromass ZQ instrument coupled to a Waters 2695 HPLC with a Waters 2996 PDA. Waters Micromass ZQ parameters used were: Capillary (kV), 3.38; Cone (V), 35; Extractor (V), 3.0; Source temperature (°C), 100; Desolvation Temperature (°C), 200; Cone flow rate (L/h), 50; De-solvation flow rate (L/h), 250. High-resolution mass spectroscopy (HRMS) data were recorded on a Waters Micromass QTOF Global in positive W-mode using metal-coated borosilicate glass tips to introduce the samples into the instrument. Thin Layer Chromatography (TLC) was performed on silica gel aluminium plates (Merck 60, $F_{254}$), and flash chromatography utilised silica gel (Merck 60, 230-400 mesh ASTM). Except for the HOBt (NovaBiochem) and solid-supported reagents (Argonaut), all other chemicals and solvents were purchased from Sigma-Aldrich and were used as supplied without further purification. Anhydrous solvents were prepared by distillation under a dry nitrogen atmosphere in the presence of an appropriate drying agent, and were stored over 4Å molecular sieves or sodium wire. Petroleum ether refers to the fraction boiling at 40-60°C.

General LC/MS conditions:

*Method 1 (default method, used unless stated otherwise)*

The HPLC (Waters Alliance 2695) was run using a mobile phase of water (A) (formic acid 0.1%) and acetonitrile (B) (formic acid 0.1%). Gradient: initial composition 5% B held over 1.0 min, then increase from 5% B to 95% B over a 3 min period. The composition was held for 0.1 min at 95% B, then returned to 5% B in 0.03 minutes and hold there for 0.87 min. Total gradient run time equals 5 minutes.

*Method 2*

The HPLC (Waters Alliance 2695) was run using a mobile phase of water (A) (formic acid 0.1%) and acetonitrile (B) (formic acid 0.1%). Gradient: initial composition 5% B held over 1.0 minute, then increase from 5% B to 95% B over a 2.5 minute period. The composition was held for 0.5 minutes at 95% B, then returned to 5% B in 0.1 minutes and hold there for 0.9 min. Total gradient run time equals 5 minutes.

*For both methods*

Flow rate 3.0 mL/min, 400$\mu$L was split *via* a zero dead volume tee piece which passes into the mass spectrometer. Wavelength detection range: 220 to 400 nm. Function type: diode array (535 scans). Column: Phenomenex Onyx Monolithic C18 50 x 4.60 mm.

The reverse phase flash purification conditions were as follows: The Flash purification system (Varian 971-Fp) was run using a mobile phase of water (A) and acetonitrile (B). Gradient: initial composition 5% B over 20 C.V. (Column Volume) then 5% B to 70% B within 60 C.V. The composition was held for 15 C.V. at 95% B, and then returned to 5% B in 5 C.V. and held at 5%B for 10 C.V. Total gradient run time equals 120 C.V. Flow rate 6.0 mL/min. Wavelength detection range: 254 nm. Column: Agilent AX1372-1 SF10-5.5gC8.

Preparative HPLC: Reverse-phase ultra-high-performance liquid chromatography (UPLC) was carried out on Phenomenex Gemini NX 5$\mu$ C-18 columns of the following dimensions: 150 x 4.6 mm for analysis, and 150 x 21.20 mm for preparative work. All UPLC experiments were performed with gradient conditions. Eluents used were solvent A ($H_2O$ with 0.1 % Formic acid) and solvent B ($CH_3CN$ with 0.1% Formic acid). Flow rates used were 1.0 ml/min for analytical, and 20.0 ml/min for preparative HPLC. Detection was at 254 and 280 nm.

### Synthesis of Intermediate 12

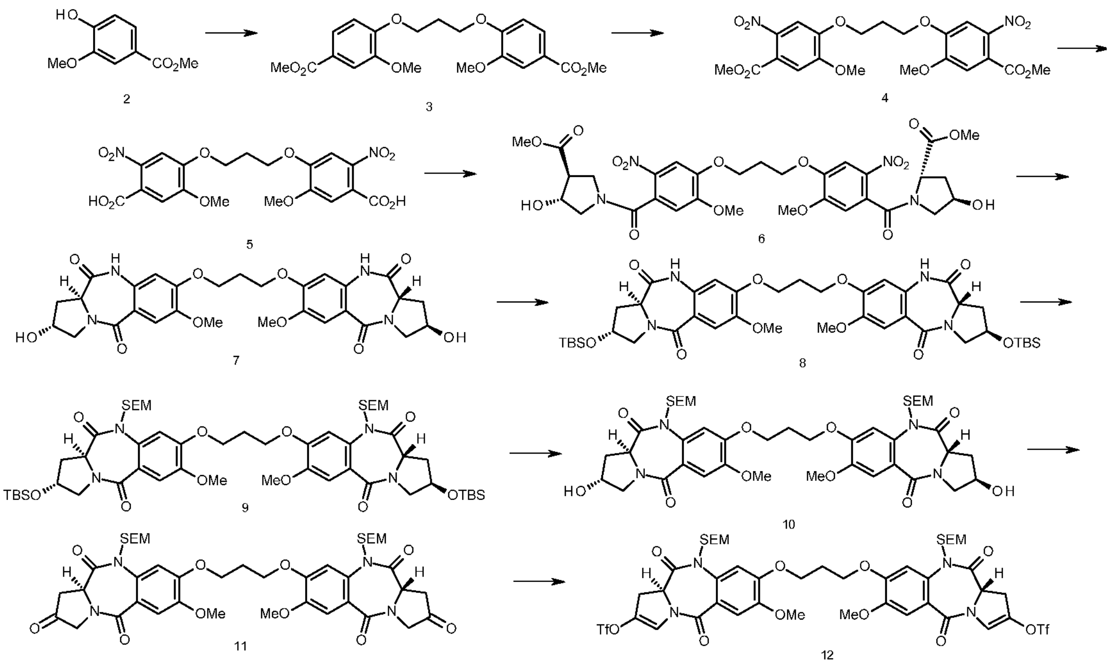

2 3 4 5 6 7 8 9 10 11 12

*(a) 1',3'-Bis[2-methoxy-4-(methoxycarbonyl)phenoxy]propane* ***(3)***

Diisopropyl azodicarboxylate (71.3 mL, 73.2 g, 362 mmol) was added drop-wise over a period of 60 min to an overhead stirred solution of methyl vanillate 2 (60.0 g, 329 mmol) and $Ph_3P$ (129.4 g, 494 mmol) in anhydrous THF (800 mL) at 0-5°C (ice/acetone) under a nitrogen atmosphere. The reaction mixture was allowed to stir at 0-5°C for an additional 1 hour after which time a solution of 1,3-propanediol (11.4 mL, 12.0 g, 158 mmol) in THF (12 mL) was added drop-wise over a period of 20 min. The reaction mixture was allowed to warm to room temperature and stirred for 5 days. The resulting white precipitate **3** was collected by vacuum filtration, washed with THF and dried in a vacuum desiccator to constant weight. Yield = 54.7 g (84% based on 1,3-propanediol). Purity satisfactory by LC/MS (3.20 min (ES+) *m/z* (relative intensity) 427 ([*M* + Na]$^{+\cdot}$, 10); $^1$H NMR (400 MHz, $CDCl_3$) $\delta$ 7.64 (dd, 2H, *J* = 1.8, 8.3 Hz), 7.54 (d, 2H, *J* = 1.8 Hz), 6.93 (d, 2H, *J* = 8.5 Hz), 4.30 (t, 4H, *J* = 6.1 Hz), 3.90 (s, 6H), 3.89 (s, 6H), 2.40 (p, 2H, J = 6.0 Hz).

*(b) 1',3'-Bis[2-methoxy-4-(methoxycarbonyl)-5-nitrophenoxy]propane* ***(4)***

Solid $Cu(NO_3)_2.3H_2O$ (81.5 g, 337.5 mmol) was added slowly to an overhead stirred slurry of the bis-ester 3 (54.7 g, 135 mmol) in acetic anhydride (650 mL) at 0-5°C (ice/acetone). The reaction mixture was allowed to stir for 1 hour at 0-5°C and then allowed to warm to room temperature. A mild exotherm (*ca.* 40-50°C), accompanied by thickening of the mixture and evolution of $NO_2$ was observed at this stage. Additional acetic anhydride (300 mL) was added and the reaction mixture was allowed to stir for 16 hours at room temperature. The reaction mixture was poured on to ice (~ 1.5 L), stirred and allowed to return to room temperature. The resulting yellow precipitate was collected by vacuum filtration and dried in a desiccator to afford the desired *bis*-nitro compound **4** as a yellow solid. Yield = 66.7 g (100%). Purity satisfactory by LC/MS (3.25 min (ES+) *m/z* (relative intensity) 517 ([M + Na]$^{+\cdot}$, 40); $^1$H NMR (400 MHz, $CDCl_3$) $\delta$ 7.49 (s, 2H), 7.06 (s, 2H), 4.32 (t, 4H, *J* = 6.0 Hz), 3.95 (s, 6H), 3.90 (s, 6H), 2.45-2.40 (m, 2H).

*(c) 1',3'-Bis(4-carboxy-2-methoxy-5-nitrophenoxy) propane* ***(5)***

A slurry of the methyl ester **4** (66.7 g, 135 mmol) in THF (700 mL) was treated with 1 N NaOH (700 mL) and the reaction mixture was allowed to stir vigorously at room temperature. After 4 days stirring, the slurry became a dark coloured solution which was subjected to rotary evaporation under reduced pressure to remove THF. The resulting aqueous residue was acidified to pH 1 with concentrated HCl and the colourless precipitate 5 was collected and dried thoroughly in a vacuum oven (50 °C). Yield = 54.5 g (87%). Purity satisfactory by LC/MS (2.65 min (ES+) *m/z* (relative intensity) 489 ([*M* + Na]$^{+\cdot}$, 30)); $^1$H NMR (400 MHz, DMSO-$d_6$) $\delta$ 7.62 (s, 2H), 7.30 (s, 2H), 4.29 (t, 4H, *J* = 6.0 Hz), 3.85 (s, 6H), 2.30-2.26 (m, 2H).

*(d) 1,1'-[[(Propane-1,3-diyl)dioxy]bis[(5-methoxy-2-nitro-1,4-phenylene)carbonyl]]bis[(2S,4R)-methyl-4-hydroxypyrrolidine-2-carboxylate]* ***(6)***

Oxalyl chloride (24.5 mL, 35.6 g, 281 mmol) was added to a stirred suspension of the nitrobenzoic acid **5** (43 g, 92.3 mmol) and DMF (6 mL) in anhydrous DCM (600mL). Following initial effervescence the reaction suspension became a solution and the mixture was allowed to stir at room temperature for 16 hours. Conversion to the acid chloride was confirmed by treating a sample of the reaction mixture with MeOH and the resulting *bis*-methyl ester was observed by LC/MS. The majority of solvent was removed by evaporation under reduced pressure; the resulting concentrated solution was re-dissolved in a minimum amount of dry DCM and triturated with diethyl ether. The resulting yellow precipitate was collected by filtration, washed with cold diethyl ether and dried for 1 hour in a vacuum oven at 40°C. The solid acid chloride was added portionwise over a period of 25 min to a stirred suspension of (2*S*,4*R*)-methyl-4-hydroxypyrrolidine-2-carboxylate hydrochloride (38.1 g, 210 mmol) and TEA (64.5 mL, g, 463 mmol) in DCM (400mL) at -40°C (dry ice/$CH_3CN$). Immediately, the reaction was complete as judged by LC/MS (2.47 min (ES+) *m*/*z* (relative intensity) 721 ([*M* + H]$^{+\cdot}$, 100). The mixture was diluted with DCM (200 mL) and washed with 1N HCl (300 mL), saturated $NaHCO_3$ (300 mL), brine (400 mL), dried ($MgSO_4$), filtered and the solvent evaporated *in vacuo* to give the pure product **6** as an orange solid (66.7 g, 100%). $[\alpha]^{22}_D$ = -46.1° (*c* = 0.47, $CHCl_3$); $^1$H NMR (400 MHz, $CDCl_3$) (rotamers) $\delta$ 7.63 (s, 2H), 6.82 (s, 2H), 4.79-4.72 (m, 2H), 4.49-4.28 (m, 6H), 3.96 (s, 6H), 3.79 (s, 6H), 3.46-3.38 (m, 2H), 3.02 (d, 2H, *J* = 11.1 Hz), 2.48-2.30 (m, 4H), 2.29-2.04 (m, 4H); $^{13}$C NMR (100 MHz, $CDCl_3$) (rotamers) $\delta$ 172.4, 166.7, 154.6, 148.4, 137.2, 127.0, 109.7, 108.2, 69.7, 65.1, 57.4, 57.0, 56.7, 52.4, 37.8, 29.0; IR (ATR, $CHCl_3$) 3410 (br), 3010, 2953, 1741, 1622, 1577, 1519, 1455, 1429, 1334, 1274, 1211, 1177, 1072, 1050, 1008, 871 cm$^{-1}$; MS (ES$^+$) *m*/*z* (relative intensity) 721 ([*M* + H]$^{+\cdot}$, 47), 388 (80); HRMS [*M* + H]$^{+\cdot}$ theoretical $C_{31}H_{36}N_4O_{16}$ *m*/*z* 721.2199, found (ES$^+$) *m*/*z* 721.2227.

*(e) 1, 1 '-[[(Propane-1,3-diyl)dioxy]bis(11aS,2R)-2-(hydroxy)-7-methoxy-1, 2, 3, 10, 11, 11a-hexahydro-5H-pyrrolo[2,1-c][1,4]-benzodiazepin-5,11-dione]* ***(7)***

**Method A:** A solution of the nitro-ester 6 (44 g, 61.1 mmol) in MeOH (2.8 L) was added to freshly purchased Raney® nickel (~ 50 g of a ~ 50% slurry in $H_2O$) and anti-bumping granules in a 5L 3-neck round bottomed flask. The mixture was heated at reflux and then treated dropwise with a solution of hydrazine hydrate (21.6 mL, 22.2 g, 693 mmol) in MeOH (200 mL) at which point vigorous effervescence was observed. When the addition was complete (~ 45 min) additional Raney® nickel was added carefully until effervescence had ceased and the initial yellow colour of the reaction mixture was discharged. The mixture was heated at reflux for a further 5 min at which point the reaction was deemed complete by TLC (90:10 v/v $CHCl_3$/MeOH) and LC/MS (2.12 min (ES+) *m*/*z* (relative intensity) 597 ([*M* + H]$^{+\cdot}$, 100)). The reaction mixture was filtered hot immediately through a sinter funnel containing celite with vacuum suction. The filtrate was reduced in volume by evaporation *in vacuo* at which point a colourless precipitate formed which was collected by filtration and dried in a vacuum desiccator to provide **7** (31 g, 85%). $[\alpha]^{27}_D$ = +404° (*c* = 0.10, DMF); $^1$H NMR (400 MHz, DMSO-*d*$_6$) $\delta$ 10.2 (s, 2H, NH), 7.26 (s, 2H), 6.73 (s, 2H), 5.11 (d, 2H, *J* = 3.98 Hz, O*H*), 4.32-4.27 (m, 2H), 4.19-4.07 (m, 6H), 3.78 (s, 6H), 3.62 (dd, 2H, *J*= 12.1, 3.60 Hz), 3.43 *(dd, 2H, J* = 12.0, 4.72 Hz), 2.67-2.57 (m, 2H), 2.26 (p, 2H, *J* = 5.90 Hz), 1.99-1.89 (m, 2H); $^{13}$C NMR (100 MHz, DMSO-*d*$_6$) b 169.1, 164.0, 149.9, 144.5, 129.8, 117.1, 111.3, 104.5, 54.8, 54.4, 53.1, 33.5, 27.5; IR (ATR, neat) 3438, 1680, 1654, 1610, 1605, 1516, 1490, 1434, 1379, 1263, 1234, 1216, 1177, 1156, 1115, 1089, 1038, 1018, 952, 870 cm$^{-1}$; MS (ES$^+$) *m*/*z* (relative intensity) 619 ([*M* + Na]$^{+\cdot}$, 10), 597 ([*M* + H]$^{+\cdot}$, 52), 445 (12), 326 (11); HRMS [*M* + H]$^{+\cdot}$ theoretical $C_{29}H_{32}N_4O_{10}$ *m*/*z* 597.2191, found (ES$^+$) *m*/*z* 597.2205.

**Method B:** A suspension of 10% Pd/C (7.5 g, 10% w/w) in DMF (40 mL) was added to a solution of the nitro-ester **6** (75 g, 104 mmol) in DMF (360 mL). The suspension was hydrogenated in a Parr hydrogenation apparatus over 8 hours. Progress of the reaction was monitored by LC/MS after the hydrogen uptake had stopped. Solid Pd/C was removed by filtration and the filtrate was concentrated by rotary evaporation under vacuum (below 10mbar) at 40°C to afford a dark oil containing traces of DMF and residual charcoal. The residue was digested in EtOH (500 mL) at 40°C on a water bath (rotary evaporator bath) and the resulting suspension was filtered through celite and washed with ethanol (500 mL) to give a clear filtrate. Hydrazine hydrate (10 mL, 321 mmol) was added to the solution and the reaction mixture was heated at reflux. After 20 minutes the formation of a white precipitate was observed and reflux was allowed to continue for a further 30 minutes. The mixture was allowed to cool down to room temperature and the precipitate was retrieved by filtration, washed with diethyl ether (2:1 volume of precipitate) and dried in a vacuum desiccator to provide **7** (50 g, 81%). Analytical data for method B: Identical to those obtained for Method A (optical rotation, $^1$H NMR, LC/MS and TLC).

*(f) 1,1'-[[(Propane-1,3-diyl)dioxy]bis(11aS,2R)-2-(tert-butyldimethylsilyloxy)-7-methoxy-1,2,3,10,11,11a-hexahydro-5H-pyrrolo[2,1-c][1,4]-benzodiazepin-5,11-dione]* ***(8)***

TBSCl (27.6 g, 182.9 mmol) and imidazole (29.9 g, 438.8 mmol) were added to a cloudy solution of the tetralactam **7** (21.8 g, 36.6 mmol) in anhydrous DMF (400 mL) at 0°C (ice/acetone). The mixture was allowed to stir under a nitrogen atmosphere for 3 hours after which time the reaction was deemed complete as judged by LC/MS (3.90 min (ES+) *m/z* (relative intensity) 825 ([*M* + H]$^{+\cdot}$, 100). The reaction mixture was poured onto ice (~ 1.75 L) and allowed to warm to room temperature with stirring. The resulting white precipitate was collected by vacuum filtration, washed with $H_2O$, diethyl ether and dried in the vacuum desicator to provide pure 8 (30.1 g, 99%). $[\alpha]^{23}_D$ = +234° (*c* = 0.41, $CHCl_3$); $^1$H NMR (400 MHz, $CDCl_3$) δ 8.65 (s, 2H, N*H*), 7.44 (s, 2H), 6.54 (s, 2H), 4.50 (p, 2H, *J* = 5.38 Hz), 4.21-4.10 (m, 6H), 3.87 (s, 6H), 3.73-3.63 (m, 4H), 2.85-2.79 (m, 2H), 2.36-2.29 (m, 2H), 2.07-1.99 (m, 2H), 0.86 (s, 18H), 0.08 (s, 12H); $^{13}$C NMR (100 MHz, $CDCl_3$) δ 170.4, 165.7, 151.4, 146.6, 129.7, 118.9, 112.8, 105.3, 69.2, 65.4, 56.3, 55.7, 54.2, 35.2, 28.7, 25.7, 18.0, -4.82 and -4.86; IR (ATR, $CHCl_3$) 3235, 2955, 2926, 2855, 1698, 1695, 1603, 1518, 1491, 1446, 1380, 1356, 1251, 1220, 1120, 1099, 1033 cm$^{-1}$; MS (ES$^+$) *m/z* (relative intensity) 825 ([*M* + H]$^{+\cdot}$, 62), 721 (14), 440 (38); HRMS [*M* + H]$^{+\cdot}$ theoretical $C_{41}H_{60}N_4O_{10}Si_2$ *m/z* 825.3921, found (ES$^+$) *m/z* 825.3948.

*(g) 1,1'-[[(Propane-1,3-diyl)dioxy]bis(11aS,2R)-2-(tert-butyldimethylsilyloxy)-7-methoxy-10-((2-(trimethylsilyl)ethoxy)methyl)-1,2,3,10,11,11a-hexahydro-5H-pyrrolo[2,1-c][1,4]-benzodiazepin-5,11-dione]* ***(9)***

A solution of *n*-BuLi (68.3 mL of a 1.6 M solution in hexane, 109 mmol) was added dropwise to a stirred suspension of the tetralactam **8** (30.08 g, 36.4 mmol) in anhydrous THF (600 mL) at -30°C (dry ice/ethylene glycol) under a nitrogen atmosphere. The reaction mixture was allowed to stir at this temperature for 1 hour (now a reddish orange colour) at which point a solution of SEMCl (19.3 mL, 18.2 g, 109 mmol) in anhydrous THF (120 mL) was added dropwise. The reaction mixture was allowed to slowly warm to room temperature and was stirred for 16 hours under a nitrogen atmosphere. The reaction was deemed complete as judged by TLC (EtOAc) and LC/MS (4.77 min (ES+) *m/z* (relative intensity) 1085 ([*M* + H]$^{+\cdot}$, 100). The THF was removed by evaporation *in vacuo* and the resulting residue dissolved in EtOAc (750 mL), washed with $H_2O$ (250 mL), brine (250 mL), dried ($MgSO_4$) filtered and evaporated *in vacuo* to provide the crude N10-SEM-protected tetralactam **9** as an oil (max$^m$ 39.5 g, 100%). Product carried through to next step without purification. $[\alpha]^{23}_D$ = +163° (*c* = 0.41, $CHCl_3$); $^1$H NMR (400 MHz, $CDCl_3$) δ 7.33 (s, 2H), 7.22 (s, 2H), 5.47 (d, 2H, *J* = 9.98 Hz), 4.68 (d, 2H, *J* = 9.99 Hz), 4.57 (p, 2H, *J* = 5.77 Hz), 4.29-4.19 (m, 6H), 3.89 (s, 6H), 3.79-3.51 (m, 8H), 2.87-2.81 (m, 2H), 2.41 (p, 2H, *J* = 5.81 Hz), 2.03-1.90 (m, 2H), 1.02-0.81 (m, 22H), 0.09 (s, 12H), 0.01 (s, 18H); $^{13}$C NMR (100 MHz, $CDCl_3$) δ 170.0, 165.7, 151.2, 147.5, 133.8, 121.8, 111.6, 106.9, 78.1, 69.6, 67.1, 65.5, 56.6, 56.3, 53.7, 35.6, 30.0, 25.8, 18.4, 18.1, -1.24, -4.73; IR (ATR, $CHCl_3$) 2951, 1685, 1640, 1606, 1517, 1462, 1433, 1360, 1247, 1127, 1065 cm$^{-1}$; MS (ES$^+$) *m/z* (relative intensity) 1113 ([*M* + Na]$^{+\cdot}$, 48), 1085 ([*M* + H]$^{+\cdot}$, 100), 1009 (5), 813 (6); HRMS [*M* + H]$^{+\cdot}$ theoretical $C_{53}H_{88}N_4O_{12}Si_4$ *m/z* 1085.5548, found (ES$^+$) *m/z* 1085.5542.

*(h) 1,1'-[[(Propane-1,3-diyl)dioxy]bis(11aS,2R)-2-hydroxy-7-methoxy-10-((2-(trimethylsilyl)ethoxy)methyl)-1,2,3,10,11,11a-hexahydro-5H-pyrrolo[2,1-c][1,4]-benzodiazepin-5,11-dione]* ***(10)***

A solution of TBAF (150 mL of a 1.0 M solution in THF, 150 mmol) was added to a stirred solution of the crude bis-silyl ether **9** [84.0 g (max$^m$ 56.8 g), 52.4 mmol] in THF (800 mL) at room temperature. After stirring for 1 hour, analysis of the reaction mixture by TLC (95:5 v/v $CHCl_3$/MeOH) revealed completion of reaction. The THF was removed by evaporation under reduced pressure at room temperature and the resulting residue dissolved in EtOAc (500 mL) and washed with $NH_4Cl$ (300 mL). The combined organic layers were washed with brine (60 mL), dried ($MgSO_4$), filtered and evaporated under reduced pressure to provide the crude product. Purification by flash chromatography (gradient elution: 100% $CHCl_3$ to 96:4 v/v $CHCl_3$/MeOH) gave the pure tetralactam **10** as a white foam (36.0 g, 79%). LC/MS 3.33 min (ES+) *m/z* (relative intensity) 879 ([*M* + Na]$^{+\cdot}$, 100), 857 ([*M* + H]$^{+\cdot}$, 40); $[\alpha]^{23}_D$ = +202°(*c* = 0.34, $CHCl_3$); $^1$H NMR (400 MHz, $CDCl_3$) δ 7.28 (s, 2H), 7.20 (s, 2H), 5.44 (d, 2H, *J* = 10.0 Hz), 4.72 (d, 2H, *J* = 10.0 Hz), 4.61-4.58 (m, 2H), 4.25 (t, 4H, *J* = 5.83 Hz), 4.20-4.16 (m, 2H), 3.91-3.85 (m, 8H), 3.77-3.54 (m, 6H), 3.01 (br s, 2H, O*H*), 2.96-2.90 (m, 2H), 2.38 (p, 2H, *J* = 5.77 Hz), 2.11-2.05 (m, 2H), 1.00-0.91 (m, 4H), 0.00 (s, 18H); $^{13}$C NMR (100 MHz, $CDCl_3$) δ 169.5, 165.9, 151.3, 147.4, 133.7, 121.5, 111.6, 106.9, 79.4, 69.3, 67.2, 65.2, 56.5, 56.2, 54.1, 35.2, 29.1, 18.4, -1.23; IR (ATR, $CHCl_3$) 2956, 1684, 1625, 1604, 1518, 1464, 1434, 1361, 1238, 1058, 1021 cm$^{-1}$; MS (ES$^+$) *m/z* (relative intensity) 885 ([*M*+29]$^{+\cdot}$, 70), 857 ([*M* + H]$^{+\cdot}$, 100), 711 (8), 448 (17); HRMS [*M* + H]$^{+\cdot}$ theoretical $C_{41}H_{60}N_4O_{12}Si_2$ *m/z* 857.3819, found (ES$^+$) *m/z* 857.3826.

*(i) 1,1'-[[(Propane-1,3-diyl)dioxy]bis(11aS)-7-methoxy-2-oxo-10-((2-(trimethylsilyl)ethoxy)methyl)-1,2,3,10,11,11a-hexahydro-5H-pyrrolo[2,1-c][1,4]-benzodiazepin-5,11-dione]* ***(11)***

Diol **10** (25.6 g, 30 mmol, 1 eq.), NaOAc (6.9 g, 84 mmol, 2.8 eq.) and TEMPO (188 mg, 1.2 mmol, 0.04 eq.) were dissolved in DCM (326 mL) under Ar. This was cooled to -8°C (internal temperature) and TCCA (9.7 g, 42 mmol, 1.4 eq.) was added portionwise over 15 minutes. TLC (EtOAc) and LC/MS [3.60 min. (ES+) *m/z* (relative intensity) 854.21 ([M + H]$^{+\cdot}$, 40), (ES-) *m/z* (relative intensity) 887.07 ([M - H + Cl]$^{-\cdot}$, 10)] after 30 minutes indicated that reaction was

complete. Cold DCM (200 mL) was added and the mixture was filtered through a pad of Celite before washing with a solution of saturated sodium hydrogen carbonate/ sodium thiosulfate (1:1 v/v; 200 mL x 2). The organic layer was dried with $MgSO_4$, filtered and the solvent removed *in vacuo* to yield a yellow/orange sponge (25.4 g, 99%). LC/MS [3.60 min. (ES+) *m/z* (relative intensity) 854.21 ($[M + H]^{+\cdot}$, 40); $[\alpha]^{20}_D$ = +291° (*c* = 0.26, $CHCl_3$); $^1H$ NMR (400 MHz, $CDCl_3$) $\delta$ 7.32 (s, 2H), 7.25 (s, 2H), 5.50 (d, 2H, *J* = 10.1 Hz), 4.75 (d, 2H, *J* = 10.1 Hz), 4.60 (dd, 2H, *J* = 9.85, 3.07 Hz), 4.31-4.18 (m, 6H), 3.89-3.84 (m, 8H), 3.78-3.62 (m, 4H), 3.55 (dd, 2H, *J* = 19.2, 2.85 Hz), 2.76 (dd, 2H, *J* = 19.2, 9.90 Hz), 2.42 (p, 2H, *J* = 5.77 Hz), 0.98-0.91 (m, 4H), 0.00 (s, 18H); $^{13}C$ NMR (100 MHz, $CDCl_3$) $\delta$ 206.8, 168.8, 165.9, 151.8, 148.0, 133.9, 120.9, 111.6, 107.2, 78.2, 67.3, 65.6, 56.3, 54.9, 52.4, 37.4, 29.0, 18.4, -1.24; IR (ATR, $CHCl_3$) 2957, 1763, 1685, 1644, 1606, 1516, 1457, 1434, 1360, 1247, 1209, 1098, 1066, 1023 $cm^{-1}$; MS ($ES^+$) *m/z* (relative intensity) 881 ($[M + 29]^{+\cdot}$, 38), 853 ($[M + H]^{+\cdot}$, 100), 707 (8), 542 (12); HRMS $[M + H]^{+\cdot}$ theoretical $C_{41}H_{56}N_4O_{12}Si_2$ *m/z* 853.3506, found ($ES^+$) *m/z* 853.3502.

*(j) 1,1'-[[(Propane-1,3-diyl)dioxy]bis(11aS)-7-methoxy-2-[[(trifluoromethyl)sulfonyl]oxy]-10-((2-(trimethylsilyl)ethoxy)methyl)-1,10,11,11a-tetrahydro-5H-pyrrolo[2,1-c][1,4]-benzodiazepin-5,11-dione] (12)*

Anhydrous 2,6-lutidine (5.15 mL, 4.74 g, 44.2 mmol) was injected in one portion to a vigorously stirred solution of bis-ketone **11** (6.08 g, 7.1 mmol) in dry DCM (180 mL) at -45°C (dry ice/acetonitrile) under a nitrogen atmosphere. Anhydrous triflic anhydride, taken from a freshly opened ampoule (7.2 mL, 12.08 g, 42.8 mmol), was injected rapidly dropwise, while maintaining the temperature at -40°C or below. The reaction mixture was allowed to stir at - 45°C for 1 hour at which point TLC (50/50 v/v n-hexane/EtOAc) revealed the complete consumption of starting material. The cold reaction mixture was immediately diluted with DCM (200 mL) and, with vigorous shaking, washed with water (1 x 100 mL), 5% citric acid solution (1 x 200 mL) saturated $NaHCO_3$ (200 mL), brine (100 mL) and dried ($MgSO_4$). Filtration and evaporation of the solvent under reduced pressure afforded the crude product which was purified by flash column chromatography (gradient elution: 90:10 v/v *n*-hexane/EtOAc to 70:30 v/v n-hexane/EtOAc) to afford bis-enol triflate **12** as a yellow foam (5.5 g, 70%). LC/MS 4.32 min (ES+) *m/z* (relative intensity) 1139 ($[M + Na]^{+\cdot}$, 20); $[\alpha]^{24}_D$ = +271° (*c* = 0.18, $CHCl_3$); $^1H$ NMR (400 MHz, $CDCl_3$) $\delta$ 7.33 (s, 2H), 7.26 (s, 2H), 7.14 (t, 2H, *J* = 1.97 Hz), 5.51 (d, 2H, *J* = 10.1 Hz), 4.76 (d, 2H, *J* = 10.1 Hz), 4.62 (dd, 2H, *J* = 11.0, 3.69 Hz), 4.32-4.23 (m, 4H), 3.94-3.90 (m, 8H), 3.81-3.64 (m, 4H), 3.16 (ddd, 2H, *J* = 16.3, 11.0, 2.36 Hz), 2.43 (p, 2H, *J* = 5.85 Hz), 1.23-0.92 (m, 4H), 0.02 (s, 18H); $^{13}C$ NMR (100 MHz, $CDCl_3$) $\delta$ 167.1, 162.7, 151.9, 148.0, 138.4, 133.6, 120.2, 118.8, 111.9, 107.4, 78.6, 67.5, 65.6, 56.7, 56.3, 30.8, 29.0, 18.4, -1.25; IR (ATR, $CHCl_3$) 2958, 1690, 1646, 1605, 1517, 1456, 1428, 1360, 1327, 1207, 1136, 1096, 1060, 1022, 938, 913 $cm^{-1}$; MS ($ES^+$) *m/z* (relative intensity) 1144 ($[M + 28]^{+\cdot}$, 100), 1117 ($[M + H]^{+\cdot}$, 48), 1041 (40), 578 (8); H RMS $[M + H]^{+\cdot}$ theoretical $C_{43}H_{54}N_4O_{16}Si_2S_2F_6$ *m/z* 1117.2491, found ($ES^+$) *m/z* 1117.2465.

**Example 1**

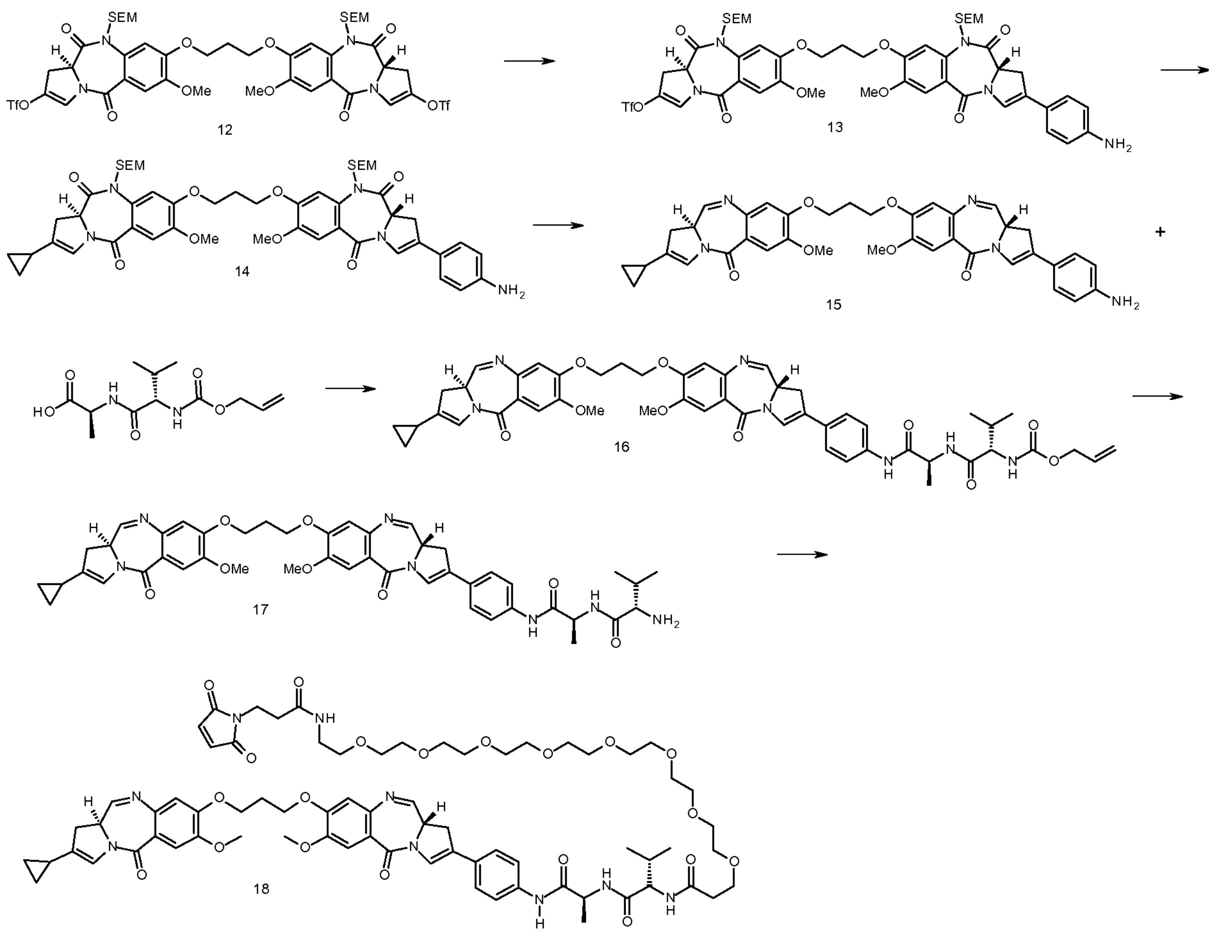

12 13 14 15 + 16 17 18

*(a) (S)-8-(3-(((S)-2-(4-aminophenyl)-7-methoxy-5,11-dioxo-10-((2-(trimethylsilyl)ethoxy)methyl)-5,10,11,11a-tetrahydro-1H-benzo[e]pyrrolo[1,2-a][1,4]diazepin-8-yl)oxy)propoxy)-7-methoxy-5,11-dioxo-10-((2-(trimethylsilyl)ethoxy)methyl)-5,10,11,11a-tetrahydro-1H-benzo[e]pyrrolo[1,2-a][1,4jdiazepin-2-yl trifluoromethanesulfonate (13)*

$Pd(PPh_3)_4$ (116.9 mg, 0.101 mmol) was added to a stirred mixture of the bis-enol triflate 12 (5.65 g, 5.06 mmol), 4-Aminophenylboronic acid pinacol ester (1 g, 4.56 mmol), $Na_2CO_3$ (2.46 g, 23.2 mmol), MeOH (37 mL), toluene (74 mL) and water (37 mL). The reaction mixture was allowed to stir at 30°C under a nitrogen atmosphere for 24 hours after which time all the boronic ester has consumed. The reaction mixture was then evaporated to dryness before the residue was taken up in EtOAc (150 mL) and washed with $H_2O$ (2 x 100 mL), brine (150 mL), dried ($MgSO_4$), filtered and evaporated under reduced pressure to provide the crude product. Purification by flash chromatography (gradient elution: 80:20 v/v Hexane/EtOAc to 60:40 v/v Hexane/EtOAc) afforded product **13** as a yellowish foam (2.4 g, 45%). LC/MS 4.02 min (ES+) *m/z* (relative intensity) 1060.21 ([*M*+ H]$^{+\cdot}$, 100); $^1$H-NMR: ($CDCl_3$, 400 MHz) $\delta$ 7.40 (s, 1H), 7.33 (s, 1 H), 7.27 (bs, 3H), 7.24 (d, 2H, *J* = 8.5 Hz), 7.15 (t, 1H, *J* = 2.0 Hz), 6.66 (d, 2H, *J* = 8.5 Hz), 5.52 (d, 2H, *J* = 10.0 Hz), 4.77 (d, 1H, *J* = 10.0 Hz), 4.76 (d, 1H, *J* = 10.0 Hz), 4.62 (dd, 1H, *J* = 3.7, 11.0 Hz), 4.58 (dd, 1H, *J* = 3.4, 10.6 Hz), 4.29 (t, 4H, *J* = 5.6 Hz), 4.00-3.85 (m, 8H), 3.80 - 3.60 (m, 4H), 3.16 (ddd, 1H, *J* = 2.4, 11.0, 16.3 Hz), 3.11 (ddd, 1H, J = 2.2, 10.5, 16.1 Hz), 2.43 (p, 2H, J = 5.9 Hz), 1.1-0.9 (m, 4H), 0.2 (s, 18H). $^{13}$C-NMR: ($CDCl_3$, 100 MHz) $\delta$ 169.8, 168.3, 164.0, 162.7, 153.3, 152.6, 149.28, 149.0, 147.6, 139.6, 134.8, 134.5, 127.9, 127.5, 125.1, 123.21, 121.5, 120.5, 120.1, 116.4, 113.2, 108.7, 79.8, 79.6, 68.7, 68.5, 67.0, 66.8, 58.8, 58.0, 57.6, 32.8, 32.0, 30.3, 19.7, 0.25.

*(b) (S)-2-(4-Aminophenyl)-8-(3-(((S)-2-cyclopropyl-7-methoxy-5,11-dioxo-10-((2-(trimethylsilyl)ethoxy)methyl)-5,10,11,11a-tetrahydro-1H-benzo[e]pyrrolo[1,2-a][1,4]diazepin-8-yl)oxy)propoxy)-7-methoxy-10-((2-(trimethylsilyl)ethoxy)methyl)-1H-benzo[e]pyrrolo[1,2-a][1,4]diazepine-5,11(10H,11aH)-dione **(14)***

Triphenylarsine (0.24 g, 0.8 mmol), silver (I) oxide (1.02 g, 4.4 mmol), cyclopropylboronic acid (0.47 g, 5.5 mmol) and starting material **13** (1.15 g, 1.1 mmol) were dissolved in dioxane (30 mL) under an argon atmosphere. Potassium phosphate tribasic (2.8 g, 13.2 mmol) was ground-up with a pestle and mortar and quickly added to the reaction mixture. The reaction mixture was evacuated and flushed with argon 3 times and heated to 71 °C. Palladium

(II) *bis* (benzonitrile chloride) (84 mg, 0.22 mmol) was added and the reaction vessel was evacuated and flushed with argon 3 times. After 10 minutes a small sample was taken for analysis by TLC (80:20 v/v ethyl acetate/hexane) and LC/MS. After 30 minutes the reaction had gone to completion (LC/MS analysis indicated complete consumption of starting material) and the reaction was filtered through celite and the filter pad washed with ethyl acetate (400 mL). The filtrate was washed with water (2 x 200 mL) and brine (2 x 200 mL). The organic layer was dried with $MgSO_4$, filtered and the solvent removed *in vacuo.* Purification by silica gel column chromatography (30:70 v/v Hexane/ Ethyl acetate) afforded the product **14** as an orangey/yellow solid (0.66 g, 63%). Method 1, LC/MS (3.85 min ($ES^+$) *m/z* (relative intensity) 952.17 ($[M + H]^{+\cdot}$, 100). $^1H$ NMR (400 MHz, $CDCl_3$) $\delta$ 7.36 (d, 2H, *J* = 8.4 Hz), 7.30 (s, 1 H), 7.25 - 7.19 (m, 4H), 6.68 (s, 1 H), 6.62 (d, 2H, *J* = 8.4 Hz), 5.49 (dd, 2H, *J* = 5.6, 10.0 Hz), 4.73 (app. t, 2H, *J* = 10.8 Hz), 4.54 (dd, 1 H, *J* = 3.2, 10.4 Hz), 4.40 (dd, 1H, *J* = 3.2, 10.4 Hz), 4.29 - 4.23 (m, 4H), 3.91 - 3.85 (m, 7H), 3.80 - 3.71 (m, 2H), 3.70 - 3.61 (m, 2H), 3.38 - 3.32 (m, 1H), 3.12 - 3.01 (m, 1 H), 2.50 - 2.69 (m, 1H), 2.40 (q, 2H, *J* = 5.6 Hz), 1.50 - 1.43 (m, 1H), 0.99 - 0.71 (m, 6H), 0.54 - 0.59 (m, 2H), 0.00 (s, 18H) ppm.

*(c) (S)-2-(4-Aminophenyl)-8-(3-(((S)-2-cyclopropyl-7-methoxy-5-oxo-5,11a-dihydro-1H-benzo[e]pyrrolo[1,2-a][1,4]diazepin-8-yl)oxy)propoxy)-7-methoxy-1H-benzo[e]pyrrolo[1,2-a][1,4]diazepin-5(11aH)-one* ***(15)***

SEM dilactam **14** (0.66 g, 0.69 mmol) was dissolved in THF (23 mL) and cooled to -78°C under an argon atmosphere. Super-Hydride® solution (1.7 mL, 1 M in THF) was added drop wise over 5 minutes while monitoring the temperature. After 20 minutes a small sample was taken and washed with water for LC/MS analysis. Water (50 mL) was added and the cold bath was removed. The organic layer was extracted and washed with brine (60 mL). The combined aqueous layers were washed with $CH_2Cl_2$/MeOH (90/10 v/v) (2 x 50 mL). The combined organic layers were dried with $MgSO_4$, filtered and the solvent removed *in vacuo*. The crude product was dissolved in MeOH (48 mL), $CH_2Cl_2$ (18 mL) and water (6 mL) and sufficient silica gel was added to afford a thick suspension. After 5 days stirring, the suspension was filtered through a sintered funnel and washed with $CH_2Cl_2$/MeOH (9:1) (~ 200 mL) until product ceased to be eluted. The organic layer was washed with brine (2 x 70 mL), dried with $MgSO_4$, filtered and the solvent removed *in vacuo.* Purification by silica gel column chromatography (100% $CHCl_3$ to 96/4 v/v $CHCl_3$/MeOH) afforded the product **15** as a yellow solid (302 mg, 66%). Method 1, LC/MS (2.42 min ($ES^+$) *m/z* (relative intensity) 660.74 ($[M + H]^{+\cdot}$, 30). $^1H$ NMR (400 MHz, $CDCl_3$) $\delta$ 7.86 (d, 1H, *J* = 3.6 Hz), 7.78 (d, 1H, *J* = 3.6 Hz), 7.58 - 7.44 (m, 3H), 7.34 - 7.20 (m, 3H), 6.88 - 6.66 (m, 4H), 4.35 - 4.15 (m, 6H), 3.95 - 3.75 (m, 7H), 3.39 - 3.22 (m, 1H), 3.14 - 3.04 (m, 1H), 2.93 - 2.85 (m, 1H), 2.46 - 2.36 (m, 2H), 1.49 - 1.41 (m, 1H), 0.80 - 0.72 (m, 2H), 0.58 - 0.51 (app. s, 2H) ppm.

*(d) Allyl ((2S)-1-(((2S)-1-((4-(B-(3-((2-cyclopropyl-7-methoxy-5-oxo-5,11a-dihydro-1H-benzo[e]pyrrolo[1,2-a][1,4]diazepin-8-yl)oxy)propoxy)-7-methoxy-5-oxo-5,11a-dihydro-1H-benzo[e]pyrrolo[1,2-a][1,4]diazepin-2-yl)phenyl)amino)-1-oxopropan-2-yl)amino)-3-methyl-1-oxobutan-2-yl)carbamate* ***(16)***

In a degassed round bottom flask filled with argon, HO-Ala-Val-alloc (149.6 mg, 0.549 mmol) and EEDQ (135.8 mg, 0.549 mmol) were dissolved in a 9:1 mixture of dry $CH_2Cl_2$/MeOH (5 mL). The flask was wrapped in aluminium foil and the reaction mixture was allowed to stir at room temperature for 1 hour before starting material **15** (302 mg, 0.457 mmol) was added. The reaction mixture was left to stir for a further 40 hours at room temperature before the volatiles were removed by rotary evaporation under reduced pressure (the reaction was followed by LC/MS, RT starting material 2.32 min, ($ES^+$ 660.29 ($[M+H]^{+\cdot}$,100)). The crude product was directly purified by silica gel chromatography column (100% $CHCl_3$ to 90/10 v/v $CHCl_3$/MeOH) to afford the pure product (**16**) in 42% yield (174 mg). Method 2 LC/MS (2.70 min (ES+) *m/z* (relative intensity) 914.73 ($[M+H]^{+\cdot}$, 60), 660.43 (60), 184.31 (100)).

*(e) (2S)-2-amino-N-((2S)-1-((4-(8-(3-((2-cyclopropyl-7-methoxy-5-oxo-5,11a-dihydro-1H-benzo[e]pyrrolo[1,2-a][1,4]diazepin-8-yl)oxy)propoxy)-7-methoxy-5-oxo-5,11a-dihydro-1H-benzo[e]pyrrolo[1,2-a][1,4]diazepin-2-yl)phenyl)amino)-1-oxopropan-2-yl)-3-methylbutanamide (17)*

The starting material **16** (170 mg, 0.185 mmol) was dissolved in dry $CH_2Cl_2$ (5 mL) in a round bottom flask filled with argon, before pyrrolidine (41 $\mu$L, 0.21 mmol) was added. The flask was purged/refilled three times with argon before $Pd(PPh_3)_4$ (14 mg, 0.084 mmol) was added and the flushing operation repeated. After 1 hour, complete consumption of starting material was observed (the reaction was followed by LC/MS) and $Et_2O$ (50 mL) was added to the reaction mixture which was allowed to stir until all the product had crashed out of solution. The solid was filtered through a sintered funnel and washed twice with $Et_2O$ (2 x 25 mL). The collecting flask was replaced and the isolated solid was dissolved in $CHCl_3$ (100 mL or until all the product had passed through the sintered funnel). The volatiles were then removed by rotary evaporation under reduced pressure to afford the crude product **17** which was used directly in the next step (168 mg). LC/MS method 2 (2.70 min (ES+) *m/z* (relative intensity) 830.27 ($[M+H]^{+\cdot}$, 50), 660.13 (80), 171.15 (100)).

*(f) N-((R)-1-(((S)-1-((4-((S)-8-(3-(((S)-2-cyclopropyl-7-methoxy-5-oxo-5,11a-dihydro-1H-benzo[e]pyrrolo[1,2-a][1,4]diazepin-8-yl)oxy)propoxy)-7-methoxy-5-oxo-5,11a-dihydro-1H-benzo[e]pyrrolo[1,2-a][1,4]diazepin-2-yl)phenyl)amino)-1-oxopropan-2-yl)amino)-3-methyl-1-oxobutan-2-yl)-1-(3-(2,5-dioxo-2,5-dihydro-1H-pyrrol-1-yl)propanamido)-3,6,9,12,15,18,21,24-octaoxaheptacosan-27-amide* ***(18)***

Starting material 17 (154 mg, 0.185 mmol) and EDCI.HCl (110 mg, 0.185 mmol) were solubilised in dry $CH_2Cl_2$ (5 mL) in a round bottom flask purged and filled with argon. The mixture was left to stir at room temperature for 1 hour before $PEG_8$-maleimide (35.6 mg, 0.185 mmol) was added and the reaction mixture stirred for a further 16 hours (or until the reaction is complete, monitered by LC/MS). The reaction solution was diluted with $CH_2Cl_2$ (50 mL) and the organics were washed with $H_2O$ (50 mL) and brine (50 mL) before being dried with $MgSO_4$, filtered and the solvent removed by rotary evaporation under reduced pressure to afford the crude product. Purification on silica gel column chromatography (100% $CHCl_3$ to 85/15 v/v $CHCl_3$/MeOH) gave the desired product (135mg), however remaining traces of unreacted $PEG_8$-maleimide were observed (by LC/MS, 2.21 min, method 2). Automated reverse phase silica gel chromatography ($H_2O/CH_3CN$) (see general information for conditions) successfully removed the impurity affording pure final product (**18**, 37mg of pure product starting from 110mg, 33%). Overall yield = 17%. Method 2 LC/MS (2.58 min (ES+) *m/z* (relative intensity) 1404.03 ($[M+H]^{+\cdot}$, 20), 702.63 (100)). $^1H$ NMR (400 MHz, $CDCl_3$) $\delta$ 7.91 (t, *J* = 3.5 Hz, 1H), 7.80 (d, *J* = 4.0 Hz, 1H), 7.75 (d, *J* = 8.8 Hz, 1H), 7.69 (d, *J* = 8.7 Hz, 1 H), 7.54 - 7.50 (m, 2H), 7.45 (s, 1 H), 7.39 - 7.31 (m, 2H), 6.87 (d, *J* = 10.5 Hz, 2H), 6.76 (s, 1H), 6.72 - 6.68 (m, 2H), 4.74 - 4.62 (m, 1H), 4.45 - 4.17 (m, 7H), 3.95 (s, 3H), 3.94 (s, 3H), 3.67 - 3.58 (m, 34H), 3.54 (m, 2H), 3.42 (dd, J = 10.2, 5.2 Hz, 2H), 3.16 - 3.07 (m, 1H), 2.92 (dd, J = 16.1, 4.1 Hz, 1H), 2.62 - 2.49 (m, 4H), 2.48 - 2.39 (m, 2H), 2.37 - 2.25 (m, 1H), 1.92 (s, 1H), 1.52 - 1.44 (m, 3H), 1.10 - 0.93 (m, 6H), 0.79 (dd, *J* = 9.2, 5.3 Hz, 2H), 0.57 (dd, *J* = 9.2, 5.3 Hz, 2H), NH were not observed.

**Example 2**

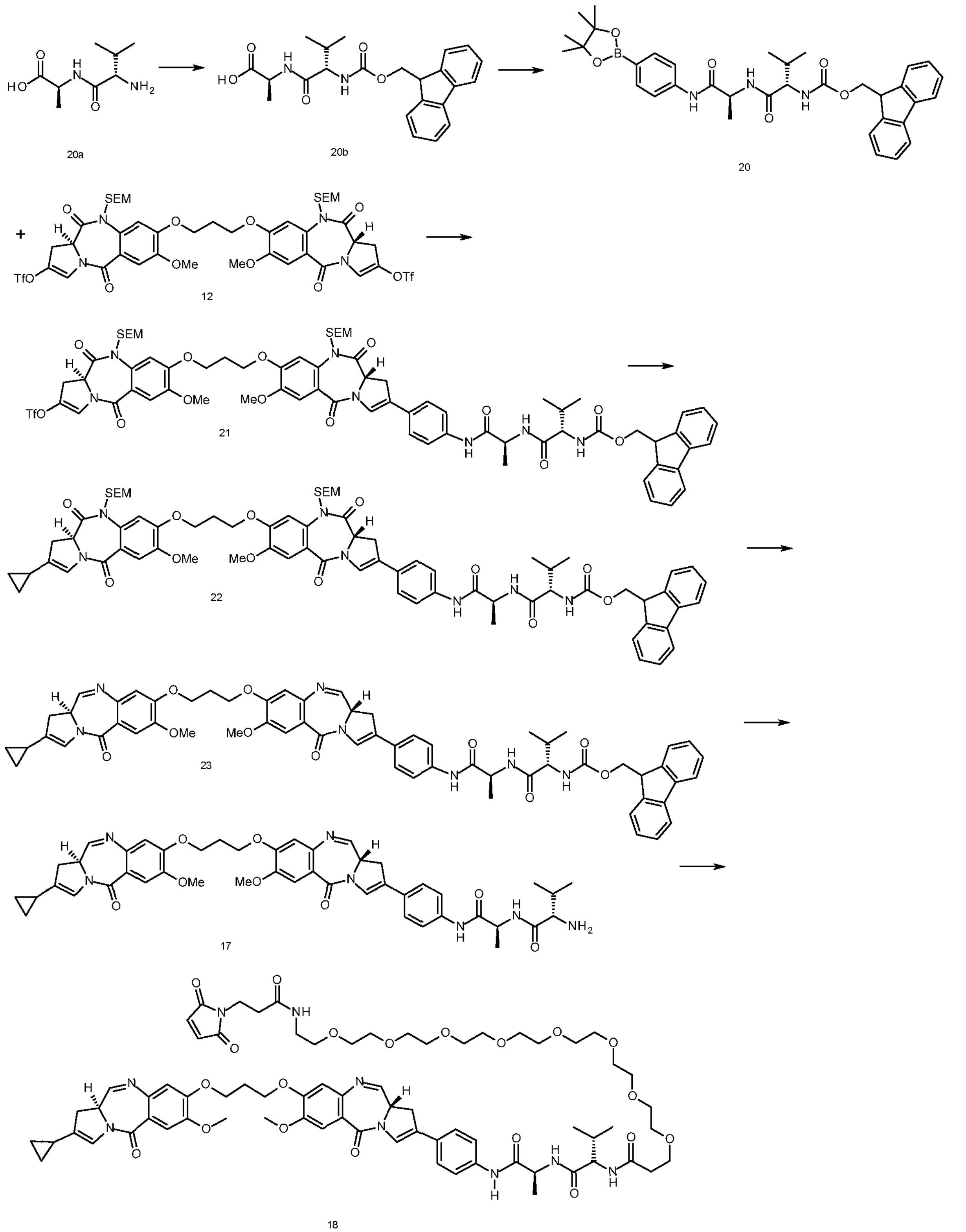

20a 20b 20

12

21

22

23

17

18

*(a) (R)-2-((R)-2-((((9H-fluoren-9-yl)methoxy)carbonyl)amino)-3-methylbutanamido) propanoic acid* ***(20b)***

HO-Ala-Val-H **20a** (350 mg, 1.86 mmol) and $Na_2CO_3$ (493 mg, 4.65 mmol) were dissolved in distilled $H_2O$ (15 mL) and the mixture was cooled to 0°C before dioxane (15 mL) was added (partial precipitation of the amino acid salt occurred). A solution of Fmoc-Cl (504 mg, 1.95 mmol) in dioxane (15 mL) was added dropwise with vigorous stirring over 10 minutes. The resulting mixture was stirred at 0°C for 2 hours before the ice bath was removed and stirring was maintained for 16 hours. The solvent was removed by rotary evaporation under reduced pressure and the residue

dissolved in water (150 mL). The pH was adjusted from 9 to 2 with 1 N HCl and the aqueous layer was subsequently extracted with EtOAc (3x100 mL). The combined organics were washed with brine (100 mL), dried with $MgSO_4$, filtered and the volatiles removed by rotary evaporation under reduced pressure to afford pure HO-Ala-Val-Fmoc 20b (746 mg, 97% yield). LC/MS 2.85 min (ES+) *m/z* (relative intensity) 410.60; $^1$H-NMR (400 MHz, $CDCl_3$) $\delta$ 7.79 (d, *J*=7.77 Hz, 2H), 7.60(d, *J*=7.77 Hz, 2H), 7.43(d, *J*=7.5 Hz, 2H), 7.34 (d, *J*=7.5 Hz, 2H), 6.30 (bs, 1H), 5.30 (bs, 1H), 4.71-7.56 (m, 1H), 4.54-4.36 (m, 2H), 4.08-3.91 (m, 1H), 2.21-2.07 (m, 1H), 1.50 (d, *J*=7.1 Hz, 3H), 1.06-0.90 (m, 6H).

*(b) (9H-fluoren-9-yl)methyl ((S)-3-methyl-1-oxo-1-(((S)-1-oxo-1-((4-(4,4,5,5-tetramethyl-1,3,2-dioxaborolan-2-yl)phenyl)amino)propan-2-yl)amino)butan-2-yl)carbamate* ***(20)***

4-Aminophenylboronic acid pinacol ester was added (146.9 mg, 0.67 mmol) was added to a solution of HO-Ala-Val-Fmoc **20b** (330mg, 0.8 mmol), DCC (166 mg, 0.8 mmol) and DMAP (5 mg, cat.) in dry DCM (8 mL) previously stirred for 30 minutes at room temperature in a flask flushed with argon. The reaction mixture was then allowed to stir at room temperature overnight. The reaction was followed by LCMS and TLC. The reaction mixture was diluted with $CH_2Cl_2$ and the organics were washed with $H_2O$ and brine before being dried with $MgSO_4$, filtered and the solvent removed by rotary evaporation under reduced pressure. The crude product was dryloaded on a silicagel chromatography column (Hexane/EtOAc, 6:4) and pure product **20** was isolated as a white solid in 88% yield (360 mg).

*(c) 8-(3-((2-(4-((S)-2-((S)-2-((((9H-fluoren-9-yl)methoxy)carbonyl)amino)-3-methylbutanamido)propanamido)phenyl)-7-methoxy-5,11-dioxo-10-((2-(trimethylsilyl)ethoxy)methyl)-5,10,11,11a-tetrahydro-1H-benzo[e]pyrrolo[1,2-a][1,4]diazepin-8-yl)oxy)propoxy)-7-methoxy-5,11-dioxo-10-((2-(trimethylsilyl)ethoxy)methyl)-5,10,11,11a-tetrahydro-1H-benzo[e]pyrrolo[1,2-a][1,4]diazepin-2-yl trifluoromethanesulfonate* ***(21)***

Bis-triflate **12** (2.03g, 1.81 mmol), boronic pinacol ester (1 g, 1.63 mmol) and $Na_2CO_3$ (881 mg, 8.31 mmol) were dissolved in a mixture of toluene/MeOH/$H_2O$, 2:1:1 (40 mL). The reaction flask was purged and filled with argon three times before *tetrakis*(triphenylphosphine)palladium(0) (41 mg, 0.035 mmol) was added and the reaction mixture heated to 30°C overnight. The solvents were removed under reduce pressure and the residue was taken up in $H_2O$ (100 mL) and extracted with EtOAc (3 x 100 mL). The combined organics were washed with brine (100 mL), dried with $MgSO_4$, filtered and the volatiles removed by rotary evaporation under reduced pressure. The crude product was purified by silica gel chromatography column (Hexane/EtOAc, 8:2 to 25:75) to afford pure **21** in 33% yield (885 mg). LC/MS 3.85 min (ES+) *m/z* (relative intensity) 1452.90 ; $^1$H NMR (400 MHz, $CDCl_3$) $\delta$ 7.78 - 7.16 (m, 17H), 7.13 (s, 1H), 6.51 - 6.24 (m, 1 H), 5.51 (dd, J = 10.0, 5.1 Hz, 2H), 5.36 - 5.11 (m, 1H), 4.74 (dd, *J* = 10.1, 4.4 Hz, 2H), 4.70 - 4.53 (m, 2H), 4.47 (d, *J* = 6.4 Hz, 1 H), 4.37 (d, *J* = 7.2 Hz, 1H), 4.27 (m, 4H), 4.20 - 4.14 (m, 1H), 3.90 (s, 3H), 3.89 (s, 3H), 3.77 (ddd, J = 16.7, 9.0, 6.4 Hz, 3H), 3.71 - 3.61 (m, 2H), 3.24 - 2.91 (m, 3H), 2.55 - 2.33 (m, 2H), 2.22 - 2.07 (m, 1H), 1.52 - 1.37 (m, 3H), 1.04 - 0.86 (m, 10H), 0.00 (s, 18H).

*(d) (9H-fluoren-9-yl)methyl((2S)-1-(((2S)-1-((4-(8-(3-((2-cyclopropyl-7-methoxy-5,11-dioxo-10-((2-(trimethylsilyl)ethoxy)methyl)-5,10,11,11a-tetrahydro-1H-benzo[e]pyrrolo[1,2-a][1,4]diazepin-8-yl)oxy)propoxy)-7-methoxy-5,11-dioxo-10-((2-(trimethylsilyl)ethoxy)methyl)-5,10,11,11a-tetrahydro-1H-benzo[e]pyrrolo[1,2-a][1,4]diazepin-2-yl)phenyl)amino)-1-oxopropan-2-yl)amino)-3-methyl-1-oxobutan-2-yl)carbamate (22)*

Triphenylarsine (42 mg, 0.137 mmol) was added to a mixture of PBD-triflate **21** (250 mg, 0.172 mmol), cyclopropylboronic acid (73.9 mg, 0.86 mmol), silver oxide (159 mg, 0.688 mmol) and potassium phosphate tribasic (438 mg, 2.06 mmol) in dry dioxane (10 mL) under an argon atmosphere. The reaction was flushed with argon 3 times and *bis*(benzonitrile)palladium(II) chloride (13.2 mg, 0.034 mmol) was added. The reaction was flushed with Argon 3 more times before being warmed to 75°C and stirred for 10 minutes. The reaction mixture was filtered through a pad of celite which was subsequently rinsed with ethyl acetate. The solvent was removed by rotary evaporation under reduced pressure. The resulting residue was subjected to flash column chromatography (silica gel; 1 % methanol/chloroform). Pure fractions were collected and combined, and excess eluent was removed by rotary evaporation under reduced pressure to afford the desired product 22 (132 mg, 50 % yield). LC/MS 3.83 min (ES+) *m/z* (relative intensity) 1345.91 ; $^1$H NMR (400 MHz, $CDCl_3$) $\delta$ 7.88 - 7.14 (m, 17H), 6.69 (s, 1H), 6.45 - 6.25 (m, 1H), 5.57 - 5.41 (m, 2H), 5.34 - 5.14 (m, 1H), 4.78 - 4.67 (m, 2H), 4.62 - 4.55 (m, 1H), 4.50 - 4.45 (m, 2H), 4.51 - 4.44 (m, 1 H), 4.31 - 4.21 (m, 4H), 4.16 (m, 1H), 3.92 (s, 3H), 3.86 (s, 3H), 3.82 - 3.71 (m, 2H), 3.66 (m, 3H), 3.40 - 3.28 (m, 1H), 3.07 (m, 1H), 2.70 - 2.57 (m, 1H), 2.47 - 2.36 (m, 2H), 2.15 (m, 1 H), 1.51 - 1.40 (m, 3H), 1.03 - 0.87 (m, 11H), 0.77 - 0.71 (m, 2H), 0.60 - 0.54 (m, 2H), 0.00 (t, *J* = 3.0 Hz, 18H).

*(e) (9H-fluoren-9-yl)methyl((2S)-1-(((2S)-1-((4-(8-(3-((2-cyclopropyl-7-methoxy-5-oxo-5,11a-dihydro-1H-benzo[e]pyrrolo[1,2-a][1,4]diazepin-8-yl)oxy)propoxy)-7-methoxy-5-oxo-5,11a-dihydro-1H-benzo[e]pyrrolo[1,2-a][1,4]diazepin-2-yl)phenyl)amino)-1-oxopropan-2-yl)amino)-3-methyl-1-oxobutan-2-yl)carbamate **(23)***

A solution of Super-Hydride® (0.5 mL, 1 M in THF) was added dropwise to a solution of SEM dilactam 22 (265 mg g, 0.19 mmol) in THF (10 mL) at -78°C under an argon atmosphere. The addition was completed over 5 minutes in order to maintain the internal temperature of the reaction mixture constant. After 20 minutes, an aliquot was quenched with water for LC/MS analysis, which revealed that the reaction was complete. Water (20 mL) was added to the reaction mixture and the cold bath was removed. The organic layer was extracted with EtOAc (3 x 30 mL) and the combined organics were washed with brine (50 mL), dried with $MgSO_4$, filtered and the solvent removed by rotary evaporation under reduced pressure. The crude product was dissolved in MeOH (12 mL), $CH_2Cl_2$ (6 mL), water (2 mL) and enough silica gel to form a thick stirring suspension. After 5 days, the suspension was filtered through a sintered funnel and washed with $CH_2Cl_2$/MeOH (9:1) (200 mL) until the elution of the product was complete. The organic layer was washed with brine (2 x 70 mL), dried with $MgSO_4$, filtered and the solvent removed by rotary evaporation under reduced pressure. Purification by silica gel column chromatography (100% $CHCl_3$ to 96% $CHCl_3$/ 4% MeOH) afforded the product **23** as a yellow solid (162 mg, 78%). LC/MS 3.02 min (ES+) *m*/*z* (relative intensity) 1052.37.

*(f) (2S)-2-amino-N-((2S)-1-((4-(8-(3-((2-cyclopropyl-7-methoxy-5-oxo-5,11a-dihydro-1H-benzo[e]pyrrolo[1,2-a][1,4]diazepin-8-yl)oxy)propoxy)-7-methoxy-5-oxo-5,11a-dihydro-1H-benzo[e]pyrrolo[1,2-a][1,4]diazepin-2-yl)phenyl)amino)-1-oxopropan-2-yl)-3-methylbutanamide **(17)***

Excess piperidine was added (0.2 mL, 2 mmol) to a solution of SEM-dilactam **23** (76 mg, 0.073 mmol) in DMF (1 mL). The mixture was allowed to stir at room temperature for 20 min, at which point the reaction had gone to completion (as monitored by LC/MS). The reaction mixture was diluted with $CH_2Cl_2$ (75 mL) and the organic phase was washed with $H_2O$ (3x75 mL) until complete piperidine removal. The organic phase was dried over $MgSO_4$, filtered and excess solvent removed by rotary evaporation under reduced pressure to afford crude product **17** which was used as such in the next step. LC/MS 2.32 min (ES+) *m*/*z* (relative intensity) 830.00.

*(g) N-((2S)-1-(((2S)-1-((4-(8-(3-((2-cyclopropyl-7-methoxy-5-oxo-5,11a-dihydro-1H-benzo[e]pyrrolo[1,2-a][1,4]diazepin-8-yl)oxy)propoxy)-7-methoxy-5-oxo-5,11a-dihydro-1H-benzo[e]pyrrolo[1,2-a][1,4]diazepin-2-yl)phenyl)amino)-1-oxopropan-2-yl)amino)-3-methyl-1-oxobutan-2-yl)-1-(3-(2,5-dioxo-2,5-dihydro-1H-pyrrol-1-yl)propanamido)-3,6,9,12,15,18,21,24-octaoxaheptacosan-27-amide **(18)***

EDCI hydrochloride (14 mg, 0.0732 mmol) was added to a suspension of Maleimide-$PEG_8$-acid (43.4 mg, 0.0732 mmol) in dry $CH_2Cl_2$ (5 mL) under argon atmosphere. The mixture was stirred for 1 hour at room temperature before PBD **17** (60.7 mg, 0.0732 mmol) was added. Stirring was maintained until the reaction was complete (usually 5 hours). The reaction was diluted with $CH_2Cl_2$ and the organic phase was washed with $H_2O$ and brine before being dried over $MgSO_4$, filtered and excess solvent removed by rotary evaporation under reduced pressure by rotary evaporation under reduced pressure. The product was purified by careful silica gel chromatography (slow elution starting with 100% $CHCl_3$ up to 9:1 $CHCl_3$/MeOH) followed by reverse phase chromatography to remove unreacted maleimide-$PEG_8$-acid. The product **18** was isolated in 17.6% (21.8 mg). LC/MS 2.57 min (ES+) *m*/*z* (relative intensity) 1405.30 ; $^1$H NMR (400 MHz, $CDCl_3$) $\delta$ 7.91 (t, *J* = 3.5 Hz, 1H), 7.80 (d, *J* = 4.0 Hz, 1H), 7.75 (d, *J* = 8.8 Hz, 1H), 7.69 (d, *J* = 8.7 Hz, 1H), 7.54 - 7.50 (m, 2H), 7.45 (s, 1 H), 7.39 - 7.31 (m, 2H), 6.87 (d, *J* = 10.5 Hz, 2H), 6.76 (s, 1H), 6.72 - 6.68 (m, 2H), 4.74 - 4.62 (m, 1H), 4.45 - 4.17 (m, 7H), 3.95 (s, 3H), 3.94 (s, 3H), 3.67 - 3.58 (m, 34H), 3.54 (m, 2H), 3.42 (dd, *J* = 10.2, 5.2 Hz, 2H), 3.16 - 3.07 (m, 1H), 2.92 (dd, *J* = 16.1, 4.1 Hz, 1H), 2.62 - 2.49 (m, 4H), 2.48 - 2.39 (m, 2H), 2.37 - 2.25 (m, 1 H), 1.92 (s, 1H), 1.52 - 1.44 (m, 3H), 1.10 - 0.93 (m, 6H), 0.79 (dd, *J* = 9.2, 5.3 Hz, 2H), 0.57 (dd, *J* = 9.2, 5.3 Hz, 2H), NH were not observed.

**Example 3**

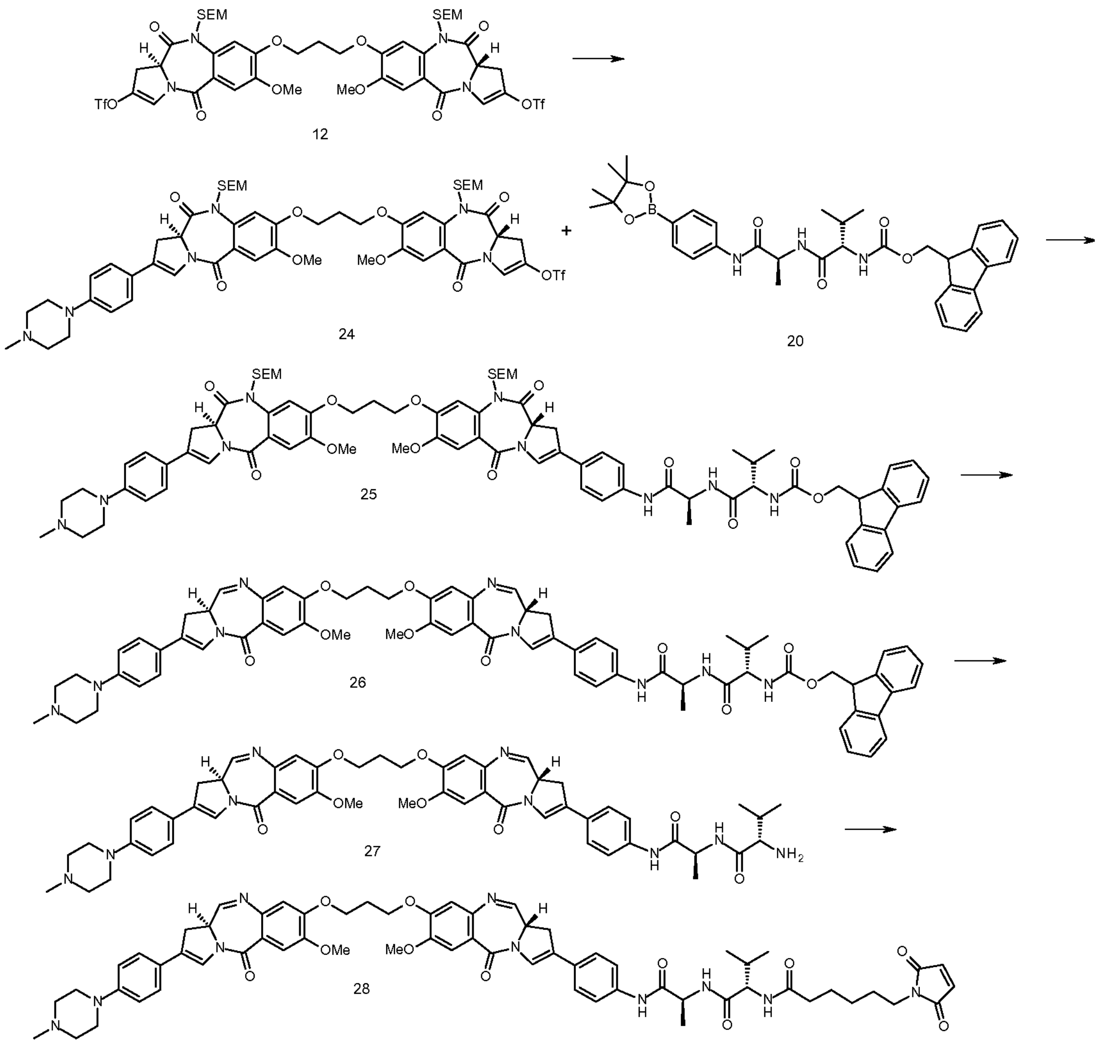

12

24

20

25

26

27

28

*(a)* ***(S)****-7-methoxy-8-(3-(((****S****)-7-methoxy-2-(4-(4-methylpiperazin-1-yl)phenyl)-5,11-dioxo-10-((2-(trimethylsilyl)ethoxy)methyl)-5,10,11,11a-tetrahydro-1H-pyrrolo[2,1-c][1,4]benzodiazepin-8-yl)oxy)propoxy)-5,11-dioxo-10-((2-(trimethylsilyl)ethoxy)methyl)-5,10,11,11a-tetrahydro-1H-pyrrolo[2,1-c][1,4]benzodiazepin-2-yl trifluoromethanesulfonate* ***(24)***

$Pd(PPh_3)_4$ (20.6 mg, 0.018 mmol) was added to a stirred mixture of the bis-enol triflate 12 (500 mg, 0.44 mmol), N-methyl piperazine boronic ester (100 mg, 0.4 mmol), $Na_2CO_3$ (218 mg, 2.05 mmol), MeOH (2.5 mL), toluene (5 mL) and water (2.5 mL). The reaction mixture was allowed to stir at 30°C under a nitrogen atmosphere for 24 hours after which time all the boronic ester has consumed. The reaction mixture was then evaporated to dryness before the residue was taken up in EtOAc (100 mL) and washed with $H_2O$ (2 x 50 mL), brine (50 mL), dried ($MgSO_4$), filtered and evaporated under reduced pressure to provide the crude product. Purification by flash chromatography (gradient elution: 80:20 v/v Hexane/EtOAc to 60:40 v/v Hexane/EtOAc) afforded product **24** as a yellowish foam (122.6 mg, 25%).

LC/MS 3.15 min (ES+) *m/z* (relative intensity) 1144 ([*M* + H]$^{+\cdot}$, 20%).

*(b) (9H-fluoren-9-yl)methyl ((S)-1-(((S)-1-((4-((S)-7-methoxy-8-(3-(((S)-7-methoxy-2-(4-(4-methylpiperazin-1-yl)phenyl)-5,11-dioxo-10-((2-(trimethylsilyl)ethoxy)methyl)-5,10,11,11a-tetrahydro-1H-pyrrolo[2,1-c][1,4]benzodiazepin-8-yl)oxy)propoxy)-5,11-dioxo-10-((2-(trimethylsilyl)ethoxy)methyl)-5,10,11,11a-tetrahydro-1H-pyrrolo[2,1-c][1,4]benzodiazepin-2-yl)phenyl)amino)-1-oxopropan-2-yl)amino)-3-methyl-1-oxobutan-2-yl)carbamate (25)*

PBD-triflate **24** (359 mg, 0.314 mmol), boronic pinacol ester **20** (250 mg, 0.408 mmol) and triethylamine (0.35 mL, 2.51 mmol) were dissolved in a mixture of toluene/MeOH/$H_2O$, 2:1:1 (3 mL). The microwave vessel was purged and filled with argon three times before *tetrakis*(triphenylphosphine)palladium(0) (21.7 mg, 0.018 mmol) was added and the reaction mixture placed in the microwave at 80°C for 10 minutes. Subsequently, $CH_2Cl_2$ (100 mL) was added and

the organics were washed with water (2 x 50 mL) and brine (50 mL) before being dried with $MgSO_4$, filtered and the volatiles removed by rotary evaporation under reduced pressure. The crude product was purified by silica gel chromatography column ($CHCl_3$/MeOH, 100% to 9:1) to afford pure 25 (200 mg, 43% yield). LC/MS 3.27 min (ES+) *m/z* (relative intensity) 1478 ([*M* + H]$^{+\cdot}$, 100%).

*(c) (9H-fluoren-9-yl)methyl ((**S**)-1-(((**S**)-1-((4-((**S**)-7-methoxy-8-(3-(((**S**)-7-methoxy-2-(4-(4-methylpiperazin-1-yl)phenyl)-5-oxo-5,11a-dihydro-1H-pyrrolo[2,1-c][1,4]benzodiazepin-8-yl)oxy)propoxy)-5-oxo-5,11a-dihydro-1H-pyrrolo[2,1-c][1,4]benzodiazepin-2-yl)phenyl)amino)-1-oxopropan-2-yl)amino)-3-methyl-1-oxobutan-2-yl)carbamate **(26)***

A solution of Super-Hydride® (0.34 mL, 1 M in THF) was added dropwise to a solution of SEM-dilactam 25 (200 mg, 0.135 mmol) in THF (5 mL) at -78°C under an argon atmosphere. The addition was completed over 5 minutes in order to maintain the internal temperature of the reaction mixture constant. After 20 minutes, an aliquot was quenched with water for LC/MS analysis, which revealed that the reaction was complete. Water (20 mL) was added to the reaction mixture and the cold bath was removed. The organic layer was extracted with EtOAc (3 x 30 mL) and the combined organics were washed with brine (50 mL), dried with $MgSO_4$, filtered and the solvent removed by rotary evaporation under reduced pressure. The crude product was dissolved in MeOH (6 mL), $CH_2Cl_2$ (3 mL), water (1 mL) and enough silica gel to form a thick stirring suspension. After 5 days, the suspension was filtered through a sintered funnel and washed with $CH_2Cl_2$/MeOH (9:1) (100 mL) until the elution of the product was complete. The organic layer was washed with brine (2 x 50 mL), dried with $MgSO_4$, filtered and the solvent removed by rotary evaporation under reduced pressure. Purification by silica gel column chromatography (100% $CHCl_3$ to 96% $CHCl_3$/ 4% MeOH) afforded the product **26** as a yellow solid (100 mg, 63%). LC/MS 2.67 min (ES+) *m/z* (relative intensity) 1186 ([M + H]$^{+\cdot}$, 5%).

*(d) (**S**)-2-amino-N-((**S**)-1-((4-((**R**)-7-methoxy-8-(3-(((**R**)-7-methoxy-2-(4-(4-methylpiperazin-1-yl)phenyl)-5-oxo-5,11a-dihydro-1H-pyrrolo[2,1-c][1,4]benzodiazepin-8-yl)oxy)propoxy)-5-oxo-5,11a-dihydro-1H-pyrrolo[2,1-c][1,4]benzodiazepin-2-yl)phenyl)amino)-1-oxopropan-2-yl)-3-methylbutanamide **(27)***

Excess piperidine was added (0.1 mL, 1 mmol) to a solution of PBD **26** (36.4 mg, 0.03 mmol) in DMF (0.9 mL). The mixture was allowed to stir at room temperature for 20 min, at which point the reaction had gone to completion (as monitored by LC/MS). The reaction mixture was diluted with $CH_2Cl_2$ (50 mL) and the organic phase was washed with $H_2O$ (3 x 50 mL) until complete piperidine removal. The organic phase was dried over $MgSO_4$, filtered and excess solvent removed by rotary evaporation under reduced pressure to afford crude product **27** which was used as such in the next step. LC/MS 2.20 min (ES+) *m/z* (relative intensity) 964 ([*M* + H]$^{+\cdot}$, 5%).

*(e) 6-(2,5-dioxo-2,5-dihydro-1H-pyrrol-1-yl)-N-((**S**)-1-(((**S**)-1-((4-((**S**)-7-methoxy-8-(3-(((S)-7-methoxy-2-(4-(4-methylpiperazin-1-yl)phenyl)-5-oxo-5,11a-dihydro-1H-pyrrolo[2,1-c][1,4]benzodiazepin-8-yl)oxy)propoxy)-5-oxo-5,11a-dihydro-1H-pyrrolo[2,1-c][1,4]benzodiazepin-2-yl)phenyl)amino)-1-oxopropan-2-yl)amino)-3-methyl-1-oxobutan-2-yl)hexanamide **(28)***

EDCI hydrochloride (4.7 mg, 0.03 mmol) was added to a suspension of 6-maleimidohexanoic acid (6.5 mg, 0.03 mmol) in dry $CH_2Cl_2$ (3 mL) under argon atmosphere. The mixture was stirred for 1 hour at room temperature before PBD **27** (34 mg, crude) was added. Stirring was maintained until the reaction was complete (6 hours). The reaction was diluted with $CH_2Cl_2$ and the organic phase was washed with $H_2O$ and brine before being dried over $MgSO_4$, filtered and excess solvent removed by rotary evaporation under reduced pressure by rotary evaporation under reduced pressure. The product was purified by careful silica gel chromatography (slow elution starting with 100% $CHCl_3$ up to 9:1 $CHCl_3$/MeOH) followed by reverse phase chromatography to remove unreacted maleimide-$PEG_8$-acid. The product 28 was isolated in 41% over two steps (14.6 mg). LC/MS 2.40 min (ES+) *m/z* (relative intensity) 1157 ([*M* + H]$^{+\cdot}$, 5%)

**Example 4 -** alternative synthesis of compound 25

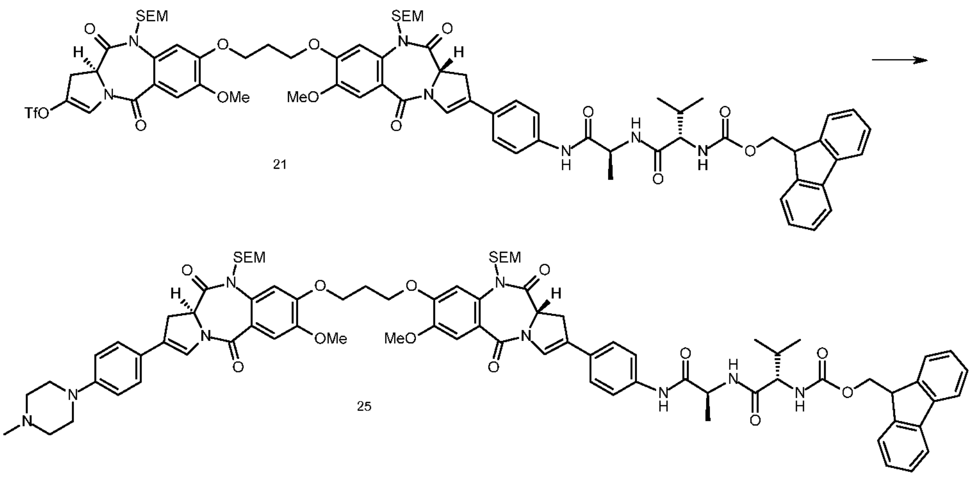

21

25

PBD-triflate **21** (469 mg, 0.323 mmol), boronic pinacol ester (146.5 mg, 0.484 mmol) and $Na_2CO_3$ (157 mg, 1.48 mmol) were dissolved in a mixture of toluene/MeOH/$H_2O$, 2:1:1 (10 mL). The reaction flask was purged with argon three times before *tetrakis*(triphenylphosphine)palladium(0) (7.41 mg, 0.0064 mmol) was added and the reaction mixture heated to 30°C overnight. The solvents were removed under reduced pressure and the residue was taken up in $H_2O$ (50 mL) and extracted with EtOAc (3 x 50 mL). The combined organics were washed with brine (100 mL), dried with $MgSO_4$, filtered and the volatiles removed by rotary evaporation under reduced pressure. The crude product was purified by silica gel column chromatography ($CHCl_3$ 100% to $CHCl_3$/MeOH 95%:5%) to afford pure **25** in 33% yield (885 mg). LC/MS 3.27 min (ES+) *m*/*z* (relative intensity) 1478 ($[M + H]^{+\cdot}$, 100%).

**Example 5**

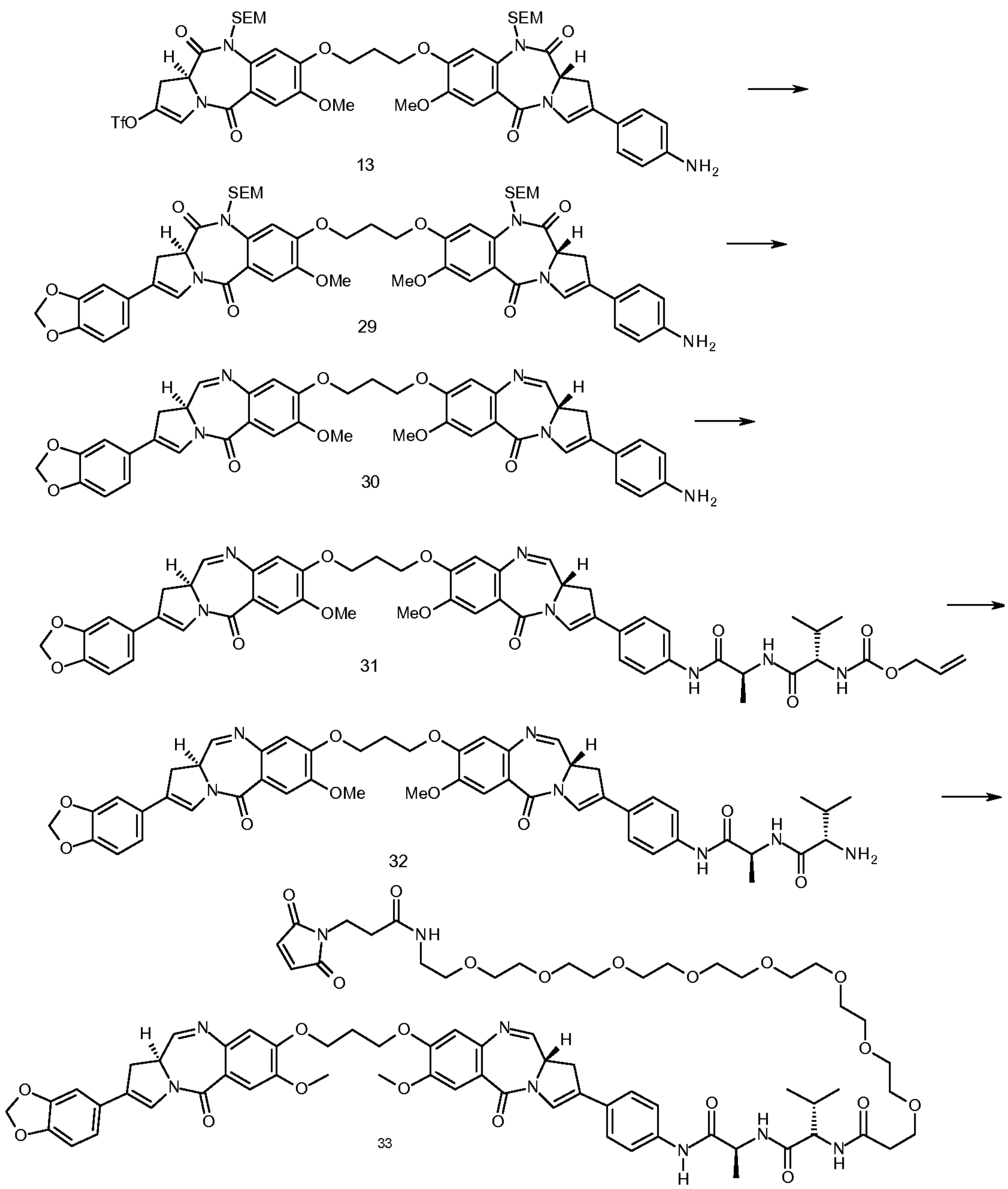
13
29
30
31
32
33

*(a) (S)-2-(4-Aminophenyl)-8-(3-(((S)-2-(benzo[d][1,3]dioxol-5-yl)-7-methoxy-5,11-dioxo-10-((2-(trimethylsilyl)ethoxy)methyl)-5,10,11,11a-tetrahydro-1H-pyrrolo[2,1-c][1,4]benzodiazepin-8-yl)oxy)propoxy)-7-methoxy-10-((2-(trimethylsilyl)ethoxy)methyl)-1H-pyrrolo[2,1-c][1,4]benzodiazepine-5,11(10H,11aH)-dione* ***(29)***

3, 4-(Methylenedioxy)phenyl boronic acid (356 mg, 2.1 mmol, 1.3 equiv.), TEA (1.8 mL, 12.9 mmol, 8 equiv.) and triflate/aniline **13** (1.75 g, 1.7 mmol, 1 equiv.) were dissolved in a mixture of ethanol (7 mL), toluene (13 mL) and water (2 mL) under an Ar atmosphere. The reaction mixture was evacuated and flushed with Ar 3 times, before addition of tetrakis(triphenylphosphine)palladium(0) (114 mg, 0.1 mmol, 0.06 equiv.). The flask was again evacuated and flushed with Ar 3 times and heated in a microwave at 80°C for 8 minutes with 30 seconds pre-stirring time. Analysis by TLC (80:20 v/v ethyl acetate/hexane) indicated complete consumption of starting material. The reaction mixture was diluted with dichloromethane (50 mL) and washed with water (50 mL). The organic layer was dried with $MgSO_4$, filtered and the solvent removed *in vacuo.* Purification by silica gel column chromatography (60:40 to 20:80 v/v hexane/ ethyl acetate) afforded the product **29** as a yellow solid (1.21 g, 71 %). LC/MS (3.92 min ($ES^+$) *m/z* (relative intensity) 1032.44 ([*M* + H]$^{+\cdot}$, 100).

*(b) (S)-2-(4-Aminophenyl)-8-(3-(((S)-2-(benzo[d][1,3]dioxol-5-yl)-7-methoxy-5-oxo-5,11a-dihydro-1H-pyrrolo[2,1-c][1,4]benzodiazepin-8-yl)oxy)propoxy)-7-methoxy-1H-pyrrolo[2,1-c][1,4]benzodiazepin-5(11aH)-one* ***(30)***

SEM dilactam **29** (0.25 g, 0.24 mmol, 1 equiv.) was dissolved in THF (8 mL) and cooled to -78°C under an Ar atmosphere. Super-Hydride® (0.6 mL, 1 M in THF, 2.5 equiv.) was added drop wise over 5 minutes while monitoring the temperature. After 20 minutes a small sample was taken and worked-up for LCMS analysis. Water (50 mL) was added, the cold bath was removed and the solution washed with ethyl acetate (50 mL). The organic layer was extracted and washed with brine (60 mL), dried with $MgSO_4$, filtered and the solvent removed *in vacuo.* The crude product was dissolved in EtOH (15 mL), $CH_2Cl_2$ (7.5 mL) and water (2.5 mL) and enough silica gel was added until it was a thick suspension. After 5 days stirring, it was filtered through a sintered funnel and washed with $CH_2Cl_2$/MeOH (9:1) (100 mL) until product ceased to be eluted. The organic layer was washed with brine (2 x 50 mL), dried with $MgSO_4$, filtered and the solvent removed *in vacuo.* Purification by silica gel column chromatography ($CHCl_3$ with 1% to 4% MeOH gradient) afforded the product **30** as a yellow solid (94 mg, 53%). LC/MS (2.53 min ($ES^+$) *m*/*z* (relative intensity) 739.64 ($[M]^{+\cdot}$, 70).

*(c) Allyl ((S)-1-(((S)-1-((4-((S)-8-(3-(((S)-2-(benzo[d][1,3]dioxol-5-yl)-7-methoxy-5-oxo-5,11a-dihydro-1H-pyrrolo[2,1-c][1,4]benzodiazepin-8-yl)oxy)propoxy)-7-methoxy-5-oxo-5,11a-dihydro-1H-pyrrolo[2,1-c][1,4]benzodiazepin-2-yl)phenyl)amino)-1-oxopropan-2-yl)amino)-3-methyl-1-oxobutan-2-yl)carbamate* ***(31)***

Under an Ar atmosphere, Alanine-Valine-Alloc (180 mg, 0.66 mmol, 1.2 equiv.) was stirred with EEDQ (163 mg, 0.66 mmol, 1.2 equiv.) in anhydrous $CH_2Cl_2$ (21 mL) and methanol (1 mL) for 1 hour. The PBD **30** (407 mg, 0.55 mmol, 1 equiv.) was dissolved in anhydrous $CH_2Cl_2$ (21 mL) and methanol (1 mL) and added to the reaction. LC/MS after 5 days stirring at room temperature showed majority product formation. The solvent was removed *in vacuo* before purification by column chromatography ($CH_2Cl_2$ with 1% to 6% MeOH gradient) to yield the product **31** as a yellow solid (184 mg, 34%). LC/MS (2.95 min ($ES^+$) *m*/*z* (relative intensity) 994.95 ($[M + H]^{+\cdot}$, 60).

*(d) (S)-2-Amino-N-((S)-1-((4-((S)-8-(3-(((S)-2-(benzo[d][1,3]dioxol-5-yl)-7-methoxy-5-oxo-5,11a-dihydro-1H-pyrrolo[2,1-c][1,4]benzodiazepin-8-yl)oxy)propoxy)-7-methoxy-5-oxo-5,11a-dihydro-1H-pyrrolo[2,1-c][1,4]benzodiazepin-2-yl)phenyl)amino)-1-oxopropan-2-yl)-3-methylbutanamide* ***(32)***

The imine **31** (100 mg, 0.1 mmol, 1 equiv.) was dissolved in anhydrous DCM (10 mL) (with the aid of one drop of methanol to aid dissolution) under an Ar atmosphere. Pyrrolidine (30 μL, 0.15 mmol, 1.5 equiv.) was added drop wise before the flask was evacuated and flushed with Ar three times. $Pd(PPh_3)_4$ (7 mg, 6 μmol, 0.06 equiv.) was added and the flask was evacuated and flushed with Ar three times. LC/MS analysis after 1 hour indicated product formation and complete loss of starting material. $Et_2O$ (60 mL) was added to the reaction mixture and it was left to stir until all the product had crashed out of solution. The precipitate was filtered through a sintered funnel and washed twice with $Et_2O$ (2 x 20 mL). The collection flask was replaced and the isolated solid was dissolved and washed through the sinter with $CHCl_3$ (100 mL). The solvent was removed *in vacuo* to afford the crude product **32** as a yellow solid which was used directly in the next step. LC/MS (1.14 min ($ES^+$) *m*/*z* (relative intensity) 910.40 ($[M + H]^{+\cdot}$, 67).

*(e) N-((S)-1-(((S)-1-((4-((S)-8-(3-(((S)-2-(Benzo[d][1,3]dioxol-5-yl)-7-methoxy-5-oxo-5,11a-dihydro-1H-pyrrolo[2,1-c][1,4]benzodiazepin-8-yl)oxy)propoxy)-7-methoxy-5-oxo-5,11a-dihydro-1H-pyrrolo[2,1-c][1,4]benzodiazepin-2-yl)phenyl)amino)-1-oxopropan-2-yl)amino)-3-methyl-1-oxobutan-2-yl)-1-(3-(2,5-dioxo-2,5-dihydro-1H-pyrrol-1-yl)propanamido)-3,6,9,12,15,18,21,24-octaoxaheptacosan-27-amide* ***(33)***

The imine **32** (92 mg, 0.1 mmol, 1.1 equiv.) was dissolved in $CHCl_3$ (6 mL) with one drop of anhydrous MeOH to aid dissolution. Maleimide-$PEG_8$-acid (53 mg, 0.09 mmol, 1 equiv.) was added followed by EEDQ (33 mg, 0.14 mmol, 1.5 equiv.). This was left to stir vigorously at room temperature under Ar for 4 days until LC/MS analysis showed majority product formation. The solvent was removed in vacuo and the crude product was partially purified by silica gel column chromatography (CHCl3 with 1% to 10% MeOH gradient) yielding **33** (81 mg). The material was purified further by preparative HPLC to give **33** as a yellow solid (26.3 mg, 18%). Fast Formic run: LC/MS (1.39 min (ES+) m/z (relative intensity) 1485.00 ($[M + H]^{+\cdot}$, 64).

**Example 6**

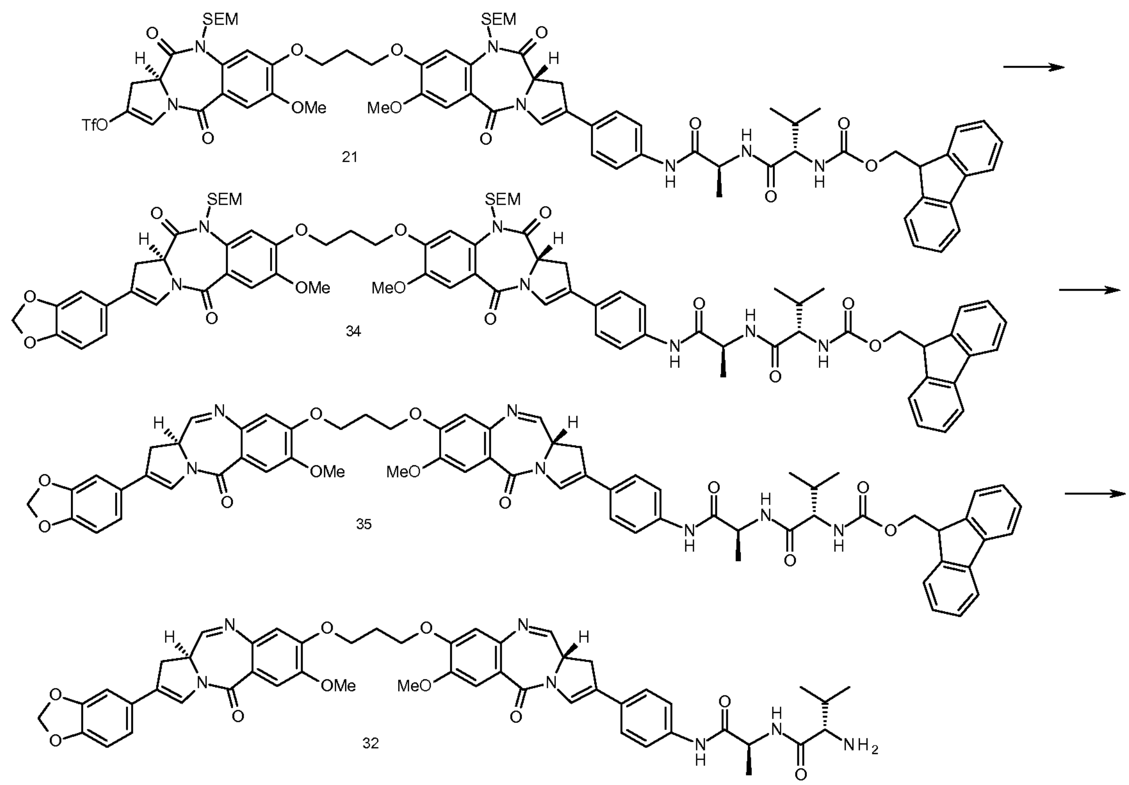

21

34

35

32

*(a) 9H-Fluoren-9-yl)methyl ((S)-1-(((S)-1-((4-((S)-8-(3-(((S)-2-(benzo[d][1,3]dioxol-5-yl)-7-methoxy-5,11-dioxo-10-((2-(trimethylsilyl)ethoxy)methyl)-5,10,11,11a-tetrahydro-1H-pyrrolo[2,1-c][1,4]benzodiazepin-8-yl)oxy)propoxy)-7-methoxy-5,11-dioxo-10-((2-(trimethylsilyl)ethoxy)methyl)-5,10,11,11a-tetrahydro-1H-pyrrolo[2,1-c][1,4]benzodiazepin-2-yl)phenyl)amino)-1-oxopropan-2-yl)amino)-3-methyl-1-oxobutan-2-yl)carbamate (**34**)*

The triflate **21** (0.5 g, 0.35 mmol, 1 equiv.), 3, 4-(methylenedioxy)phenyl boronic acid (75 mg, 0.45 mmol, 1.3 equiv.) and $Na_2CO_3$ (0.17 g, 1.6 mmol, 4.5 equiv.) were dissolved in toluene (11 mL), EtOH (5.5 mL) and water (5.5 mL) under an Ar atmosphere. The flask was evacuated and flushed with Ar three times. $Pd(PPh_3)_4$ (24 mg, 0.02 mmol, 0.06 equiv.) was added and again the flask was evacuated and flushed with Ar three times. This was heated to 30°C and left stirring overnight. Analysis by LC/MS showed complete loss of starting material. The solvent was removed *in vacuo* and the residue dissolved in water (60 mL) before washing with ethyl acetate (60 mL x 3). The combined organic layers were washed with brine (50 mL), dried with $MgSO_4$, filtered and the solvent removed *in vacuo.* Purification by column chromatography (50:50 to 25:75 v/v hexane/ ethyl acetate) afforded the product **34** as a yellow solid (310 mg, 64%). LC/MS (1.44 min ($ES^-$) *m*/*z* (relative intensity) 1423.35 ([*M* - H]$^{-\cdot}$, 79).

*(b) (9H-Fluoren-9-yl)methyl ((S)-1-(((S)-1-((4-((S)-8-(3-(((S)-2-(benzo[d][1,3]dioxol-5-yl)-7-methoxy-5-oxo-5,11a-dihydro-1H-pyrrolo[2,1-c][1,4]benzodiazepin-8-yl)oxy)propoxy)-7-methoxy-5-oxo-5,11a-dihydro-1H-pyrrolo[2,1-c][1,4]benzodiazepin-2-yl)phenyl)amino)-1-oxopropan-2-yl)amino)-3-methyl-1-oxobutan-2-yl)carbamate (**35**)*

SEM dilactam **34** (0.31 g, 0.22 mmol, 1 equiv.) was dissolved in THF (10 mL) and cooled to -78°C under an Ar atmosphere. Super-Hydride® (0.5 mL, 1 M in THF, 2.5 equiv.) was added drop wise over 5 minutes while monitoring the temperature. After 30 minutes a small sample was taken and worked-up for LC/MS analysis. Water (50 mL) was added, the cold bath was removed and the solution washed with ethyl acetate (50 mL). The organic layer was extracted and washed with brine (60 mL), dried with $MgSO_4$, filtered and the solvent removed *in vacuo.* The crude product was dissolved in EtOH (13.2 mL), $CH_2Cl_2$ (6.6 mL) and water (2.2 mL) and enough silica gel was added until it was a thick suspension. After 5 days stirring, it was filtered through a sintered funnel and washed with $CH_2Cl_2$/MeOH (9:1) (100 mL) until product ceased to be eluted. The organic layer was washed with brine (2 x 50 mL), dried with $MgSO_4$, filtered and the solvent removed *in vacuo.* Purification by silica gel column chromatography ($CHCl_3$ with 1% to 4% MeOH gradient) afforded the pure product **35** as a yellow solid (185 mg, 75%). LC/MS (1.70 min ($ES^+$) *m*/*z* (relative intensity) 1132.85 ([*M* + H]$^{+\cdot}$, 60).

*(c) (S)-2-Amino-N-((S)-1-((4-((S)-8-(3-(((S)-2-(benzo[d][1,3]dioxol-5-yl)-7-methoxy-5-oxo-5,11a-dihydro-1H-pyrrolo[2,1-c][1,4]benzodiazepin-8-yl)oxy)propoxy)-7-methoxy-5-oxo-5,11a-dihydro-1H-pyrrolo[2,1-c][1,4]benzodiazepin-2-yl)phenyl)amino)-1-oxopropan-2-yl)-3-methylbutanamide (**32**)*

The imine **35** (82 mg, 0.07 mmol, 1 equiv.) was dissolved in DMF (1 mL) before piperidine (0.2 mL, 2 mmol, excess) was added slowly. This solution was left to stir at room temperature for 20 minutes until LC/MS analysis showed complete consumption of starting material. The reaction mixture was diluted with $CH_2Cl_2$ (50 mL), washed with water (50 mL x 4), dried with $MgSO_4$, filtered and the solvent removed *in vacuo.* The product **33** was used without further purification in the next step. LC/MS (1.15 min ($ES^+$) *m*/*z* (relative intensity) 910.60 ($[M + H]^{+\cdot}$, 58).

**Example** 7

<u>(i) (S)-(2-amino-5-methoxy-4-((triisopropylsilyl)oxy)phenyl)(2-(((tert-butyldimethylsilyl)oxy)methyl)-4-methyl-2,3-dihydro-1H-pyrrol-1-yl)methanone (4**9**)</u>

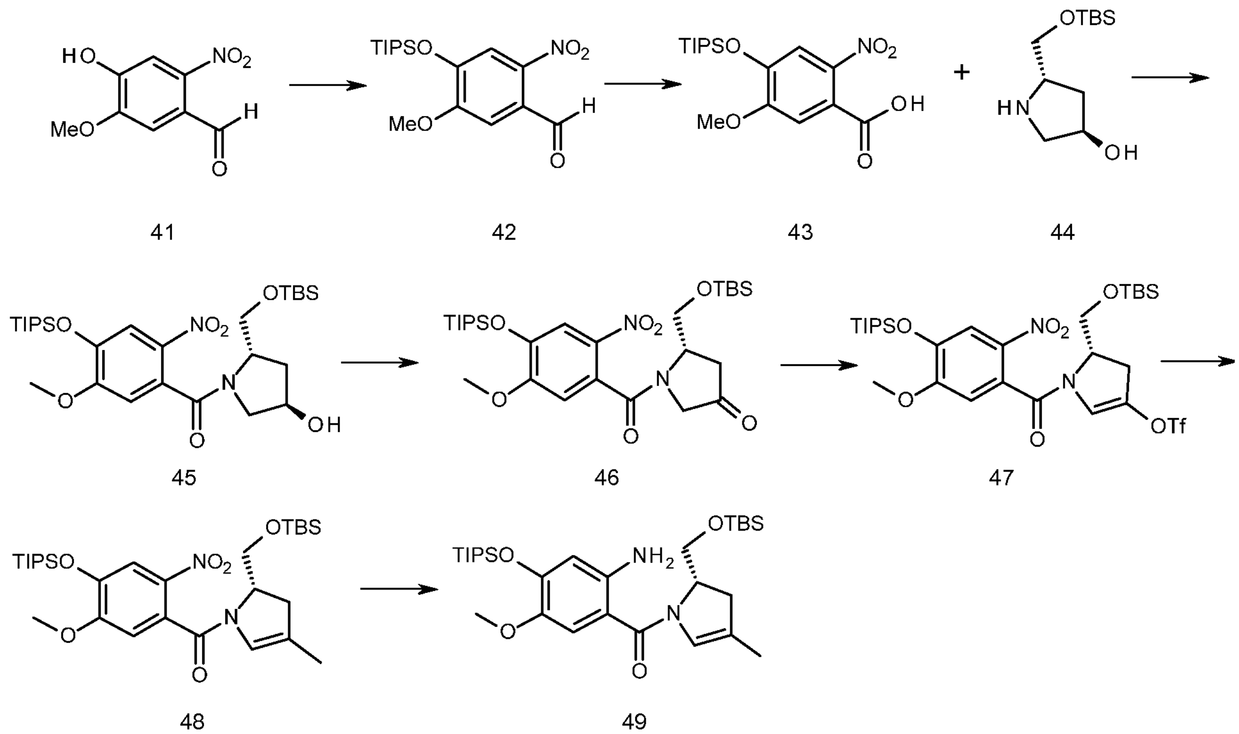

41 42 43 44

45 46 47

48 49

*(a) 5-methoxy-2-nitro-4-((triisopropylsilyl)oxy)benzaldehyde (**42**)*

Neat triisopropylsilylchloride (56.4 mL, 262 mmol) was added to a mixture of imidazole (48.7 g, 715.23 mmol) and 4-hydroxy-5-methoxy-2-nitrobenzaldehyde 41 (47 g, 238 mmol) (ground together). The mixture was heated until the phenol and imidazole melted and went into solution (100 °C). The reaction mixture was allowed to stir for 15 minutes and was then allowed to cool, whereupon a solid was observed to form at the bottom of the flask (imidazole chloride). The reaction mixture was diluted with 5% EtOAc/ hexanes and loaded directly onto silica gel and the pad was eluted with 5% EtOAc/ hexanes , followed by 10% EtOAc/hexanes (due to the low excess, very little unreacted TIPSCl was found in the product). The desired product was eluted with 5 % ethyl acetate in hexane. Excess eluent was removed by rotary evaporation under reduced pressure, followed by drying under high vacuum to afford a crystalline light sensitive solid (74.4 g, 88 %). Purity satisfactory by LC/MS (4.22 min (ES+) *m*/*z* (relative intensity) 353.88 ($[M + H]^{+\cdot}$, 100)); $^1H$ NMR (400 MHz, $CDCl_3$) δ 10.43 (s, 1 H), 7.60 (s, 1 H), 7.40 (s, 1 H), 3.96 (s, 3H), 1.35 - 1.24 (m, 3H), 1.10 (m, 18H).

*(b) 5-methoxy-2-nitro-4-((triisopropylsilyl)oxy)benzoic acid (**43**)*

A solution of sodium chlorite (47.3 g, 523 mmol, 80 % technical grade) and sodium dihydrogenphosphate

monobasic (35.2 g, 293 mmol) ($NaH_2PO_4$) in water (800 mL) was added to a solution of compound **2** (74 g, 209 mmol) in tetrahydrofuran (500 mL) at room temperature. Hydrogen peroxide (60 % w/w, 140 mL, 2.93 mol) was immediately added to the vigorously stirred biphasic mixture. The reaction mixture evolved gas (oxygen), the starting material dissolved and the temperature of the reaction mixture rose to 45°C. After 30 minutes LC/MS revealed that the reaction was complete. The reaction mixture was cooled in an ice bath and hydrochloric acid (1 M) was added to lower the pH to 3 (this step was found unnecessary in many instances, as the pH at the end of the reaction is already acidic; please check the pH before extraction). The reaction mixture was then extracted with ethyl acetate (1 L) and the organic phases washed with brine (2 x 100 mL) and dried over magnesium sulphate. The organic phase was filtered and excess solvent removed by rotary evaporation under reduced pressure to afford the product **43** in quantitative yield as a yellow solid. LC/MS (3.93 min (ES-) *m*/*z* (relative intensity) 367.74 ([*M* - H]$^{-\cdot}$, 100)); $^1$H NMR (400 MHz, $CDCl_3$) δ 7.36 (s, 1H), 7.24 (s, 1H), 3.93 (s, 3H), 1.34 - 1.22 (m, 3H), 1.10 (m, 18H).

*(c) ((2S,4R)-2-(((tert-butyldimethylsilyl)oxy)methyl)-4-hydroxypyrrolidin-1-yl)(5-methoxy-2-nitro-4-((triisopropylsilyl)oxy)phenyl)methanone (**45**)*

DCC (29.2 g, 141 mmol, 1.2 eq) was added to a solution of acid **3** (43.5 g, 117.8 mmol, 1 eq), and hydroxybenzotriazole hydrate (19.8 g, 129.6 mmol, 1.1 eq) in dichloromethane (200 mL) at 0 °C. The cold bath was removed and the reaction was allowed to proceed for 30 mins at room temperature, at which time a solution of (*2S,4R*)-2-*t*-butyldimethylsilyloxymethyl-4-hydroxypyrrolidine **44** (30 g, 129.6 mmol, 1.1 eq) and triethylamine (24.66 mL, 176 mmol, 1.5 eq) in dichloromethane (100 mL) was added rapidly at -10 °C under argon (on large scale, the addition time could be shortened by cooling the reaction mixture even further. The reaction mixture was allowed to stir at room temperature for 40 minutes to 1 hour and monitored by LC/MS and TLC (EtOAc). The solids were removed by filtration over celite and the organic phase was washed with cold aqueous 0.1 M HCl until the pH was measured at 4 or 5. The organic phase was then washed with water, followed by saturated aqueous sodium bicarbonate and brine. The organic layer was dried over magnesium sulphate, filtered and excess solvent removed by rotary evaporation under reduced pressure. The residue was subjected to column flash chromatography (silica gel; gradient 40/60 ethyl acetate/hexane to 80/20 ethyl acetate/ hexane). Excess solvent was removed by rotary evaporation under reduced pressure afforded the pure product **45**, (45.5 g of pure product 66%, and 17 g of slightly impure product, 90% in total). LC/MS 4.43 min (ES+) *m*/*z* (relative intensity) 582.92 ([*M* + H]$^{+\cdot}$, 100); $^1$H NMR (400 MHz, $CDCl_3$) δ 7.66 (s, 1H), 6.74 (s, 1H), 4.54 (s, 1H), 4.40 (s, 1H), 4.13 (s, 1H), 3.86 (s, 3H), 3.77 (d, *J* = 9.2 Hz, 1H), 3.36 (dd, *J* = 11.3, 4.5 Hz, 1H), 3.14 - 3.02 (m, 1H), 2.38 - 2.28 (m, 1H), 2.10 (ddd, *J* = 13.3, 8.4, 2.2 Hz, 1H), 1.36 - 1.19 (m, 3H), 1.15 - 1.05 (m, 18H), 0.91 (s, 9H), 0.17 - 0.05 (m, 6H), (presence of rotamers).

*(d) (S)-5-(((tert-butyldimethylsilyl)oxy)methyl)-1-(5-methoxy-2-nitro-4-((triisopropylsilyl)oxy)benzoyl)pyrrolidin-3-one (**46**)*

TCCA (8.82 g, 40 mmol, 0.7 eq) was added to a stirred solution of **45** (31.7 g, 54 mmol, 1 eq) and TEMPO (0.85 g, 5.4 mmol, 0.1 eq) in dry dichloromethane (250 mL) at 0 °C. The reaction mixture was vigorously stirred for 20 minutess, at which point TLC (50/50 ethyl acetate/hexane) revealed complete consumption of the starting material. The reaction mixture was filtered through celite and the filtrate washed with aqueous saturated sodium bicarbonate (100 mL), sodium thiosulphate (9 g in 300 mL), brine (100 mL) and dried over magnesium sulphate. Rotary evaporation under reduced pressure afforded product 46 in quantitative yield. LC/MS 4.52 min (ES+) *m*/*z* (relative intensity) 581.08 ([*M* + H]$^{+\cdot}$, 100); $^1$H NMR (400 MHz, $CDCl_3$) δ 7.78 - 7.60 (m, 1H), 6.85 - 6.62 (m, 1H), 4.94 (dd, *J* = 30.8, 7.8 Hz, 1H), 4.50 - 4.16 (m, 1H), 3.99 - 3.82 (m, 3H), 3.80 - 3.34 (m, 3H), 2.92 - 2.17 (m, 2H), 1.40 - 1.18 (m, 3H), 1.11 (t, *J* = 6.2 Hz, 18H), 0.97 - 0.75 (m, 9H), 0.15 --0.06 (m, 6H), (presence of rotamers).

*(e) (S)-5-(((tert-butyldimethylsilyl)oxy)methyl)-1-(5-methoxy-2-nitro-4-((triisopropylsilyl)oxy)benzoyl)-4,5-dihydro-1H-pyrrol-3-yl trifluoromethanesulfonate (**47**)*

Triflic anhydride (27.7 mL, 46.4 g, 165 mmol, 3 eq) was injected (temperature controlled) to a vigorously stirred suspension of ketone **46** (31.9 g, 55 mmol, 1 eq) in dry dichloromethane (900 mL) in the presence of 2,6-lutidine (25.6 mL, 23.5 g, 220 mmol, 4 eq, dried over sieves) at -50 °C (acetone/dry ice bath). The reaction mixture was allowed to stir for 1.5 hours when LC/MS, following a mini work-up (water/dichloromethane), revealed the reaction to be complete. Water was added to the still cold reaction mixture and the organic layer was separated and washed with saturated sodium bicarbonate, brine and magnesium sulphate. The organic phase was filtered and excess solvent was removed by rotary evaporation under reduced pressure. The residue was subjected to column flash chromatography (silica gel; 10/90 v/v ethyl acetate/hexane), removal of excess eluent afforded the product **47** (37.6 g, 96 %) LC/MS, method 2, 4.32 min (ES+) *m*/*z* (relative intensity) 712.89 ([*M* + H]$^{+\cdot}$, 100); $^1$H NMR (400 MHz, $CDCl_3$) δ 7.71 (s, 1H), 6.75 (s, 1H),

6.05 (d, $J$ = 1.8 Hz, 1H), 4.78 (dd, $J$ = 9.8, 5.5 Hz, 1H), 4.15 - 3.75 (m, 5H), 3.17 (ddd, $J$ = 16.2, 10.4, 2.3 Hz, 1 H), 2.99 (ddd, $J$= 16.3, 4.0, 1.6 Hz, 1H), 1.45 - 1.19 (m, 3H), 1.15 - 1.08 (m, 18H), 1.05 (s, 6H), 0.95 - 0.87 (m, 9H), 0.15 - 0.08 (m, 6H).

*(f) (S)-(2-(((tert-butyldimethylsilyl)oxy)methyl)-4-methyl-2,3-dihydro-1H-pyrrol-1-yl)(5-methoxy-2-nitro-4-((triisopropylsilyl)oxy)phenyl)methanone (**48**)*

Triphenylarsine (1.71 g, 5.60 mmol, 0.4 eq) was added to a mixture of triflate **47** (10.00 g, 14 mmol, 1eq), methylboronic acid (2.94 g, 49.1 mmol, 3.5 eq), silver oxide (13 g, 56 mmol, 4 eq) and potassium phosphate tribasic (17.8 g, 84 mmol, 6 eq) in dry dioxane (80 mL) under an argon atmosphere. The reaction was flushed with argon 3 times and bis(benzonitrile)palladium(II) chloride (540 mg, 1.40 mmol, 0.1 eq) was added. The reaction was flushed with argon 3 more times before being warmed instantaneously to 110°C (the drysyn heating block was previously warmed to 110°C prior addition of the flask). After 10 mins the reaction was cooled to room temperature and filtered through a pad celite. The solvent was removed by rotary evaporation under reduced pressure. The resulting residue was subjected to column flash chromatography (silica gel; 10 % ethyl acetate / hexane). Pure fractions were collected and combined, and excess eluent was removed by rotary evaporation under reduced pressure afforded the product **48** (4.5 g, 55 %). LC/MS, 4.27 min (ES+) $m/z$ (relative intensity) 579.18 ([M+ H]$^{+\cdot}$, 100); $^1$H NMR (400 MHz, $CDCl_3$) $\delta$ 7.70 (s, 1H), 6.77 (s, 1H), 5.51 (d, $J$ = 1.7 Hz, 1H), 4.77 - 4.59 (m, 1H), 3.89 (s, 3H), 2.92 - 2.65 (m, 1H), 2.55 (d, $J$ = 14.8 Hz, 1H), 1.62 (d, $J$ = 1.1 Hz, 3H), 1.40 - 1.18 (m, 3H), 1.11 (s, 9H), 1.10 (s, 9H), 0.90 (s, 9H), 0.11 (d, $J$ = 2.3 Hz, 6H).

*(g) (S)-(2-amino-5-methoxy-4-((triisopropylsilyl)oxy)phenyl)(2-(((tert-butyldimethylsilyl)oxy)methyl)-4-methyl-2,3-dihydro-1H-pyrrol-1-yl)methanone (**49**)*

Zinc powder (28 g, 430 mmol, 37 eq) was added to a solution of compound **48** (6.7 g, 11.58 mmol) in 5% formic acid in ethanol v/v (70 mL) at around 15°C. The resulting exotherm was controlled using an ice bath to maintain the temperature of the reaction mixture below 30°C. After 30 minutes the reaction mixture was filtered through a pad of celite. The filtrate was diluted with ethyl acetate and the organic phase was washed with water, saturated aqueous sodium bicarbonate and brine. The organic phase was dried over magnesium sulphate, filtered and excess solvent removed by rotary evaporation under reduced pressure. The resulting residue was subjected to flash column chromatography (silica gel; 10 % ethyl acetate in hexane). The pure fractions were collected and combined and excess solvent was removed by rotary evaporation under reduced pressure to afford the product **49** (5.1 g, 80 %). LC/MS, 4.23 min (ES+) $m/z$ (relative intensity) 550.21 ([$M$+ H]$^{+\cdot}$, 100); $^1$H NMR (400 MHz, $CDCl_3$) $\delta$ 7.28 (s, 1H), 6.67 (s, 1H), 6.19 (s, 1H), 4.64 - 4.53 (m, $J$ = 4.1 Hz, 1H), 4.17 (s, 1H), 3.87 (s, 1H), 3.77 - 3.69 (m, 1H), 3.66 (s, 3H), 2.71 - 2.60 (m, 1H), 2.53 - 2.43 (m, 1H), 2.04 - 1.97 (m, $J$ = 11.9 Hz, 1H), 1.62 (s, 3H), 1.26 - 1.13 (m, 3H), 1.08 - 0.99 (m, 18H), 0.82 (s, 9H), 0.03 - -0.03 (m, $J$ = 6.2 Hz, 6H).

<u>(ii) (11S,11aS)-allyl 11-((tert-butyldimethylsilyl)oxy)-8-((5-iodopentyl)oxy)-7-methoxy-2-methyl-5-oxo-11,11a-dihydro-1H-benzo[e]pyrrolo[1,2-a][1,4]diazepine-10(5H)-carboxylate</u>

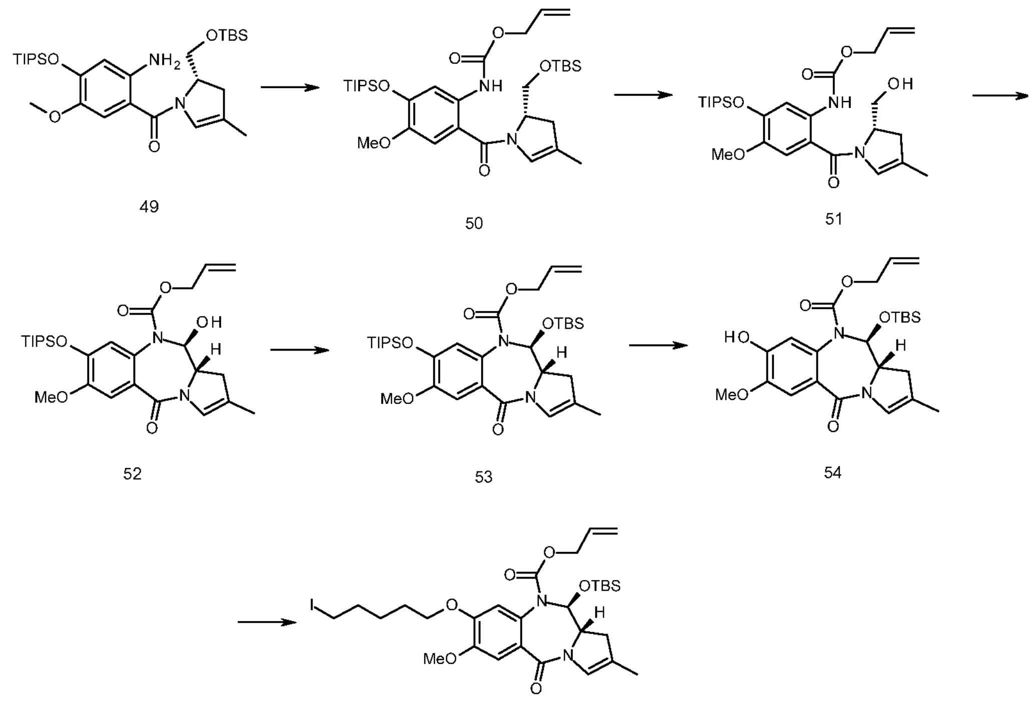

49 50 51 52 53 54

55

*(a) (S)-allyl (2-(2-(((tert-butyldimethylsilyl)oxy)methyl)-4-methyl-2,3-dihydro-1H-pyrrole-1-carbonyl)-4-methoxy-5-((triisopropylsilyl)oxy)phenyl)carbamate (50)*

Allyl chloroformate (0.30 mL, 3.00 mmol, 1.1 eq) was added to a solution of amine **49** (1.5 g, 2.73 mmol) in the presence of dry pyridine (0.48 mL, 6.00 mmol, 2.2 eq) in dry dichloromethane (20 mL) at -78°C (acetone/dry ice bath). After 30 minutes, the bath was removed and the reaction mixture was allowed to warm to room temperature. The reaction mixture was diluted with dichloromethane and saturated aqueous copper sulphate was added. The organic layer was then washed sequentially with saturated aqueous sodium bicarbonate and brine. The organic phase was dried over magnesium sulphate, filtered and excess solvent removed by rotary evaporation under reduced pressure to afford the product **50** which was used directly in the next reaction. LC/MS, 4.45 min (ES+) *m/z* (relative intensity) 632.91 ([*M* + H]$^{+\cdot}$, 100)

*(b) (S)-allyl (2-(2-(hydroxymethyl)-4-methyl-2,3-dihydro-1H-pyrrole-1-carbonyl)-4-methoxy-5-((triisopropylsilyl)oxy)phenyl)carbamate (**51**)*

The crude **50** was dissolved in a 7:1:1:2 mixture of acetic acid/methanol/tetrahydrofuran/water (28:4:4:8 mL) and allowed to stir at room temperature. After 3 hours, complete disappearance of starting material was observed by LC/MS. The reaction mixture was diluted with ethyl acetate and washed sequentially with water (2 x 500 mL), saturated aqueous sodium bicarbonate (200 mL) and brine. The organic phase was dried over magnesium sulphate filtered and excess ethyl acetate removed by rotary evaporation under reduced pressure. The resulting residue was subjected to flash column chromatography (silica gel, 25% ethyl acetate in hexane). Pure fractions were collected and combined and excess eluent was removed by rotary evaporation under reduced pressure to afford the desired product **51** (1 g, 71 %). LC/MS, 3.70 min (ES+) *m/z* (relative intensity) 519.13 ([*M* + H]$^{+\cdot}$, 95); $^1$H NMR (400 MHz, $CDCl_3$) δ 8.34 (s, 1H), 7.69 (s, 1H), 6.78 (s, 1H), 6.15 (s, 1H), 5.95 (ddt, *J* = 17.2, 10.5, 5.7 Hz, 1H), 5.33 (dq, *J* = 17.2, 1.5 Hz, 1H), 5.23 (ddd, *J* = 10.4, 2.6, 1.3 Hz, 1H), 4.73 (tt, *J* = 7.8, 4.8 Hz, 1H), 4.63 (dt, *J* = 5.7, 1.4 Hz, 2H), 4.54 (s, 1H), 3.89 - 3.70 (m, 5H), 2.87 (dd, *J* = 16.5, 10.5 Hz, 1H), 2.19 (dd, *J* = 16.8, 4.6 Hz, 1H), 1.70 (d, *J* = 1.3 Hz, 3H), 1.38 - 1.23 (m, 3H), 1.12 (s, 10H), 1.10 (s, 8H).

*(c) (11S,11aS)-allyl 11-hydroxy-7-methoxy-2-methyl-5-oxo-8-((triisopropylsilyl)oxy)-11,11a-dihydro-1H-benzo[e]pyrrolo[1,2-a][1,4]diazepine-10(5H)-carboxylate (52)*

Dimethyl sulphoxide (0.35 mL, 4.83 mmol, 2.5 eq) was added dropwise to a solution of oxalyl chloride (0.2 mL,

2.32 mmol, 1.2 eq) in dry dichloromethane (10 mL) at -78°C (dry ice /acetone bath) under an atmosphere of argon. After 10 minutes a solution of **51** (1 g, 1.93 mmol) in dry dichloromethane (8 mL) was added slowly with the temperature still at -78°C. After 15 min triethylamine (1.35 mL, dried over 4Å molecular sieves, 9.65 mmol, 5 eq) was added dropwise and the dry ice/acetone bath was removed. The reaction mixture was allowed to reach room temperature and was extracted with cold hydrochloric acid (0.1 M), saturated aqueous sodium bicarbonate and brine. The organic phase was dried over magnesium sulphate, filtered and excess dichloromethane was removed by rotary evaporation under reduced pressure to afford product **52** (658 mg, 66%). LC/MS, 3.52 min (ES+) *m*/*z* (relative intensity) 517.14 ([*M*+ H]$^{+\cdot}$, 100); $^1$H NMR (400 MHz, $CDCl_3$) δ 7.20 (s, 1H), 6.75 - 6.63 (m, *J* = 8.8, 4.0 Hz, 2H), 5.89 - 5.64 (m, *J* = 9.6, 4.1 Hz, 2H), 5.23 - 5.03 (m, 2H), 4.68 - 4.38 (m, 2H), 3.84 (s, 3H), 3.83 - 3.77 (m, 1H), 3.40 (s, 1H), 3.05 - 2.83 (m, 1H), 2.59 (d, *J* = 17.1 Hz, 1H), 1.78 (d, *J* = 1.3 Hz, 3H), 1.33 - 1.16 (m, 3H), 1.09 (d, *J* = 2.2 Hz, 9H), 1.07 (d, *J* = 2.1 Hz, 9H).

*(d) (11S,11aS)-allyl 11-((tert-butyldimethylsilyl)oxy)-7-methoxy-2-methyl-5-oxo-8-((triisopropylsilyl)oxy)-11,11a-dihydro-1H-benzo[e]pyrrolo[1,2-a][1,4]diazepine-10(5H)-carboxylate (**53**)*

Tert-butyldimethylsilyltriflate (0.70 mL, 3.00 mmol, 3 eq) was added to a solution of compound **52** (520 mg, 1.00 mmol) and 2,6-lutidine (0.46 mL, 4.00 mmol, 4 eq) in dry dichloromethane (40 mL) at 0°C under argon. After 10 min, the cold bath was removed and the reaction mixture was stirred at room temperature for 1 hour. The reaction mixture was extracted with water, saturated aqueous sodium bicarbonate and brine. The organic phase was dried over magnesium sulphate, filtered and excess was removed by rotary evaporation under reduced pressure. The resulting residue was subjected to flash column chromatography (silica gel; gradient, 10 % ethyl acetate in hexane to 20 % ethyl acetate in hexane). Pure fractions were collected and combined and excess eluent was removed by rotary evaporation under reduced pressure to give the product **53** (540 mg, 85 %). LC/MS, 4.42 min (ES+) *m*/*z* (relative intensity) 653.14 ([*M*+ Na]$^{+\cdot}$, 100); $^1$H NMR (400 MHz, $CDCl_3$) δ 7.20 (s, 1H), 6.71 - 6.64 (m, *J* = 5.5 Hz, 2H), 5.83 (d, *J* = 9.0 Hz, 1H), 5.80 - 5.68 (m, *J* = 5.9 Hz, 1H), 5.14 - 5.06 (m, 2H), 4.58 (dd, *J* = 13.2, 5.2 Hz, 1H), 4.36 (dd, *J* = 13.3, 5.5 Hz, 1H), 3.84 (s, 3H), 3.71 (td, *J* = 10.1, 3.8 Hz, 1H), 2.91 (dd, *J* = 16.9, 10.3 Hz, 1H), 2.36 (d, *J* = 16.8 Hz, 1H), 1.75 (s, 3H), 1.31 - 1.16 (m, 3H), 1.12 - 1.01 (m, *J* = 7.4, 2.1 Hz, 18H), 0.89 - 0.81 (m, 9H), 0.25 (s, 3H), 0.19 (s, 3H).

*(e) (11S,11aS)-allyl 11-((tert-butyldimethylsilyl)oxy)-8-hydroxy-7-methoxy-2-methyl-5-oxo-11,11a-dihydro-1H-benzo[e]pyrrolo[1,2-a][1,4]diazepine-10(5H)-carboxylate (**54**)*

Lithium acetate (87 mg, 0.85 mmol) was added to a solution of compound **53** (540 mg, 0.85 mmol) in wet dimethylformamide (6 mL, 50:1 DMF/water). After 4 hours, the reaction was complete and the reaction mixture was diluted with ethyl acetate (25 mL) and washed with aqueous citric acid solution (pH ~ 3), water and brine. The organic layer was dried over magnesium sulphate filtered and excess ethyl acetate was removed by rotary evaporation under reduced pressure. The resulting residue was subjected to flash column chromatography (silica gel; gradient, 25% to 75% ethyl acetate in hexane). Pure fractions were collected and combined and excess eluent was removed by rotary evaporation under reduced pressure to give the product **54** (400 mg, quantitative). LC/MS, (3.33 min (ES+) *m*/*z* (relative intensity) 475.26 ([M+H]$^+$, 100).

*(f) (11S,11aS)-allyl 11-((tert-butyldimethylsilyl)oxy)-8-((5-iodopentyl)oxy)-7-methoxy-2-methyl-5-oxo-11,11a-dihydro-1H-benzo[e]pyrrolo[1,2-a][1,4]diazepine-10(5H)-carboxylate (**55**)*

Diiodopentane (0.63 mL, 4.21 mmol, 5 eq) and potassium carbonate (116 mg, 0.84 mmol, 1 eq) were added to a solution of phenol **54** (400 mg, 0.84 mmol) in acetone (4 mL, dried over molecular sieves). The reaction mixture was then warmed to 60°C and stirred for 6 hours. Acetone was removed by rotary evaporation under reduced pressure. The resulting residue was subjected to flash column chromatography (silica gel; 50/50, v/v, hexane/ethyl acetate,). Pure fractions were collected and combined and excess eluent was removed to provide **55** in 90% yield. LC/MS, 3.90 min (ES+) *m*/*z* (relative intensity) 670.91 ([M]$^+$, 100). $^1$H NMR (400 MHz, $CDCl_3$) δ 7.23 (s, 1H), 6.69 (s, 1H), 6.60 (s, 1H), 5.87 (d, *J* = 8.8 Hz, 1H), 5.83 - 5.68 (m, *J* = 5.6 Hz, 1H), 5.15 - 5.01 (m, 2H), 4.67 - 4.58 (m, 1H), 4.45 - 4.35 (m, 1H), 4.04 - 3.93 (m, 2H), 3.91 (s, 3H), 3.73 (td, *J* = 10.0, 3.8 Hz, 1H), 3.25 - 3.14 (m, *J* = 8.5, 7.0 Hz, 2H), 2.92 (dd, *J* = 16.8, 10.3 Hz, 1H), 2.38 (d, *J* = 16.8 Hz, 1H), 1.95 - 1.81 (m, 4H), 1.77 (s, 3H), 1.64 - 1.49 (m, 2H), 0.88 (s, 9H), 0.25 (s, 3H), 0.23 (s, 3H).

(iii) (11S,11aS)-4-(2-(1-((1-(allyloxy)-4-methyl-1,2-dioxopentan-3-yl)amino)-1-oxopropan-2-yl)hydrazinyl)benzyl 11-((tert-butyldimethylsilyl)oxy)-8-hydroxy-7-methoxy-2-methyl-5-oxo-11,11a-dihydro-1H-benzo[e]pyrrolo[1,2-a][1,4]diazepine-10(5H)-carboxylate (**70**)

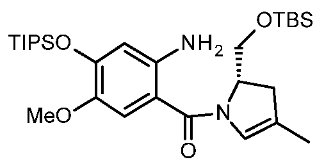

49

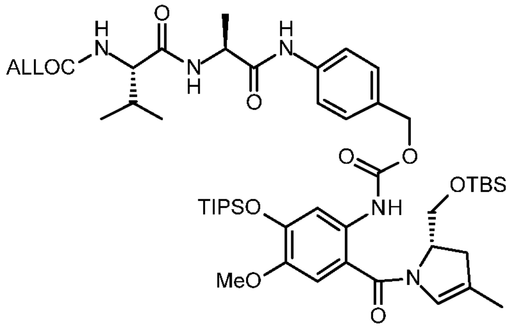

56

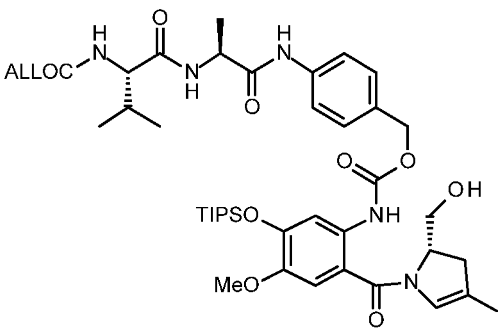

57

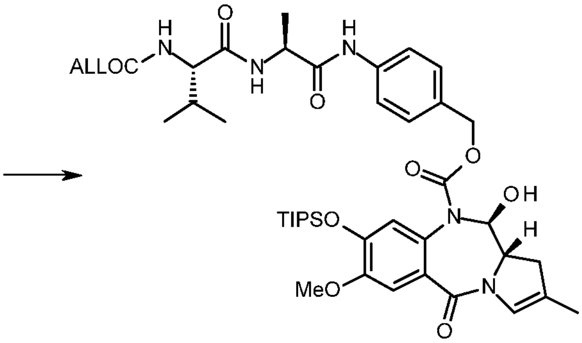

58

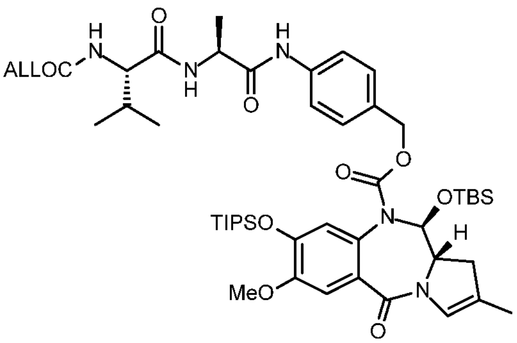

59

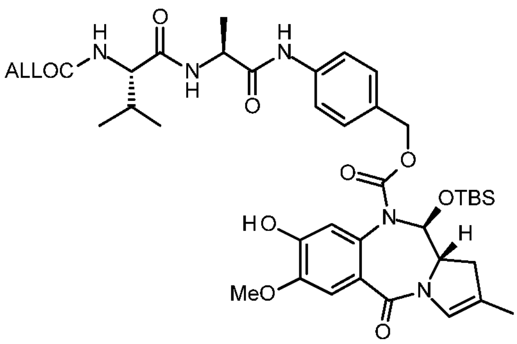

60

*(a) Allyl 3-(2-(2-(4-((((2-((S)-2-(((tert-butyldimethylsilyl)oxy)methyl)-4-methyl-2,3-dihydro-1H-pyrrole-1-carbonyl)-4-methoxy-5-((triisopropylsilyl)oxy)phenyl)carbamoyl)oxy)methyl)phenyl)hydrazinyl)propanamido)-4-methyl-2-oxopentanoate (**56**)*

Triethylamine (2.23 mL, 18.04 mmol, 2.2 eq) was added to a stirred solution of the amine **49** (4 g, 8.20 mmol) and triphosgene (778 mg, 2.95 mmol, 0.36 eq) in dry tetrahydrofuran (40 mL) at 5 °C (ice bath). The progress of the isocyanate reaction was monitored by periodically removing aliquots from the reaction mixture and quenching with methanol and performing LC/MS analysis. Once the isocyanate formation was complete a solution of the alloc-Val-Ala-PABOH (4.12 g, 12.30 mmol, 1.5 eq) and triethylamine (1.52 mL, 12.30 mmol, 1.5 eq) in dry tetrahydrofuran (40 mL) was rapidly added by injection to the freshly prepared isocyanate. The reaction mixture was allowed to stir at 40 °C for 4 hours. Excess solvent was removed by rotary evaporation under reduced pressure. The resulting residue was subjected to flash column chromatography (silica gel; gradient, 1 % methanol to 5% methanol in dichloromethane). (Alternative chromatography conditions using EtOAc and Hexane have also been successful). Pure fractions were collected and combined and excess eluent was removed by rotary evaporation under reduced pressure to give the product **56** (3.9 g, 50%). LC/MS, 4.23 min (ES+) *m/z* (relative intensity) 952.36 ([*M* + H]$^{+\cdot}$, 100); $^1$H NMR (400 MHz, $CDCl_3$) δ 8.62 (br s, 1H), 8.46 (s, 1H), 7.77 (br s, 1H), 7.53 (d, *J* = 8.4 Hz, 2H), 7.32 (d, *J* = 8.5 Hz, 2H), 6.76 (s, 1H), 6.57 (d, *J* = 7.6 Hz, 1H), 6.17 (s, 1H), 6.03 - 5.83 (m, 1H), 5.26 (dd, *J* = 33.8, 13.5 Hz, 3H), 5.10 (s, 2H), 4.70 - 4.60 (m, 2H), 4.58 (dd, *J* = 5.7, 1.3 Hz, 2H), 4.06 - 3.99 (m, 1H), 3.92 (s, 1H), 3.82 - 3.71 (m, 1H), 3.75 (s, 3H), 2.79 - 2.64 (m, 1H), 2.54 (d, *J* = 12.9 Hz, 1H), 2.16 (dq, *J* = 13.5, 6.7 Hz, 1H), 1.67 (s, 3H), 1.46 (d, *J* = 7.0 Hz, 3H), 1.35 - 1.24 (m, 3H), 1.12 (s, 9H),

1.10 (s, 9H), 0.97 (d, *J* = 6.8 Hz, 3H), 0.94 (d, *J* = 6.8 Hz, 3H), 0.87 (s, 9H), 0.07 - -0.02 (m, 6H).

*(b) Allyl 3-(2-(2-(4-((((2-((S)-2-(hydroxymethyl)-4-methyl-2,3-dihydro-1H-pyrrole-1-carbonyl)-4-methoxy-5-((triisopropylsilyl)oxy)phenyl)carbamoyl)oxy)methyl)phenyl)hydrazinyl)propanamido)-4-methyl-2-oxopentanoate (**57**)*

The TBS ether **56** (1.32 g, 1.38 mmol) was dissolved in a 7:1:1:2 mixture of acetic acid/methanol/tetrahydrofuran/water (14:2:2:4 mL) and allowed to stir at room temperature. After 3 hours no more starting material was observed by LC/MS. The reaction mixture was diluted with ethyl acetate (25 mL) and washed sequentially with water, saturated aqueous sodium bicarbonate and brine. The organic phase was dried over magnesium sulphate filtered and excess ethyl acetate removed by rotary evaporation under reduced pressure. The resulting residue was subjected to flash column chromatography (silica gel, 2% methanol in dichloromethane). Pure fractions were collected and combined and excess eluent was removed by rotary evaporation under reduced pressure to afford the desired product **57** (920 mg, 80%). LC/MS, 3.60 min (ES+) *m/z* (relative intensity) 838.18 ($[M+H]^{+\cdot}$, 100). $^1H$ NMR (400 MHz, $CDCl_3$) $\delta$ 8.55 (s, 1H), 8.35 (s, 1H), 7.68 (s, 1H), 7.52 (d, *J* = 8.1 Hz, 2H), 7.31 (d, *J* = 8.4 Hz, 2H), 6.77 (s, 1H), 6.71 (d, *J* = 7.5 Hz, 1 H), 6.13 (s, 1H), 5.97 - 5.82 (m, *J* = 5.7 Hz, 1H), 5.41 - 5.15 (m, 3H), 5.10 (d, *J* = 3.5 Hz, 2H), 4.76 - 4.42 (m, 5H), 4.03 (t, *J* = 6.6 Hz, 1H), 3.77 (s, 5H), 2.84 (dd, *J* = 16.7, 10.4 Hz, 1H), 2.26 - 2.08 (m, 2H), 1.68 (s, 3H), 1.44 (d, *J* = 7.0 Hz, 3H), 1.30 (dt, *J* = 14.7, 7.4 Hz, 3H), 1.12 (s, 9H), 1.10 (s, 9H), 0.96 (d, *J* = 6.8 Hz, 3H), 0.93 (d, *J* = 6.8 Hz, 3H).

*(c) (11S,11aS)-4-(2-(1-((1-(allyloxy)-4-methyl-1,2-dioxopentan-3-yl)amino)-1-oxopropan-2-yl)hydrazinyl)benzyl 11-hydroxy-7-methoxy-2-methyl-5-oxo-8-((triisopropylsilyl)oxy)-11,11a-dihydro-1H-benzo[e]pyrrolo[1,2-a][1,4]diazepine-10(5H)-carboxylate (58)*

Dimethyl sulphoxide (0.2 mL, 2.75 mmol, 2.5 eq) was added dropwise to a solution of oxalyl chloride (0.11 mL, 1.32 mmol, 1.2 eq) in dry dichloromethane (7 mL) at -78°C (dry ice /acetone bath) under an atmosphere of argon. After 10 minutes a solution of **57** (920 mg, 1.10 mmol) in dry dichloromethane (5 mL) was added slowly with the temperature still at - 78°C. After 15 min triethylamine (0.77 mL, dried over 4Å molecular sieves, 5.50 mmol, 5 eq) was added dropwise and the dry ice/acetone bath was removed. The reaction mixture was allowed to reach room temperature and was extracted with cold hydrochloric acid (0.1 M), saturated aqueous sodium bicarbonate and brine. The organic phase was dried over magnesium sulphate, filtered and excess dichloromethane was removed by rotary evaporation under reduced pressure. The resulting residue was subjected to column flash chromatography (silica gel; gradient 2% methanol to 5 % methanol in dichloromethane). Pure fractions were collected and combined and removal of excess eluent by rotary evaporation under reduced pressure afforded the product **58** (550 mg, 60%). LC/MS, 3.43 min (ES+) m/z (relative intensity) 836.01 ($[M]^{+\cdot}$, 100). $^1H$ NMR (400 MHz, $CDCl_3$) $\delta$ 8.39 (s, 1H), 7.52 - 7.40 (m, 2H), 7.21 - 7.08 (m, *J* = 11.5 Hz, 2H), 6.67 (s, 1H), 6.60 - 6.47 (m, *J* = 7.4 Hz, 1H), 5.97 - 5.83 (m, 1H), 5.79 - 5.66 (m, 1H), 5.38 - 4.90 (m, 6H), 4.68 - 4.52 (m, *J* = 18.4, 5.5 Hz, 4H), 4.04 - 3.94 (m, *J* = 6.5 Hz, 1H), 3.87 - 3.76 (m, 5H), 3.00 - 2.88 (m, 1 H), 2.66 - 2.49 (m, 2H), 2.21 - 2.08 (m, 2H), 1.76 (s, 3H), 1.45 (d, *J* = 7.0 Hz, 3H), 1.09 - 0.98 (m, *J* = 8.9 Hz, 18H), 0.96 (d, *J* = 6.7 Hz, 3H), 0.93 (d, *J* = 6.9 Hz, 3H).

*(d) (11S,11aS)-4-(2-(1-((1-(Allyloxy)-4-methyl-1,2-dioxopentan-3-yl)amino)-1-oxopropan-2-yl)hydrazinyl)benzyl 11-((tert-butyldimethylsilyl)oxy)-7-methoxy-2-methyl-5-oxo-8-((triisopropylsilyl)oxy)-11,11a-dihydro-1H-benzo[e]pyrrolo[1,2-a][1,4]diazepine-10(5H)-carboxylate (**59**)*

*Tert*-butyldimethylsilyltriflate (0.38 mL, 1.62 mmol, 3 eq) was added to a solution of compound **58** (450 mg, 0.54 mmol) and 2,6-lutidine (0.25 mL, 2.16 mmol, 4 eq) in dry dichloromethane (5 mL) at 0°C under argon. After 10 min, the cold bath was removed and the reaction mixture was stirred at room temperature for 1 hour. The reaction mixture was extracted with water, saturated aqueous sodium bicarbonate and brine. The organic phase was dried over magnesium sulphate, filtered and excess solvent was removed by rotary evaporation under reduced pressure. The resulting residue was subjected to column flash chromatography (silica gel; 50/50 v/v hexane/ethyl acetate). Pure fractions were collected and combined and excess eluent was removed by rotary evaporation under reduced pressure to give the product **59** (334 mg, 65%). LC/MS, 4.18 min (ES+) *m/z* (relative intensity) 950.50 ($[M]^{+\cdot}$, 100). $^1H$ NMR (400 MHz, $CDCl_3$) $\delta$ 8.53 (s, 1H), 8.02 (s, 1H), 7.44 (d, *J* = 7.6 Hz, 2H), 7.21 (s, 1H), 7.08 (d, *J* = 8.2 Hz, 2H), 6.72 - 6.61 (m, *J* = 8.9 Hz, 2H), 6.16 (s, 1H), 5.97 - 5.79 (m, *J* = 24.4, 7.5 Hz, 2H), 5.41 - 5.08 (m, 5H), 4.86 (d, *J* = 12.5 Hz, 1H), 4.69 - 4.60 (m, 1H), 4.57 (s, 1H), 4.03 (t, *J* = 6.7 Hz, 1H), 3.87 (s, 3H), 3.74 (td, *J* = 9.6, 3.6 Hz, 1H), 2.43 - 2.09 (m, *J* = 34.8, 19.4, 11.7 Hz, 3H), 1.76 (s, 3H), 1.43 (d, *J* = 6.9 Hz, 3H), 1.30 - 1.21 (m, 3H), 0.97 (d, *J* = 6.7 Hz, 3H), 0.92 (t, *J* = 8.4 Hz, 3H), 0.84 (s, 9H), 0.23 (s, 3H), 0.12 (s, 3H).

*(e) (11S,11aS)-4-(2-(1-((1-(Allyloxy)-4-methyl-1,2-dioxopentan-3-yl)amino)-1-oxopropan-2-yl)hydrazinyl)benzyl 11-((tert-butyldimethylsilyl)oxy)-8-hydroxy-7-methoxy-2-methyl-5-oxo-11,11a-dihydro-1H-benzo[e]pyrrolo[1,2-a][1,4]diazepine-10(5H)-carboxylate (**60**)*

Lithium acetate (50 mg, 0.49 mmol) was added to a solution of compound **59** (470 mg, 0.49 mmol) in wet dimethylformamide (4 mL, 50:1 DMF/water). After 4 hours, the reaction was complete and the reaction mixture was diluted with ethyl acetate and washed with citric acid (pH ~ 3), water and brine. The organic layer was dried over magnesium sulphate filtered and excess ethyl acetate was removed by rotary evaporation under reduced pressure. The resulting residue was subjected to column flash chromatography (silica gel; gradient, 50/50 to 25/75 v/v hexane/ethyl acetate). Pure fractions were collected and combined and excess eluent was removed by rotary evaporation under reduced pressure to give the product **60** (400 mg, quantitative). LC/MS, 3.32 min (ES+) *m/z* (relative intensity) 794.18 ($[M+H]^{+\cdot}$, 100). $^1H$ NMR (400 MHz, $CDCl_3$) δ 8.53 (s, 1H), 8.02 (s, 1H), 7.44 (d, *J* = 7.6 Hz, 2H), 7.21 (s, 1H), 7.08 (d, *J* = 8.2 Hz, 2H), 6.72 - 6.61 (m, *J* = 8.9 Hz, 2H), 6.16 (s, 1H), 5.97 - 5.79 (m, *J* = 24.4, 7.5 Hz, 2H), 5.41 - 5.08 (m, 5H), 4.86 (d, *J* = 12.5 Hz, 1H), 4.69 - 4.60 (m, 1H), 4.57 (s, 1H), 4.03 (t, *J* = 6.7 Hz, 1H), 3.87 (s, 3H), 3.74 (td, *J* = 9.6, 3.6 Hz, 1H), 2.43 - 2.09 (m, *J* = 34.8, 19.4, 11.7 Hz, 3H), 1.76 (s, 3H), 1.43 (d, *J* = 6.9 Hz, 3H), 1.30 - 1.21 (m, 3H), 0.97 (d, *J* = 6.7 Hz, 3H), 0.92 (t, *J* = 8.4 Hz, 3H), 0.84 (s, 9H), 0.23 (s, 3H), 0.12 (s, 3H).

(iv) (11S,11aS)-4-((2S,5S)-37-(2,5-dioxo-2,5-dihydro-1H-pyrrol-1-yl)-5-isopropyl-2-methyl-4,7,35-trioxo-10,13,16,19,22,25,28,31-octaoxa-3,6,34-triazaheptatriacontanamido)benzyl 11-hydroxy-7-methoxy-8-((5-(((S)-7-methoxy-2-methyl-5-oxo-5,11a-dihydro-1H-benzo[e]pyrrolo[1,2-a][1,4]diazepin-8-yl)oxy)pentyl)oxy)-2-methyl-5-oxo-11,11a-dihydro-1H-benzo[e]pyrrolo[1,2-a[[1,4]diazepine-10(5H)-carboxylate (**64**)

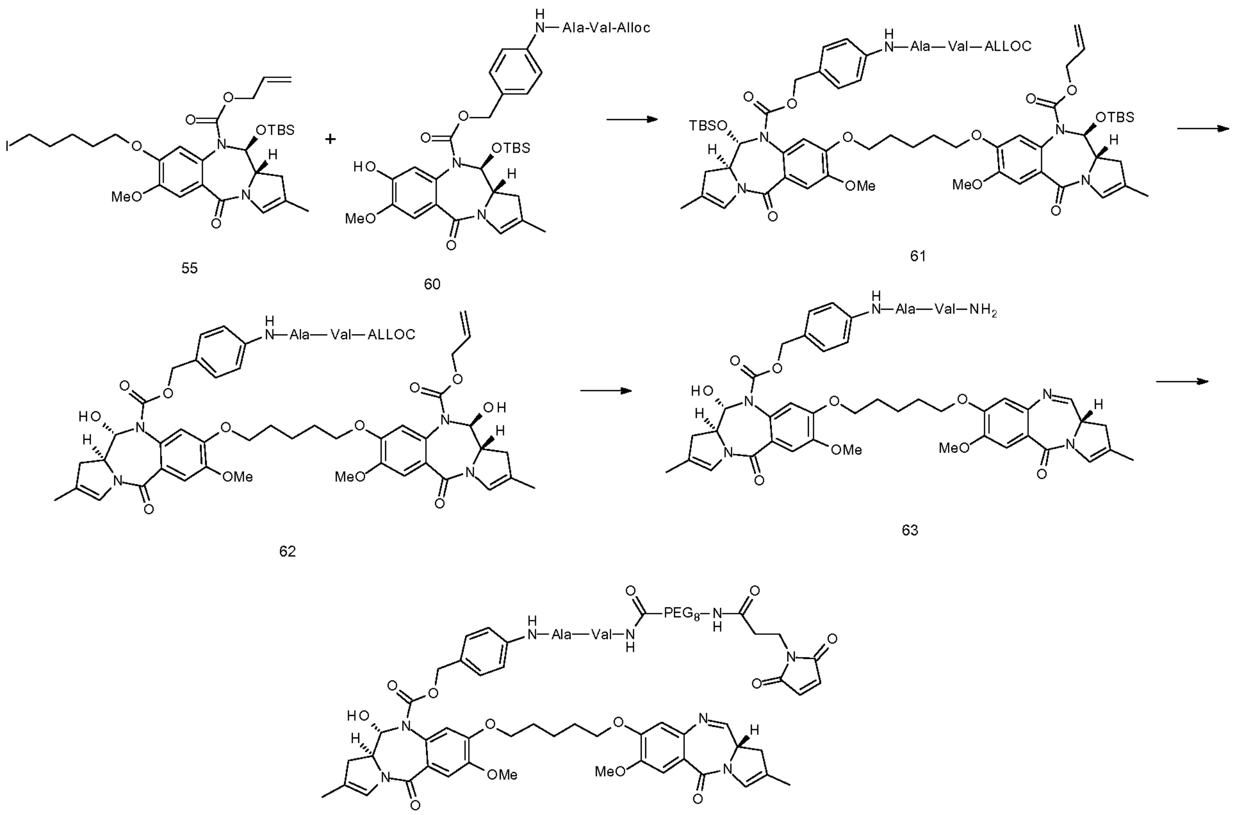

55 60 61

62 63

64

(a) *(11S)-allyl 8-((5-(((11S)-10-(((4-(2-(1-((1-(allyloxy)-4-methyl-1,2-dioxopentan-3-yl)amino)-1-oxopropan-2-yl)hydrazinyl)benzyl)oxy)carbonyl)-11-((tert-butyldimethylsilyl)oxy)-7-methoxy-2-methyl-5-oxo-5,10,11,11a-tetrahydro-1H-benzo[e]pyrrolo[1,2-a][1,4]diazepin-8-yl)oxy)pentyl)oxy)-11-((tert-butyldimethylsilyl)oxy)-7-methoxy-2-methyl-5-oxo-11,11a-dihydro-1H-benzo[e]pyrrolo[1,2-a][1,4]diazepine-10(5H)-carboxylate (**61**)*

Potassium carbonate (70 mg, 0.504 mmol, 1 eq) was added to a solution of **55** (370 mg, 0.552 mmol, 1.2 eq)

and phenol **60** (400 mg, 0.504 mmol) in dry acetone (25 mL). The reaction was stirred 8 hours at 70°C. The LC/MS showed that all the starting material was not consumed, so the reaction was allowed to stir overnight at room temperature and stirred for an additional 2 hours the next day. Acetone was removed by rotary evaporation under reduced pressure. The resulting residue was subjected to flash column chromatography (silica gel; 80% ethyl acetate in hexane to 100% ethyl acetate). Pure fractions were collected and combined and excess eluent was removed by rotary evaporation under reduced pressure to give the product **61** (385 mg, 57%). LC/MS, 4.07 min (ES+) *m/z* (relative intensity) 1336.55 ($[M+H]^{+\cdot}$, 50).

*(b) (11S)-allyl 8-((5-(((11S)-10-(((4-(2-(1-((1-(allyloxy)-4-methyl-1,2-dioxopentan-3-yl)amino)-1-oxopropan-2-yl)hydrazinyl)benzyl)oxy)carbonyl)-11-hydroxy-7-methoxy-2-methyl-5-oxo-5,10,11,11a-tetrahydro-1H-benzo[e]pyrrolo[1,2-a][1,4]diazepin-8-yl)oxy)pentyl)oxy)-11-hydroxy-7-methoxy-2-methyl-5-oxo-11,11a-dihydro-1H-benzo[e]pyrrolo[1,2-a][1,4]diazepine-10(5H)-carboxylate (**62**)*

Tetra-*n*-butylammonium fluoride (1 M, 0.34 mL, 0.34 mmol, 2 eq) was added to a solution of **61** (230 mg, 0.172 mmol) in dry tetrahydrofuran (3 mL). The starting material was totally consumed after 10 minutes. The reaction mixture was diluted with ethyl acetate (30 mL) and washed sequentially with water and brine. The organic phase was dried over magnesium sulphate filtered and excess ethyl acetate removed by rotary evaporation under reduced pressure. The resulting residue **62** was used as a crude mixture for the next reaction. LC/MS, 2.87 min (ES+) *m/z* (relative intensity) 1108.11 ($[M+H]^{+\cdot}$, 100).

*(c) (11S)-4-(2-(1-((1-amino-3-methyl-1-oxobutan-2-yl)amino)-1-oxopropan-2-yl)hydrazinyl)benzyl 11-hydroxy-7-methoxy-8-((5-((7-methoxy-2-methyl-5-oxo-5,11a-dihydro-1H-benzo[e]pyrrolo[1,2-a][1,4]diazepin-8-yl)oxy)pentyl)oxy)-2-methyl-5-oxo-11,11a-dihydro-1H-benzo[e]pyrrolo[1,2-a][1,4]diazepine-10(5H)-carboxylate (**63**)*

*Tetrakis*(triphenylphosphine)palladium(0) (12 mg, 0.01 mmol, 0.06 eq) was added to a solution of crude **62** (0.172 mmol) and pyrrolidine (36 μL, 0.43 mmol, 2.5 eq) in dry dichloromethane (10 mL). The reaction mixture was stirred 20 minutes and diluted with dichloromethane and washed sequentially with saturated aqueous ammonium chloride and brine. The organic phase was dried over magnesium sulphate filtered and excess dichloromethane removed by rotary evaporation under reduced pressure. The resulting residue **63** was used as a crude mixture for the next reaction. LC/MS, 2.38 min (ES+) *m/z* (relative intensity) 922.16 ($[M+H]^{+\cdot}$, 40).

*(d) (11S,11aS)-4-((2S,5S)-37-(2,5-dioxo-2,5-dihydro-1H-pyrrol-1-yl)-5-isopropyl-2-methyl-4,7,35-trioxo-10,13,16,19,22,25,28,31-octaoxa-3,6,34-triazaheptatriacontanamido)benzyl 11-hydroxy-7-methoxy-8-((5-(((S)-7-methoxy-2-methyl-5-oxo-5,11a-dihydro-1H-benzo[e]pyrrolo[1,2-a][1,4]diazepin-8-yl)oxy)pentyl)oxy)-2-methyl-5-oxo-11,11a-dihydro-1H-benzo[e]pyrrolo[1,2-a][1,4]diazepine-10(5H)-carboxylate (**64**)*

1-ethyl-3-(3'-dimethylaminopropyl)carbodiimide (EDCl, 33 mg, 0.172 mmol) was added to a solution of crude **63** (0.172 mmol) and Mal-$(PEG)_8$-acid (100 mg, 0.172 mmol) in dry dichloromethane (10 mL). The reaction was stirred for 2 hours and the presence ofstarting material was no longer observed by LC/MS. The reaction was diluted with dichloromethane and washed sequentially with water and brine. The organic phase was dried over magnesium sulphate filtered and excess dichloromethane removed by rotary evaporation under reduced pressure. The resulting residue was subjected to flash column chromatography (silica gel; 100% chloroform to 10% methanol in chloroform). Pure fractions were collected and combined and excess eluent was removed by rotary evaporation under reduced pressure to give **64 (E)** (60 mg, 25% over 3 steps).

**Example 8**

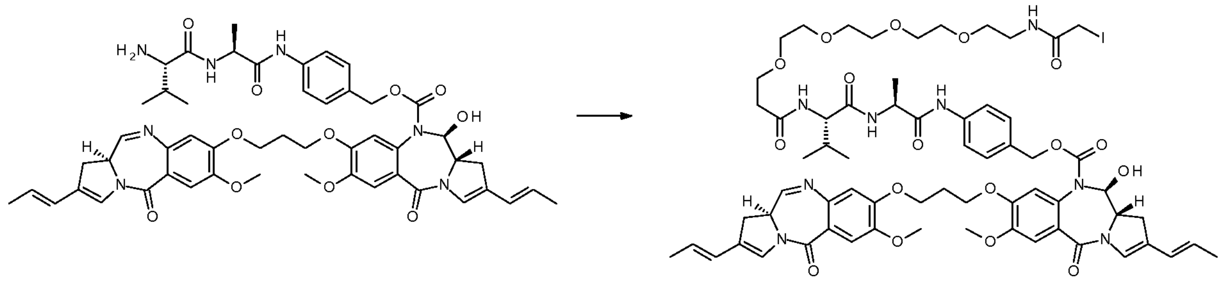

65 66

Compound 65 is compound 79 of WO 2011/130598

*(11S)-4-(1-iodo-20-isopropyl-23-methyl-2,18,21-trioxo-6,9,12,15-tetraoxa-3,19,22-triazatetracosanamido)benzyl 11-hydroxy-7-methoxy-8-(3-((7-methoxy-5-oxo-2-((E)-prop-1-en-1-yl)-5,11a-dihydro-1H-benzo[e]pyrrolo[1,2-a][1,4]diazepin-8-yl)oxy)propoxy)-5-oxo-2-((E)-prop-1-en-1-yl)-11,11a-dihydro-1H-benzo[e]pyrrolo[1,2-a][1,4]diazepine-10(5H)-carboxylate (**66**)*

*N,N'*-diisopropylcarbodiimide (DIC, 4.71 μL, 0.0304 mmol) was added to a solution of amine **65** (0.0276 mmol) and Iodo-$(PEG)_4$-acid (13.1 mg, 0.0304 mmol) in dry dichloromethane (0.8 mL). The reaction was stirred for 3 hours and the presence of starting material was no longer observed by LC/MS. The reaction mixture was directly loaded onto a thin-layer chromatography (TLC) plate and purified by prep-TLC (10% methanol in chloroform). Pure bands were scraped off the TLC plate, taken up in 10% methanol in chloroform, filtered and excess eluent removed by rotary evaporation under reduced pressure to give **66** (D) (20.9 mg, 56%). LC/MS, method 2, 3.08 min (ES+) *m/z* (relative intensity) 1361.16 ($[M+H]^+$, 100).

**General Experimental Methods for Example 9**

LCMS data were obtained using an Agilent 1200 series LC/MS with an Agilent 6110 quadrupole MS, with Electrospray ionisation. Mobile phase A - 0.1% Acetic acid in water. Mobile Phase B - 0.1 % in acetonitrile. Flow rate of 1.00ml/min. Gradient from 5% B rising up to 95% B over 3 minutes, remaining at 95% B for 1 minute and then back down to 5% B over 6 seconds. The total run time is 5 minutes. Column: Phenomenex Gemini-NX 3μm C18, 30 x 2.00mm. Chromatograms based on UV detection at 254nm. Mass Spectra were achieved using the MS in positive mode. Proton NMR chemical shift values were measured on the delta scale at 400 MHz using a Bruker AV400. The following abbreviations have been used: s, singlet; d, doublet; t, triplet; q, quartet; m, multiplet; br, broad. Coupling constants are reported in Hz. Unless otherwise stated, column chromatography (by the flash procedure) were performed on Merck Kieselgel silica (Art. 9385). Mass spectroscopy (MS) data were collected using a Waters Micromass LCT instrument coupled to a Waters 2795 HPLC separations module. Thin Layer Chromatography (TLC) was performed on silica gel aluminium plates (Merck 60, $F_{254}$). All other chemicals and solvents were purchased from Sigma-Aldrich or Fisher Scientific and were used as supplied without further purification.

Optical rotations were measured on an ADP 220 polarimeter (Bellingham Stanley Ltd.) and concentrations (*c*) are given in g/100mL. Melting points were measured using a digital melting point apparatus (Electrothermal). IR spectra were recorded on a Perkin-Elmer Spectrum 1000 FT IR Spectrometer. $^1H$ and $^{13}C$ NMR spectra were acquired at 300 K using a Bruker Avance NMR spectrometer at 400 and 100 MHz, respectively. Chemical shifts are reported relative to TMS (δ = 0.0 ppm), and signals are designated as s (singlet), d (doublet), t (triplet), dt (double triplet), dd (doublet of doublets), ddd (double doublet of doublets) or m (multiplet), with coupling constants given in Hertz (Hz). Mass spectroscopy (MS) data were collected using a Waters Micromass ZQ instrument coupled to a Waters 2695 HPLC with a Waters 2996 PDA. Waters Micromass ZQ parameters used were: Capillary (kV), 3.38; Cone (V), 35; Extractor (V), 3.0; Source temperature (°C), 100; Desolvation Temperature (°C), 200; Cone flow rate (L/h), 50; De-solvation flow rate (L/h), 250. High-resolution mass spectroscopy (HRMS) data were recorded on a Waters Micromass QTOF Global in positive W-mode using metal-coated borosilicate glass tips to introduce the samples into the instrument. Thin Layer Chromatography (TLC) was performed on silica gel aluminium plates (Merck 60, $F_{254}$), and flash chromatography utilised silica gel (Merck 60, 230-400 mesh ASTM). Except for the HOBt (NovaBiochem) and solid-supported reagents (Argonaut), all other chemicals and solvents were purchased from Sigma-Aldrich and were used as supplied without further purification. Anhydrous solvents were prepared by distillation under a dry nitrogen atmosphere in the presence of an appropriate drying agent, and were stored over 4Å molecular sieves or sodium wire. Petroleum ether refers to the fraction boiling at 40-60°C.

General LC/MS conditions: The HPLC (Waters Alliance 2695) was run using a mobile phase of water (A) (formic

acid 0.1%) and acetonitrile (B) (formic acid 0.1%). Gradient: initial composition 5% B over 1.0 min then 5% B to 95% B within 3 min. The composition was held for 0.5 min at 95% B, and then returned to 5% B in 0.3 minutes. Total gradient run time equals 5 min. Flow rate 3.0 mL/min, 400μL was split *via* a zero dead volume tee piece which passes into the mass spectrometer. Wavelength detection range: 220 to 400 nm. Function type: diode array (535 scans). Column: Phenomenex® Onyx Monolithic C18 50 x 4.60 mm

**Example 9**

(i) Key Intermediates

(a)

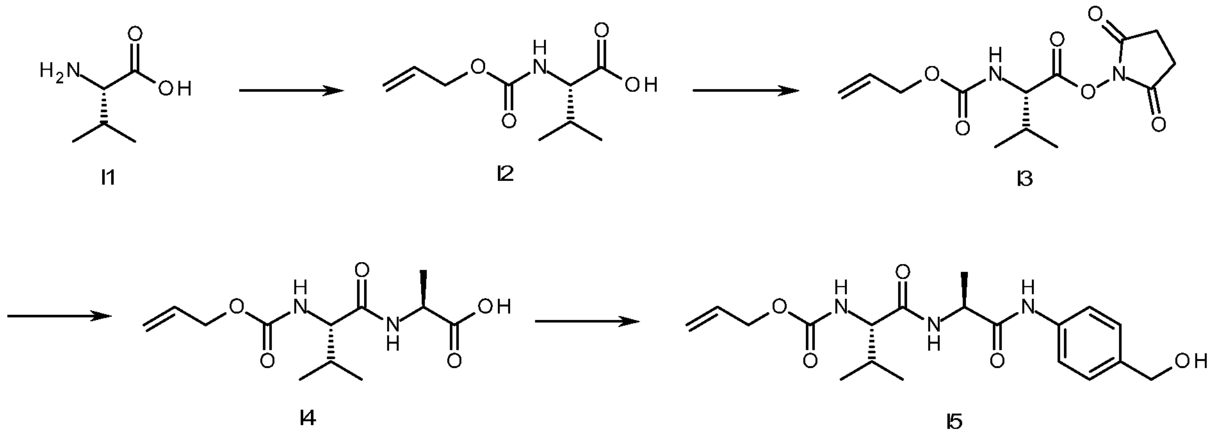

I1 I2 I3 I4 I5

*(a-i) (S)-2-(allyloxycarbonylamino)-3-methylbutanoic acid (**I2**)*

Allyl chloroformate (36.2 ml, 340.59 mmol, 1.2 eq) was added dropwise to a stirred solution of L-valine (**I1**)(33.25 g, 283.82 mmol, 1.0 eq) and potassium carbonate (59.27 g, 425.74 mmol, 1.5 eq) in water (650 mL) and THF (650 mL). The reaction mixture was stirred at room temperature for 18 hours, then the solvent was concentrated under reduced pressure and the remaining solution extracted with diethyl ether (3 x 100 mL). The aqueous portion was acidified to pH 2 with conc. HCl and extracted with DCM (3 x 100 mL). The combined organics were washed with brine, dried over $MgSO_4$, filtered and concentrated under reduced pressure to afford the product as a colourless oil (57.1 g, assumed 100% yield). LC/MS (1.966 min ($ES^+$)), *m/z:* 202.1 $[M+H]^+$. $^1H$ NMR (400 MHz, DMSO-$d_6$) δ 12.57 (br s, 1H), 7.43 (d, 1H, *J* = 8.6 Hz), 5.96 - 5.86 (m, 1H), 5.30 (ddd, 1H, *J* = 17.2, 3.4, 1.7 Hz), 5.18 (ddd, 1H, *J* = 10.4, 2.9, 1.6 Hz), 4.48 (dt, 2H, *J* = 5.3, 1.5 Hz), 3.85 (dd, 1H, *J* = 8.6, 6.0 Hz), 2.03 (oct, 1H, *J* = 6.6 Hz), 0.89 (d, 3H, *J* = 6.4 Hz), 0.87 (d, 3H, *J* = 6.5 Hz).

*(a-ii) (S)-2,5-dioxopyrrolidin-1-y12-(allyloxycarbonylamino)-3-methylbutanoate (**I3**)*

To a stirred solution of the protected acid **I2** (60.6 g, 301.16 mmol, 1.0 eq) and N-hydroxysuccinimide (34.66 g, 301.16 mmol, 1.0 eq) in dry THF (800 mL) was added dicyclohexylcarbodiimide (62.14 g, 301.16 mmol, 1 eq). The reaction was stirred for 18 hours at room temperature. The reaction mixture was then filtered, the solid washed with THF and the combined filtrate was concentrated under reduced pressure. The residue was redissolved in DCM and left to stand at 0°C for 30 minutes. The suspension was filtered and washed with cold DCM. Concentration of the filtrate under reduced pressure afforded the product as a viscous colourless oil (84.7 g, assumed 100% yield) which was used in the next step without further purification. LC/MS (2.194 min ($ES^+$)), *m/z:* 321.0 $[M+Na]^+$. $^1H$ NMR (400 MHz, DMSO-$d_6$) δ 8.0 (d, 1H, *J* = 8.3 Hz), 5.97 - 5.87 (m, 1H), 5.30 (ddd, 1H, *J* = 17.2, 3.0, 1.7 Hz), 5.19 (ddd, 1H, *J* = 10.4, 2.7, 1.4 Hz), 4.52 (dt, 2H, *J* = 5.3, 1.4 Hz), 4.32 (dd, 1H, *J* = 8.3, 6.6 Hz), 2.81 (m, 4H), 2.18 (oct, 1H, *J* = 6.7 Hz), 1.00 (d, 6H, *J* = 6.8 Hz),

*(a-iii) (S)-2-((S)-2-(allyloxycarbonylamino)-3-methylbutanamido)propanoic acid (14)*

A solution of succinimide ester **I3**(12.99 g, 43.55 mmol, 1.0 eq) in THF (50 mL) was added to a solution of L-alanine (4.07 g, 45.73 mmol, 1.05 eq) and $NaHCO_3$ (4.02 g, 47.90 mmol, 1.1 eq) in THF (100 mL) and $H_2O$ (100 mL). The mixture was stirred at room temperature for 72 hours when the THF was removed under reduced pressure. The

pH was adjusted to 3-4 with citric acid to precipitate a white gum. After extraction with ethyl acetate (6 x 150 mL), the combined organics were washed with $H_2O$ (200 mL), dried over $MgSO_4$, filtered and concentrated under reduced pressure. Trituration with diethyl ether afforded the product as a white powder which was collected by filtration and washed with diethyl ether (5.78 g, 49%). LC/MS (1.925 min (ES$^+$)), *m/z*: 273.1 [M+H]$^+$. $^1$H NMR (400 MHz, DMSO-$d_6$) δ 12.47 (br s, 1H), 8.17 (d, 1H, *J* = 6.8 Hz), 7.16 (d, 1H, *J* = 9.0 Hz), 5.95 - 5.85 (m, 1H), 5.29 (dd, 1H, *J* = 17.2, 1.7 Hz), 5.17 (dd, 1H, *J* = 10.4, 1.5 Hz), 4.46 (m, 2H), 4.18 (quin, 1H, *J* = 7.2 Hz), 3.87 (dd, 1H, *J* = 9.0, 7.1 Hz), 1.95 (oct, 1H, *J* = 6.8 Hz), 1.26 (d, 3H, *J* = 7.3 Hz), 0.88 (d, 3H, *J* = 6.8 Hz), 0.83 (d, 3H, *J* = 6.8 Hz).

*(a-iv) Allyl (S)-1-((S)-1-(4-(hydroxymethyl)phenylamino)-1-oxopropan-2-ylamino)-3-methyl-1-oxobutan-2-ylcarbamate **(I5)***

EEDQ (5.51 g, 22.29 mmol, 1.05 eq) was added to a solution of *p*-aminobenzyl alcohol (2.74 g, 22.29 mmol, 1.05 eq) and acid **I4** (5.78 g, 21.23 mmol, 1 eq) in dry THF (100 mL). and stirred at room temperature for 72 hours. The reaction mixture was then concentrated under reduced pressure and the resulting brown solid was triturated with diethyl ether and filtered with subsequent washing with an excess of diethyl ether to afford the product as an off-white solid (7.1 g, 88 %). LC/MS (1.980 min (ES$^+$)), *m/z:* 378.0 [M+H]$^+$. $^1$H NMR (400 MHz, DMSO-$d_6$) δ 9.89 (br s, 1H), 8.13 (d, 1H, *J* = 7.0 Hz), 7.52 (d, 2H, *J* = 8.5 Hz), 7.26 (m, 1H), 7.23 (d, 2H, *J* = 8.5 Hz), 5.91 (m, 1H), 5.30 (m, 1H), 5.17 (m, 1H), 4.46 (m, 2H), 5.09 (t, 1H, *J* = 5.6 Hz), 4.48 (m, 2H), 4.42 (m, 3H), 3.89 (dd, 1H, *J* = 8.6, 6.8 Hz), 1.97 (m, 1H), 1.30 (d, 3H, *J* = 7.1 Hz), 0.88 (d, 3H, *J* = 6.8 Hz), 0.83 (d, 3H, *J* = 6.7 Hz).

(b)

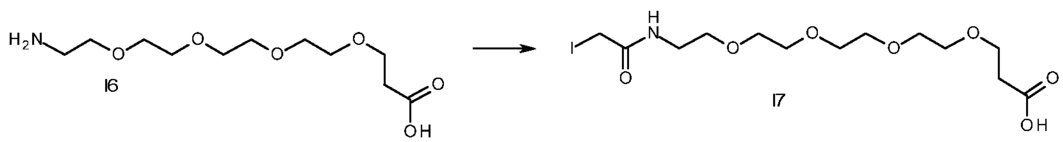

*1-iodo-2-oxo-6,9,12,15-tetraoxa-3-azaoctadecan-18-oic acid **(I7)***

A solution of iodoacetic anhydride (0.250 g, 0.706 mmol, 1.1 eq) in dry DCM (1 mL) was added to amino-PEG(4)-acid **I6** (0.170 g, 0.642 mmol, 1.0 eq) in DCM (1 mL). The mixture was stirred in the dark at room temperature overnight. The reaction mixture was washed with 0.1 M HCl, water, dried over $MgSO_4$, filtered and concentrated under reduced pressure. The residue was purified by flash chromatography (silica gel, 3% MeOH and 0.1% formic acid in chloroform to 10% MeOH and 0.1% formic acid in chloroform) to afford the product as an orange oil (0.118 g, 42%). LC/MS (1.623 min (ES$^+$)), *m/z*: 433..98 [M+H]$^+$. $^1$H NMR (400 MHz, $CDCl_3$) δ 8.069 (s, 1H), 7.22 (br s, 1H), 3.79 (t, 2H, *J* = 5.8 Hz), 3.74 (s, 2H), 3.72 - 3.58 (m, 14H), 3.50 - 3.46 (m, 2H), 2.62 (t, 2H, *J* = 5.8 Hz).

(ii) (11S,11aS)-allyl 11-(tert-butyldimethylsilyloxy)-8-(3-iodopropoxy)-7-methoxy-5-oxo-2-((E)-prop-1-enyl)-11,11a-dihydro-1H-benzo[e]pyrrolo[1,2-a][1,4]diazepine-10(5H)-carboxylate (**74**)

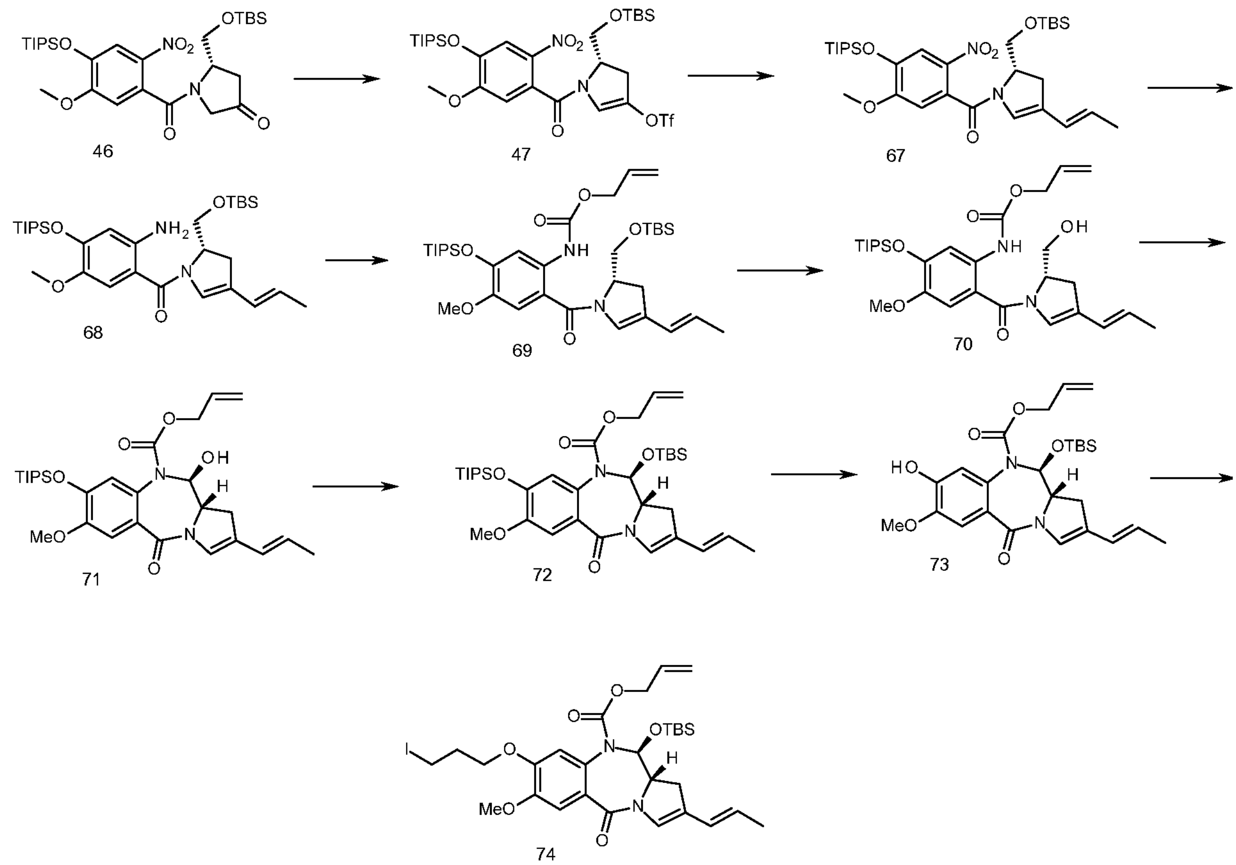

46 47 67 68 69 70 71 72 73 74

*(a) (S)-5-((tert-butyldimethylsilyloxy)methyl)-1-(5-methoxy-2-nitro-4-(triisopropylsilyloxy)benzoyl)-4,5-dihydro-1H-pyrrol-3-yl trifluoromethanesulfonate (**47**)*

Triflic anhydride (28.4 g, 100.0 mmol, 3.0 eq) was added dropwise, over 25 mins, to a vigorously stirred solution of the ketone 46 (19.5 g, 30.0 mmol, 1.0 eq) in DCM (550 mL) containing 2,6-lutidine (14.4 g, 130.0 mmol, 4.0 eq) at -50°C. The reaction mixture was stirred for 1.5 hours when LC/MS indicated complete reaction. The organic phase was washed successively with water (100 mL), saturated sodium bicarbonate (150 mL), brine (50 mL), and the organic phase was dried over $MgSO_4$, filtered and concentrated under reduced pressure. The residue was purified by flash chromatography (silica gel, 90/10 v/v *n*-hexane/EtOAc) to afford the product as a pale yellow oil (19.5 g, 82 %). LC/MS (4.391 min ($ES^+$)), *m/z*: 713.25 $[M+H]^+$. $^1H$ NMR (400 MHz, $CDCl_3$) δ 7.68 (s, 1H), 6.72 (s, 1H), 6.02 (t, 1H, *J* = 1.9 Hz), 4.75 (m, 1H), 4.05 (m, 2H), 3.87 (s, 3H), 3.15 (ddd, 1H, *J* = 16.2, 10.3, 2.3 Hz), 2.96 (ddd, 1H, *J* = 16.2, 4.0, 1.6 Hz), 1.28 - 1.21 (m, 3H), 1.07 (d, 18H, *J* = 7.2 Hz), 0.88 (s, 9H), 0.09 (s, 3H), 0.08 (s, 3H).

*(b) (S,E)-(2-((tert-butyldimethylsilyloxy)methyl)-4-(prop-1-enyl)-2,3-dihydro-1H-pyrrol-1-yl)(5-methoxy-2-nitro-4-(triisopropylsilyloxy)phenyl)methanone (**67**)*

Tetrakis(triphenylphosphine)palladium(0) (0.41 g, 0.35 mmol, 0.03 eq) was added to a mixture of the triflate **47** (8.4 g, 11.8 mmol, 1.0 eq), E-1-propene-1-ylboronic acid (1.42 g, 16.5 mmol, 1.4 eq) and potassium phosphate (5.0 g, 23.6 mmol, 2.0 eq) in dry dioxane (60 mL) under a nitrogen atmosphere. The mixture was stirred at 25°C for 120 mins when LC/MS indicated complete reaction. Ethyl acetate (120 mL) and water (120 mL) were added, the organic phase was removed, washed with brine (20 mL), dried over $MgSO_4$, filtered and concentrated under reduced pressure. The residue was purified by flash chromatography (silica gel, 95/5 v/v n-hexane/EtOAc to 90/10 v/v n-hexane/EtOAc) to afford the product as a yellow foam (4.96 g, 70 %). LC/MS (4.477 min ($ES^+$)), *m/z*: 605.0 $[M+H]^+$. $^1H$ NMR (400 MHz, $CDCl_3$) δ 7.67 (s, 1H), 6.74 (s, 1H), 5.93 (d, 1H, *J* = 15.4 Hz), 5.67 (s, 1H), 4.65 (m, 1H), 4.04 (m, 2H), 3.86 (s, 3H), 2.85 (m, 1H), 2.71 (m, 1H), 1.72 (dd, 3H, *J* = 6.8, 1.0 Hz), 1.30 - 1.22 (m, 3H), 1.07 (d, 18H, *J* = 7.2 Hz), 0.87 (s, 9H), 0.08 (s, 3H), 0.07 (s, 3H).

*(c) (S,E)-(2-amino-5-methoxy-4-(triisopropylsilyloxy)phenyl)(2-((tert-butyldimethylsilyloxy)methyl)-4-(prop-1-enyl)-2,3-dihydro-1H-pyrrol-1-yl)methanone (**68**)*

Zinc dust (22.0 g, 0.33 mol, 37 eq) was added, in portions over 20 mins, to a solution of the propenyl intermediate **67** (5.5 g, 9.1 mmol, 1.0 eq) in 5% v/v formic acid / ethanol (55 mL), using an ice bath to maintain the temperature between 25-30°C. After 30 mins, the reaction mixture was filtered through a short bed of celite®. The celite® was washed with ethyl acetate (65 mL) and the combined organics were washed successively with water (35 mL), saturated sodium bicarbonate (35 mL) and brine (10 mL). The organic phase was dried over $MgSO_4$, filtered and concentrated under reduced pressure. The residue was purified by flash chromatography (silica gel, 90/10 v/v n-hexane/EtOAc) to afford the product as a pale yellow oil (3.6 g, 69.0 %). LC/MS (4.439 min ($ES^+$)), *m/z*: 575.2 $[M+H]^+$. $^1H$ NMR (400 MHz, $CDCl_3$) $\delta$ 6.75 (m, 1H), 6.40 (br s, 1H), 6.28 (m, 1H), 6.11 (d, 1H, *J* = 15.4 Hz), 5.53 (m, 1H), 4.67 (m, 1H), 4.36 (m, 2H), 3.93 (br s, 1H), 3.84 (br s, 1H), 3.73 (s, 3H), 2.86 (dd, 1H, *J* = 15.7, 10.4 Hz), 2.73 (dd, 1H, *J* = 15.9, 4.5 Hz), 1.80 (dd, 3H, *J* = 6.8, 1.3 Hz), 1.35 - 1.23 (m, 3H), 1.12 (d, 18H, *J* = 7.3 Hz), 0.89 (s, 9H), 0.08 (s, 3H), 0.07 (s, 3H).

*(d) (S,E)-allyl 2-(2-((tert-butyldimethylsilyloxy)methyl)-4-(prop-1-enyl)-2,3-dihydro-1H-pyrrole-1-carbonyl)-4-methoxy-5-(triisopropylsilyloxy)phenylcarbamate (**69**)*

Allyl chloroformate (0.83 g, 6.88 mmol, 1.1 eq) was added to a solution of the amine **68** (3.6 g, 6.26 mmol, 1.0 eq) in dry DCM (80 mL) containing dry pyridine (1.09 g, 13.77 mmol, 2.2 eq) at -78°C. The dry ice was removed and the reaction mixture allowed to warm to room temperature. After stirring for a further 15 minutes, LC/MS indicated complete reaction. The organic phase was washed successively with 0.01 N HCl (50 mL), saturated sodium bicarbonate (50 mL), brine (10 mL), dried over $MgSO_4$, filtered and concentrated under reduced pressure to leave a pale yellow oil which was used in the next step without further purification (4.12g, assumed 100% yield). LC/MS (4.862 min ($ES^+$)), *m/z*: 659.2 $[M+H]^+$.

*(e)(S,E)-allyl 2-(2-(hydroxymethyl)-4-(prop-1-enyl)-2,3-dihydro-1H-pyrrole-1-carbonyl)-4-methoxy-5-(triisopropylsilyloxy)phenylcarbamate (**70**)*

The crude intermediate **69** (assumed 100% yield, 4.12 g, 6.25 mmol, 1.0 eq) was dissolved in a mixture of acetic acid (70 mL), methanol (10 mL), THF (10 mL) and water (20 mL) and allowed to stir at room temperature. After 6 hours the reaction mixture was diluted with ethyl acetate (500 mL) and washed successively with water (2 x 500 mL), saturated sodium bicarbonate (300 mL) and brine (50 mL). The organic phase was dried over $MgSO_4$, filtered and concentrated under reduced pressure. The residue was purified by flash chromatography (silica gel, 1/99 v/v methanol/DCM to 5/95 v/v methanol/DCM) to afford the product as a yellow oil and a further 1 g of unreacted starting material was recovered. This material was subjected to the same reaction conditions as above, but was left stirring for 16 h. After work up and purification, additional product was isolated (2.7 g, 79%, 2 steps) LC/MS (3.742 min ($ES^+$)), *m/z:* 545.2 $[M+H]^+$. $^1H$ NMR (400 MHz, $CDCl_3$) $\delta$ 8.38 (m, 1H), 7.72 (m, 1H), 6.81 (s, 1H), 6.37 (m, 1H), 6.10 (d, 1H, *J* = 15.8 Hz), 5.97 (m, 1H), 5.53 (m, 1H), 5.36 (ddd, 1H, *J* = 17.2, 3.1, 1.5 Hz), 5.25 (ddd, 1H, *J* = 10.4, 2.5, 1.3 Hz), 4.78 (m, 1H), 4.65 (dt, 2H, *J* = 5.7, 1.3 Hz), 3.84 (m, 3H), 3.79 (s, 3H), 3.04 (dd, 1H, *J* = 16.7, 10.5 Hz), 2.40 (dd, 1H, *J* = 16.0, 4.5 Hz), 1.82 (dd, 3H, *J* = 6.8, 1.0 Hz), 1.36 - 1.26 (m, 3H), 1.14 (d, 18H, *J* = 7.3 Hz).

*(f) (11S,11aS)-allyl 11-hydroxy-7-methoxy-5-oxo-2-((E)-prop-1-enyl)-8-(triisopropylsilyloxy)-11,11a-dihydro-1H-benzo[e]pyrrolo[1,2-a][1,4]diazepine-10(5H)-carboxylate (**71**)*

Dry dimethyl sulfoxide (1.16 g, 14.87 mmol, 3.0 eq) was added dropwise to a solution of oxalyl chloride (0.94 g, 7.43 mmol, 1.5 eq) in DCM (25 mL) at -78°C under an atmosphere of nitrogen. Maintaining the temperature at -78°C, after 10 mins a solution of the primary alcohol **70** (2.7 g, 4.96 mmol, 1.0 eq) in DCM (20 mL) was added dropwise. After a further 15 mins, dry triethylamine (2.5g, 24.78 mmol, 5.0 eq) was added, and the reaction mixture allowed to warm to room temperature. The reaction mixture was washed successively with cold 0.1 N HCl (50 mL), saturated sodium hydrogen carbonate (50 mL) and brine (10 mL) and the organic layer was dried over $MgSO_4$, filtered and concentrated under reduced pressure to afford the product as a yellow oil which was used in the next step without further purification (2.68g, assumed 100% yield). LC/MS (3.548 min ($ES^+$)), m/z*:* 543.2 $[M+H]^+$.

*(g) (11S,11aS)-allyl 11-(tert-butyldimethylsilyloxy)-7-methoxy-5-oxo-2-((E)-prop-1-enyl)-8-(triisopropylsilyloxy)-11,11a-dihydro-1H-benzo[e]pyrrolo[1,2-a][1,4]diazepine-10(5H)-carboxylate (**72**)*

*Tert*-butyldimethylsilyltrifluoromethane sulfonate (3.93 g, 14.87 mmol, 3.0 eq) was added to a solution of the carbinolamine **71** (assumed 100% yield, 2.68 g, 4.96 mmol, 1.0 eq) and 2,6-lutidine (2.12 g, 19.83 mmol, 4.0 eq) in dry

DCM (40 mL) at 0°C under an atmosphere of nitrogen. After 10 minutes, the reaction mixture was allowed to warm to room temperature and stirred for a further 60 minutes. The organic phase was washed successively with water (10 mL), saturated sodium bicarbonate (10 mL) and brine (5 mL), dried over $MgSO_4$, filtered and concentrated under reduced pressure. The residue was purified by flash chromatography (silica gel, chloroform to 2/98 v/v Methanol/chloroform) to afford the product as a yellow oil (2.0g, 63%, 2 steps). LC/MS (4.748 min ($ES^+$)), *m/z*: 657.2 $[M+H]^+$. $^1H$ NMR (400 MHz, $CDCl_3$) $\delta$ 7.19 (s, 1H), 6.86 (m, 1H), 6.66 (s, 1H), 6.22 (d, 1H, *J* = 15.4 Hz), 5.81 (d, 1H, *J* = 8.8 Hz), 5.78 (m, 1H), 5.48 (m, 1H), 5.11 (d, 1H, *J* = 5.0 Hz), 5.08 (m, 1H), 4.58 (dd, 1H, *J* = 13.4, 5.4 Hz), 4.35 (dd, 1H, *J* = 13.2, 5.7 Hz), 3.83 (s, 3H), 3.76 (s, 1H), 3.00 (dd, 1H, *J* = 15.6, 11.0 Hz), 2.53 (m, 1H), 1.81 (dd, 3H, *J* = 6.8, 0.9 Hz), 1.30 - 1.18 (m, 3H), 1.08 (d, 9H, *J* = 2.3 Hz), 1.06 (d, 9H, *J* = 2.3 Hz), 0.86 (s, 9H), 0.25 (s, 3H), 0.18 (s, 3H).

*(h) (11S,11aS)-allyl 11-(tert-butyldimethylsilyloxy)-8-hydroxy-7-methoxy-5-oxo-2-((E)-prop-1-enyl)-11,11a-dihydro-1H-benzo[e]pyrrolo[1,2-a][1,4]diazepine-10(5H)-carboxylate (**73**)*

Lithium acetate dihydrate (0.31 g, 3.04 mmol, 1.0 eq) was added to a solution of the diazepine **72** (2.0 g, 3.04 mmol, 1.0 eq) in wet DMF (20 mL) at 25°C and stirred for 4 hours. The reaction mixture was diluted with ethyl acetate (200 mL) and washed successively with 0.1 M citric acid (50 mL, pH 3), water (50 mL) and brine (10 mL), dried over $MgSO_4$, filtered and concentrated under reduced pressure. The residue was purified by flash chromatography (silica gel, 50/50 v/v n-hexane/EtOAc to 25/75 v/v n-hexane/EtOAc) to afford the product as a pale yellow solid (0.68g, 45 %). LC/MS (3.352 min ($ES^+$)), *m/z*: 501.1 $[M+H]^+$. $^1H$ NMR (400 MHz, $CDCl_3$) $\delta$ 7.02 (s, 1H), 6.66 (m, 1H), 6.53 (s, 1H), 6.03 (d, 1H, *J* = 15.5 Hz), 5.80 (s, 1H), 5.63 (d, 1H, *J* = 8.9 Hz), 5.55 (m, 1H), 5.29 (m, 1H), 4.87 (m, 2H), 4.39 (dd, 1H, *J* = 13.5, 4.2 Hz), 4.20 (dd, 1H, *J* = 13.2, 5.7 Hz), 3.73 (s, 3H), 3.59 (m, 1H), 2.81 (dd, 1H, *J* = 16.1, 10.5 Hz), 2.35 (d, 1H, *J* = 15.7 Hz), 1.61 (d, 3H, *J* = 6.4 Hz), 0.67 (s, 9H), 0.05 (s, 3H), 0.00 (s, 3H).

*(i) (11S,11aS)-allyl 11-(tert-butyldimethylsilyloxy)-8-(3-iodopropoxy)-7-methoxy-5-oxo-2-((E)-prop-1-enyl)-11,11a-dihydro-1H-benzo[e]pyrrolo[1,2-a][1,4]diazepine-10(5H)-carboxylate (**74**)*

Diiodopropane (0.295 g, 1.00 mmol, 5.0 eq) and potassium carbonate (0.028 g, 0.20 mmol, 1.0 eq) were added to a solution of the phenol **33** (0.100 g, 0.020 mmol, 1.0 eq) in dry acetone (5 mL). The reaction mixture was heated at 60°C for 6 hours when LC/MS showed complete reaction. The reaction mixture was concentrated to dryness under reduced pressure and the residue was purified by flash chromatography (silica gel, 75/25 v/v *n*-hexane/EtOAc to 50/50 v/v n-hexane/EtOAc) to afford the product as a colourless oil (0.074 g, 56%). LC/MS (3.853 min ($ES^+$)), *m/z:* 669.0 $[M+H]^+$. $^1H$ NMR (400 MHz, $CDCl_3$) $\delta$ 7.26 (s, 1H), 6.90 (s, 1H), 6.68 (s, 1H), 6.24 (d, 1H, *J* = 15.3 Hz), 5.87 (d, 1H, *J* = 8.9 Hz), 5.78 (m, 1H), 5.53 (m, 1H), 5.12 (m, 2H), 4.65 (m, 2H), 4.41 (m, 1H), 4.11 (m, 1H), 3.93 (s, 3H), 3.81 (m, 1H), 3.40 (t, 2H, *J* = 6.7 Hz), 3.05 (dd, 1H, *J* = 16.3, 10.1 Hz), 2.57 (m, 1H), 2.34 (m, 2H), 1.84 (d, 3H, *J* = 6.6 Hz), 0.92 (s, 9H), 0.28 (s, 3H), 0.26 (s, 3H).

(iii) (11S,11aS)-4-((S)-2-((S)-2-(allyloxycarbonylamino)-3-methylbutanamido)propanamido)benzyl 11-(tert-butyldimethylsilyloxy)-8-hydroxy-7-methoxy-5-oxo-2-((E)-prop-1-enyl)-11,11a-dihydro-1H-benzo[e]pyrrolo[1,2-a][1,4]diazepine-10(5H)-carboxylate **79**)

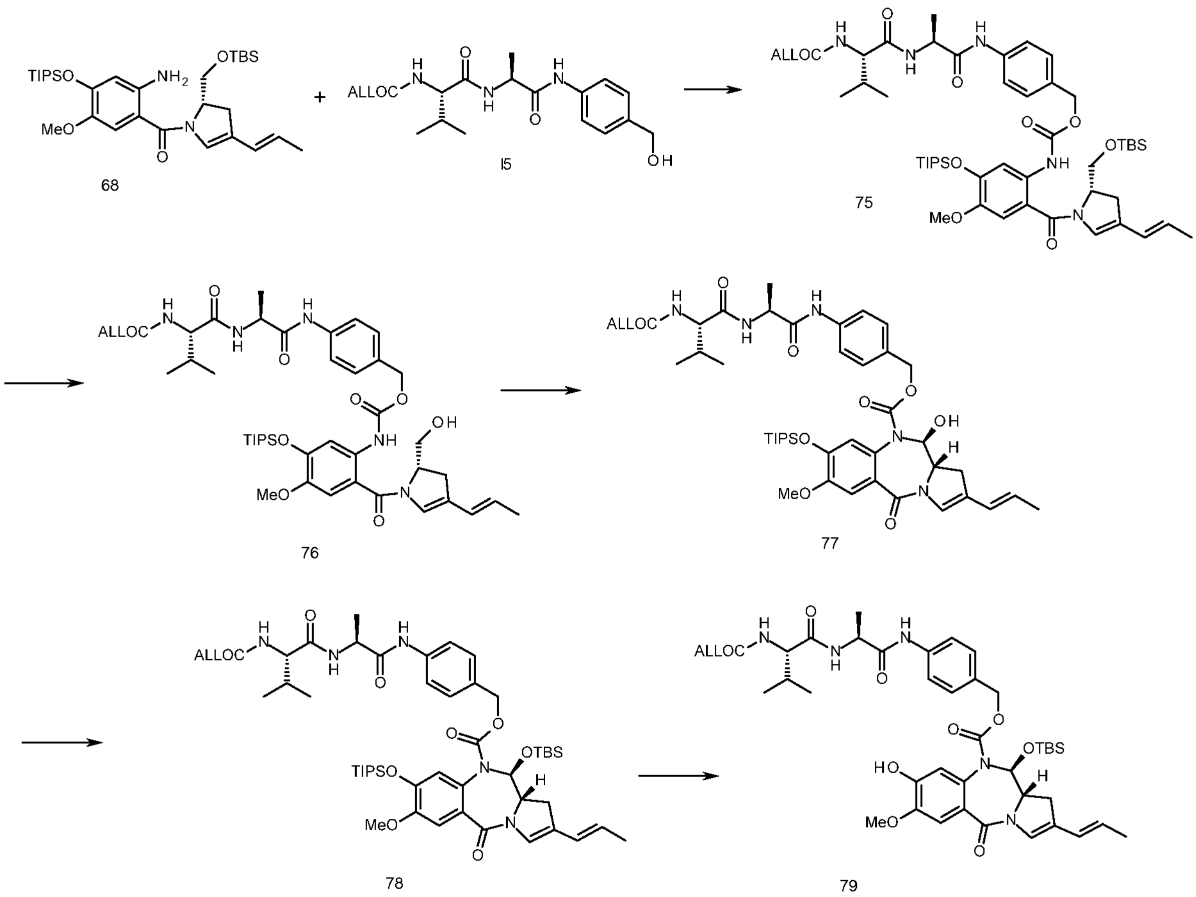

68 I5 75 76 77 78 79

*(a) Allyl ((S)-1-(((S)-1-((4-((((2-((S)-2-(((tert-butyldimethylsilyl)oxy)methyl)-4-((E)-prop-1-en-1-yl)-2,3-dihydro-1H-pyrrole-1-carbonyl)-4-methoxy-5-((triisopropylsilyl)oxy)phenyl)carbamoyl)oxy)methyl)phenyl)amino)-1-oxopropan-2-yl)amino)-3-methyl-1-oxobutan-2-yl)carbamate (**75**)*

Triethylamine (0.256 mL, 1.84 mmol, 2.2 eq) was added to a stirred solution of the amine **68** (0.480 g, 0.835 mmol, 1.0 eq) and triphosgene (0.089 g, 0.301 mmol, 0.36 eq) in dry THF (15 mL) at 5°C (ice bath). The progress of the isocyanate reaction was monitored by periodically removing aliquots from the reaction mixture and quenching with methanol and performing LCMS analysis. Once the isocyanate reaction was complete a solution of Alloc-Val-Ala-PABOH **I5** (0.473 g, 1.25 mmol, 1.5 eq) and triethylamine (0.174 mL, 1.25 mmol, 1.5 eq) in dry THF (10 mL) was rapidly added by injection to the freshly prepared isocyanate. The reaction was allowed to stir at 40°C for 4 hours followed by stirring at room temperature overnight. The mixture was concentrated under reduced pressure, and purified by flash chromatography (silica gel, 20/80 v/v *n*-hexane/EtOAc to 50/50 v/v *n*-hexane/EtOAc, then 1/99 v/v DCM/MeOH to 5/95 v/v DCM/MeOH) to afford the product as a yellow solid (0.579 g, 71%). LC/MS (4.468 min (ES$^+$)), *m/z:* 978.55 $[M+H]^+$. $^1H$ NMR (400 MHz, $CDCl_3$) δ 8.63 (br s, 1H), 8.42 (s, 1H), 7.78 (br s, 1H), 7.53 (d, 2H, *J* = 8.1 Hz), 7.31 (d, 2H, *J* = 8.6 Hz), 6.76 (s, 1H), 6.59 (d, 1H, *J* = 7.6 Hz), 6.36 (br s, 1H), 6.04 (d, 1H, *J* = 15.9 Hz), 5.90 (m, 1H), 5.55 (m, 1H), 5.33 - 5.21 (m, 3H), 5.10 (s, 2H), 4.66 (m, 2H), 4.57 (dd, 2H, *J* = 5.6, 1.0 Hz), 3.98 (dd, 1H, *J* = 7.3, 6.8 Hz), 3.90 (m, 1H), 3.81 (m, 1H), 3.78 (s, 3H), 2.82 (dd, 1H, *J* = 15.4, 9.6 Hz), 2.72 (dd, 1H, *J* = 15.9, 3.5 Hz), 2.17 (m, 1H), 1.78 (dd, 3H, *J* = 6.5, 0.8 Hz), 1.46 (d, 3H, *J* = 7.1 Hz), 1.29 (m, 3H), 1.11 (d, 18H, *J* = 7.1 Hz), 0.97 (d, 3H, *J* = 6.8 Hz), 0.92 (d, 3H, *J* = 6.8 Hz), 0.83 (s, 9H), 0.04 (s, 3H), 0.01 (s, 3H).

*(b) Allyl ((S)-1-(((S)-1-((4-((((2-((S)-2-(hydroxymethyl)-4-((E)-prop-1-en-1-yl)-2,3-dihydro-1H-pyrrole-1-carbonyl)-4-methoxy-5-((triisopropylsilyl)oxy)phenyl)carbamoyl)oxy)methyl)phenyl)amino)-1-oxopropan-2-yl)amino)-3-methyl-1-oxobutan-2-yl)carbamate (76)*

The silyl ether 75 (1.49 g, 1.52 mmol, 1.0 eq) was dissolved in a 7:1:1:2 mixture of acetic acid/ methanol/ tetrahydrofuran/ water (14:2:2:4 mL) and allowed to stir at room temperature. After 2 hours the reaction was diluted with EtOAc (100 mL), washed sequentially with water, aq. sodium bicarbonate then brine. The organic phase was then dried over $MgSO_4$, filtered and concentrated under reduced pressure. The residue was purified by flash chromatography (silica

gel, 100/0 then 99/1 to 92/8 v/v DCM/ MeOH) to afford the product as an orange solid (1.2 g, 92%). LC/MS (3.649 min ($ES^+$)), m/z: 865.44 $[M+H]^+$. $^1H$ NMR (400 MHz, $CDCl_3$) δ 8.44 (s, 1H), 8.35 (s, 1H), 7.69 (br s, 1H), 7.53 (d, 2H, *J* = 8.7 Hz), 7.32 (d, 2H, *J* = 8.3 Hz), 6.78 (s, 1H), 6.56 (m, 2H), 6.32 (br s, 1H), 6.05 (d, 1H, *J* = 14.9 Hz), 5.90 (m, 1H), 5.56 (m, 1H), 5.30 (m, 2H), 5.22 (m, 1H), 5.10 (d, 2H, *J* = 3.1 Hz), 4.73 (m, 1H), 4.64 (m, 1H), 4.57 (d, 2H, *J* = 5.8 Hz), 4.01 (m, 1H), 3.79 (m, 2H), 3.76 (s, 3H), 2.98 (dd, 1H, *J* = 16.3, 10.2 Hz), 2.38 (dd, 1H, *J* = 16.6,4.1 Hz), 2.16 (m, 1H), 1.78 (dd, 3H, *J* = 6.8, 0.9 Hz), 1.46 (d, 3H, *J* = 7.1 Hz), 1.29 (m, 3H), 1.11 (d, 18H, *J* = 7.4 Hz), 0.97 (d, 3H, *J* = 6.7 Hz), 0.92 (d, 3H, *J* = 6.8 Hz).

*(c) (11S,11aS)-4-((S)-2-((S)-2-(allyloxycarbonylamino)-3-methylbutanamido)propanamido)benzyl 11-hydroxy-7-methoxy-5-oxo-2-((E)-prop-1-enyl)-8-(triisopropylsilyloxy)-11,11a-dihydro-1H-benzo[e]pyrrolo[1,2-a][1,4]diazepine-10(5H)-carboxylate (**77**)*

Dry dimethyl sulfoxide (0.180 g, 2.3 mmol, 3.0 eq) was added dropwise to a solution of oxalyl chloride (0.147 g, 1.1 mmol, 1.5 eq) in DCM (10 mL) at -78°C under an atmosphere of nitrogen. Maintaining the temperature at -78°C, after 20 minutes, a solution of the primary alcohol **76** (0.666 g, 0.77 mmol, 1.0 eq) in DCM (10 mL) was added dropwise. After a further 15 minutes, dry triethylamine (0.390 g, 3.85 mmol, 5.0 eq) was added, and the reaction mixture allowed to warm to room temperature. The reaction mixture was washed successively with cold 0.1 N HCl (10 mL), saturated sodium hydrogen carbonate (10 mL) and brine (5 mL). The organic layer was then dried over $MgSO_4$, filtered and concentrated under reduced pressure. The residue was then purified by flash chromatography (silica gel, 50/50 v/v n-hexane/EtOAc to 25/75 v/v n-hexane/EtOAc) to afford the product as a white solid (0.356g, 54 %). LC/MS (3.487 min ($ES^+$)), *m*/*z:* 862.2 $[M+H]^+$. $^1H$ NMR (400 MHz, $CDCl_3$) δ 8.34 (br s, 1H), 7.47 (d, 2H, *J* = 7.6 Hz), 7.17 (s, 1H), 7.14 (d, 2H, *J* = 7.5 Hz), 6.86 (br s, 1H), 6.65 (br s, 1H), 6.42 (d, 1H, *J* = 7.6 Hz), 6.22 (d, 1H, *J* = 14.4 Hz), 5.80 (m, 1H), 5.40 (m, 1H), 5.53 (m, 1H), 5.32 (m, 1H), 5.21 (d, 2H, *J* = 9.6 Hz), 5.06 (d, 1H, *J* = 12.3 Hz), 4.90 (m, 1H), 4.58 (m, 3H), 3.98 (m, 1H), 3.84 (m, 1H), 3.81 (s, 3H), 3.50 (m, 1H), 3.05 (dd, 1H, *J* = 16.0, 10.3 Hz), 2.76 (m, 1H), 2.15 (m, 1H), 1.80 (dd, 3H, *J* = 6.7, 0.8 Hz), 1.44 (d, 3H, *J* = 7.1 Hz), 1.16 (m, 3H), 1.01 (d, 18H, *J* = 6.6 Hz), 0.96 (d, 3H, *J* = 6.8 Hz), 0.92 (d, 3H, *J* = 6.8 Hz).

*(d) (11S,11aS)-4-((S)-2-((S)-2-(allyloxycarbonylamino)-3-methylbutanamido)propanamido)benzyl 11-(tert-butyldimethylsilyloxy)-7-methoxy-5-oxo-2-((E)-prop-1-enyl)-8-(triisopropylsilyloxy)-11,11a-dihydro-1H-benzo[e]pyrrolo[1,2-a][1,4]diazepine-10(5H)-carboxylate (**78**)*

Tert-butyldimethylsilyltrifluoromethane sulfonate (0.46 g, 1.74mmol, 3.0 eq) was added to a solution of secondary alcohol **77** (0.5 g, 0.58 mmol, 1.0 eq) and 2,6-lutidine (0.25 g, 2.32 mmol, 4.0 eq) in dry DCM (10 mL) at 0°C under an atmosphere of nitrogen. After 10 minutes, the reaction mixture was allowed to warm to room temperature and stirred for a further 120 mins. The organic phase was then washed successively with water (10 mL), saturated sodium bicarbonate (10 mL) and brine (5 mL), dried over $MgSO_4$, filtered and concentrated under reduced pressure. The residue was purified by flash chromatography (silica gel, 50/50 v/v n-hexane/EtOAc) to afford the product as a white solid (0.320 g, 57 %). LC/MS (4.415 min ($ES^+$)), *m*/*z*: 976.52 $[M+H]^+$. $^1H$ NMR (400 MHz, $CDCl_3$) δ 8.31 (br s, 1H), 7.48 (d, 2H, *J* = 8.0 Hz), 7.21 (s, 1H), 7.14 (d, 2H, *J* = 8.3 Hz), 6.89 (s, 1H), 6.65 (s, 1H), 6.38 (d, 1H, *J* = 7.3 Hz), 6.25 (d, 1H, *J* = 14.6 Hz), 5.93 (m, 1H), 5.85 (d, 1H, *J* = 8.8 Hz), 5.50 (m, 1H), 5.34 (m, 1H), 5.24 (m, 2H), 5.15 (d, 1H, *J* = 12.5 Hz), 4.86 (d, 1H, *J* = 12.2 Hz), 4.62 (m, 3H), 4.01 (m, 1H), 3.86 (s, 3H), 3.78 (m, 1H), 3.04 (m, 1H), 2.56 (m, 1H), 2.20 (m, 1 H), 1.84 (dd, 3H, *J* = 6.6, 0.7 Hz), 1.48 (d, 3H, *J* = 6.8 Hz), 1.20 (m, 3H), 1.05 (d, 9H, *J* = 2.9 Hz), 1.03 (d, 9H, *J* = 2.9 Hz), 0.99 (d, 3H, *J* = 6.8 Hz), 0.95 (d, 3H, *J* = 6.8 Hz), 0.88 (s, 9H), 0.27 (s, 3H), 0.14 (s, 3H).

*(e) (11S,11aS)-4-((S)-2-((S)-2-(allyloxycarbonylamino)-3-methylbutanamido)propanamido)benzyl 11-(tert-butyldimethylsilyloxy)-8-hydroxy-7-methoxy-5-oxo-2-((E)-prop-1-enyl)-11,11a-dihydro-1H-benzo[e]pyrrolo[1,2-a][1,4]diazepine-10(5H)-carboxylate (**79**)*

Lithium acetate dihydrate (0.010 g, 0.10 mmol, 1.0 eq) was added to a solution of the silyl ether **78** (0.100 g, 0.10 mmol, 1.0 eq) in wet DMF (2 mL) at 25°C for 3 hours. The reaction mixture was then diluted with ethyl acetate (20 mL) and washed successively with 0.1 M citric acid (20 mL, pH 3), water (20 mL) and brine (5 mL), dried over $MgSO_4$, filtered and concentrated under reduced pressure. The residue was purified by flash chromatography (silica gel, 5/95 v/v methanol/DCM) to afford the product as a pale yellow oil (0.070 g, 83 %). LC/MS (3.362 min ($ES^+$)), *m*/*z:* 820.2 $[M+H]^+$. $^1H$ NMR (400 MHz, $CDCl_3$) δ 8.39 (s, 1H), 7.48 (d, 2H, *J* = 8.2 Hz), 7.25 (s, 1H), 7.12 (d, 2H, *J* = 8.1 Hz), 6.88 (s, 1H), 6.68 (s, 1H), 6.47 (d, 1H, *J* = 7.6 Hz), 6.24 (d, 1H, *J* = 15.2 Hz), 6.03 (s, 1H), 5.92 (m, 1H), 5.84 (d, 1H, *J* = 8.9 Hz), 5.50 (m, 1H), 5.34 (m, 1H), 5.26 (m, 2H), 5.18 (d, 1H, *J* = 12.3 Hz), 4.80 (d, 1H, *J* = 12.4 Hz), 4.66 - 4.60 (m, 3H), 4.02 (m, 1H), 3.95 (s, 3H), 3.81 (m, 1H), 3.03 (m, 1H), 2.57 (m, 1H), 2.19 (m, 1H), 1.84 (dd, 3H, *J* = 6.8, 0.8 Hz), 1.48 (d, 3H, *J* = 7.1 Hz), 1.00 (d, 3H, *J* = 6.8 Hz), 0.95 (d, 3H, *J* = 6.8 Hz), 0.87 (s, 9H), 0.26 (s, 3H), 0.12 (s, 3H).

(iv) (11S,11aS)-4-((20S,23S)-1-iodo-20-isopropyl-23-methyl-2,18,21-trioxo-6,9,12,15-tetraoxa-3,19,22-triazatetracosanamido)benzyl 11-hydroxy-7-methoxy-8-(3-((S)-7-methoxy-5-oxo-2-((E)-prop-1-enyl)-5,11a-dihydro-1H-benzo[e]pyrrolo[1,2-a][1,4]diazepin-8-yloxy)propoxy)-5-oxo-2-((E)-prop-1-enyl)-11,11a-dihydro-1H-benzo[e]pyrrolo[1,2-a][1,4]diazepine-10(5H)-carboxylate (**66, D**)

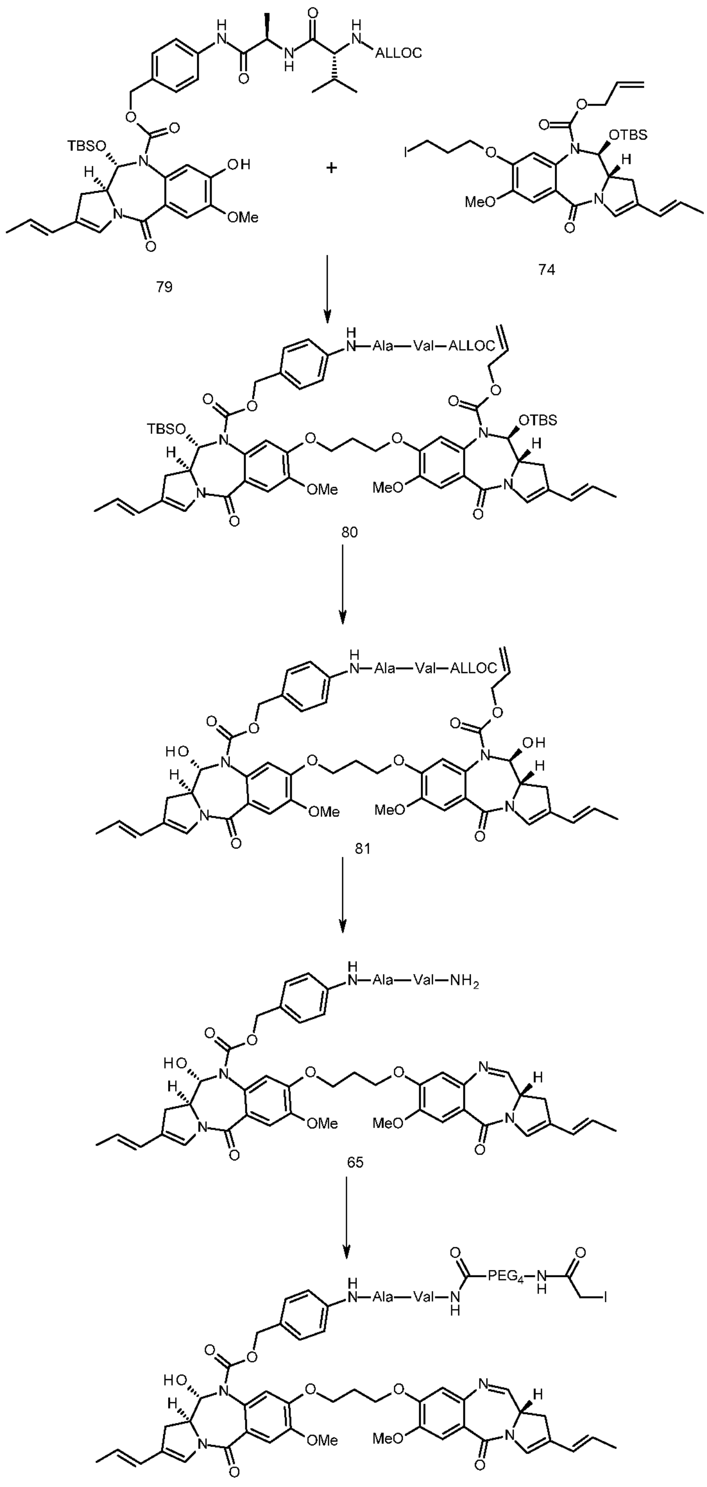

79

74

80

81

65

66

*(a) (11S,11aS)-allyl 8-(3-((11S,11aS)-10-((4-((R)-2-((R)-2-(allyloxycarbonylamino)-3-methylbutanamido)propanamido)benzyloxy)carbonyl)-11-(tert-butyldimethylsilyloxy)-7-methoxy-5-oxo-2-((E)-prop-1-enyl)-5,10,11,11a-tetrahydro-1H-benzo[e]pyrrolo[1,2-a][1,4]diazepin-8-yloxy)propoxy)-11-(tert-butyldimethylsilyloxy)-7-methoxy-5-oxo-2-((E)-prop-1-enyl)-11,11a-dihydro-1H-benzo[e]pyrrolo[1,2-a][1,4]diazepine-10(5H)-carboxylate (**80**)*

Potassium carbonate (0.030 g, 0.21 mmol, 1.0 eq) was added to a solution of the phenol **79** (0.175 g, 0.21 mmol, 1.0 eq) and the iodo linker **74** (0.214 g, 0.32 mmol, 1.5 eq) in acetone (10 mL). The reaction mixture was heated under a nitrogen atmosphere at 75°C in a sealed flask for 17 hours. The reaction mixture was concentrated to dryness under reduced pressure and purified by flash chromatography (silica gel, 2/98 v/v methanol/DCM to 5/95 v/v methanol/DCM) to afford the product as a pale yellow solid (0.100 g, 35%). LC/MS (4.293 min ($ES^+$)), *m/z:* 1359.13 $[M]^+$.

*(b) (11S,11aS)-allyl 8-(3-((11S,11aS)-10-((4-((R)-2-((R)-2-(allyloxycarbonylamino)-3-methylbutanamido)propanamido)benzyloxy)carbonyl)-11-hydroxy-7-methoxy-5-oxo-2-((E)-prop-1-enyl)-5,10,11,11a-tetrahydro-1H-benzo[e]pyrrolo[1,2-a][1,4]diazepin-8-yloxy)propoxy)-11-hydroxy-7-methoxy-5-oxo-2-((E)-prop-1-enyl)-11,11a-dihydro-1H-benzo[e]pyrrolo[1,2-a][1,4]diazepine-10(5H)-carboxylate (**81**)*

Tetra-n-butylammonium fluoride (1 M, 0.22 mL, 0.22 mmol, 2.0 eq) was added to a solution of silyl ether **80** (0.150 g, 0.11 mmol, 1.0 eq) in dry THF (2 mL). The reaction mixture was stirred at room temperature for 20 minutes, after which LC/MS indicated complete reaction. The reaction mixture was diluted with ethyl acetate (10 mL) and washed sequentially with water (5 mL) and brine (5 mL). The organic phase was dried over $MgSO_4$, filtered and concentrated under reduced pressure to leave a yellow solid. Purification by flash chromatography (silica gel, 6/94 v/v methanol/DCM to 10/90 v/v methanol/DCM) afforded the product as a pale yellow solid (0.090 g, 73%). LC/MS (2.947 min ($ES^+$)), *m/z*: 1154.0 $[M+Na]^+$. $^1H$ NMR (400 MHz, $CDCl_3$) $\delta$ 8.39 (br s, 1H), 7.39 (d, 2H, *J* = 7.6 Hz), 7.18 (d, 2H, *J* = 10.6 Hz), 7.10 (m, 3H), 6.86 (d, 2H, *J* = 10.0 Hz), 6.74 (s, 1H), 6.55 (s, 1H), 6.22 (dd, 2H, *J* = 15.3, 6.6 Hz), 5.85 (m, 2H), 5.74 (m, 3H), 5.52 (m, 2H), 5.22 (m, 1H), 5.00 (m, 2H), 4.57 (m, 6H), 4.41 (m, 2H), 4.09 (m, 4H), 3.85 (m, 11H), 3.06 (m, 2H), 2.76 (m, 2H), 2.20 (m, 2H), 2.08 (m, 1H), 1.79 (d, 6H, *J* = 6.4 Hz), 1.40 (d, 3H, *J* = 6.1 Hz), 0.90 (m, 6H).

*(c) (11S,11aS)-4-((R)-2-((R)-2-amino-3-methylbutanamido)propanamido)benzyl 11-hydroxy-7-methoxy-8-(3-((S)-7-methoxy-5-oxo-2-((E)-prop-1-enyl)-5,11a-dihydro-1H-benzo[e]pyrrolo[1,2-a][1,4]diazepin-8-yloxy)propoxy)-5-oxo-2-((E)-prop-1-enyl)-11,11a-dihydro-1H-benzo[e]pyrrolo[1,2-a][1,4]diazepine-10(5H)-carboxylate (**65**)*

Tetrakis(triphenylphospene)palladium(0) (0.005 g, 0.005 mmol, 0.06 eq) was added to a solution of the bis-carbinolamine **81** (0.090 g, 0.08 mmol, 1.0 eq) and pyrrolidine (16 $\mu$L, 0.20 mmol, 2.5 eq) in dry DCM (5 mL). After 20 minutes, the reaction mixture was diluted with DCM (10 mL) and washed sequentially with saturated ammonium chloride (5 mL) and brine (5 mL), dried over $MgSO_4$, filtered and the solvent was removed under reduced pressure to leave the crude product as a pale yellow solid which was used in the next step without further purification (0.075 g, assumed 100% yield). LC/MS (2.060 min ($ES^+$)), *m/z:* 947.2 $[M+H]^+$.

*(d) (11S,11aS)-4-((20S,23S)-1-iodo-20-isopropyl-23-methyl-2,18,21-trioxo-6,9,12,15-tetraoxa-3,19,22-triazatetracosanamido)benzyl 11-hydroxy-7-methoxy-8-(3-((S)-7-methoxy-5-oxo-2-((E)-prop-1-enyl)-5,11a-dihydro-1H-benzo[e]pyrrolo[1,2-a][1,4]diazepin-8-yloxy)propoxy)-5-oxo-2-((E)-prop-1-enyl)-11,11a-dihydro-1H-benzo[e]pyrrolo[1,2-a][1,4]diazepine-10(5H)-carboxylate (**66, D**)*

EDCI (0.015 g, 0.08 mmol, 1.0 eq) was added to a solution of amine **65** (assumed 100% yield 0.075 g, 0.08 mmol, 1.0 eq) and iodoacetamide-$PEG_4$-acid **I7** (0.034 g, 0.08 mmol, 1.0 eq) in dry dichloromethane (5 mL) and the reaction was stirred in the dark. After 50 minutes, a further amount of iodoacetamide-$PEG_4$-acid **I7** (0.007 g, 0.016 mmol, 0.2 eq) was added along with a further amount of EDCI (0.003 g, 0.016 mmol, 0.2 eq). After a total of 2.5 hours, the reaction mixture was diluted with dichloromethane (15 mL) and washed sequentially with water (10 mL) and brine (10 mL). The organic phase was dried over $MgSO_4$, filtered and concentrated under reduced pressure. The resulting residue was purified by flash chromatography (silica gel, Chloroform 100% to 90:10 v/v Chloroform:Methanol). Pure fractions were combined to afford the product (0.0254 g, 23%, 2 steps). The crude fractions were collected and purified by preparative TLC (silica gel, 90:10 v/v Chloroform:Methanol) to afford a second batch of product (0.0036 g, 3%, 2 steps). LC/MS (2.689 min ($ES^+$)), *m/z*: 681.0 1/2$[M+2H]^+$.

**Example 10: Activity of released compounds**

K562 assay

K562 human chronic myeloid leukaemia cells were maintained in RPM1 1640 medium supplemented with 10% fetal calf serum and 2 mM glutamine at 37°C in a humidified atmosphere containing 5% $CO_2$ and were incubated with a specified dose of drug for 1 hour or 96 hours at 37°C in the dark. The incubation was terminated by centrifugation (5 min, 300 g) and the cells were washed once with drug-free medium. Following the appropriate drug treatment, the cells were transferred to 96-well microtiter plates ($10^4$ cells per well, 8 wells per sample). Plates were then kept in the dark at 37°C in a humidified atmosphere containing 5% $CO_2$. The assay is based on the ability of viable cells to reduce a yellow soluble tetrazolium salt, 3-(4,5-dimethylthiazol-2-yl)-2,5-diphenyl-2*H*-tetrazolium bromide (MTT, Aldrich-Sigma), to an insoluble purple formazan precipitate. Following incubation of the plates for 4 days (to allow control cells to increase in number by approximately 10 fold), 20 μL of MTT solution (5 mg/mL in phosphate-buffered saline) was added to each well and the plates further incubated for 5 h. The plates were then centrifuged for 5 min at 300 g and the bulk of the medium pipetted from the cell pellet leaving 10-20 μL per well. DMSO (200 μL) was added to each well and the samples agitated to ensure complete mixing. The optical density was then read at a wavelength of 550 nm on a Titertek Multiscan ELISA plate reader, and a dose-response curve was constructed. For each curve, an $IC_{50}$ value was read as the dose required to reduce the final optical density to 50% of the control value.

Compound RelC has an $IC_{50}$ of less than 0.1 pM in this assay.

Compound RelE has an $IC_{50}$ of 0.425 nM in this assay.

**Example 11: Formation of conjugates**

*General antibody conjugation procedure*

Antibodies are diluted to 1-5 mg/mL in a reduction buffer (examples: phosphate buffered saline PBS, histidine buffer, sodium borate buffer,TRIS buffer). A freshly prepared solution of TCEP (tris(2-carboxyethyl)phosphine hydrochloride) is added to selectively reduce cysteine disulfide bridges. The amount of TCEP is proportional to the target level of reduction, within 1 to 4 molar equivalents per antibody, generating 2 to 8 reactive thiols. After reduction for several hours at 37°C, the mixture is cooled down to room temperature and excess drug-linker (**A, B, C, D, E**) added as a diluted DMSO solution (final DMSO content of up to 10% volume/volume of reaction mixture). The mixture was gently shaken at either 4°C or room temperature for the appropriate time, generally 1-3 hours. Excess reactive thiols can be reacted with a 'thiol capping reagent' like N-ethyl maleimide (NEM) at the end of the conjugation. Antibody-drug conjugates are concentrated using centrifugal spin-filters with a molecular weight cut-off of 10 kDa or higher, then purified by tangential flow filtration (TFF) or Fast Protein Liquid Chromatography (FPLC). Corresponding antibody-drug conjugates can be determined by analysis by High-Performance Liquid Chromatography (HPLC) or Ultra-High-Performance Liquid Chromatography (UHPLC) to assess drug-per-antibody ratio (DAR) using reverse-phase chromatography (RP) or Hydrophobic-Interaction Chromatography (HIC), coupled with UV-Visible, Fluorescence or Mass-Spectrometer detection; aggregate level and monomer purity can be analysed by HPLC or UHPLC using size-exclusion chromatography coupled with UV-Visible, Fluorescence or Mass-Spectrometer detection. Final conjugate concentration is determined by a combination of spectroscopic (absorbance at 280, 214 and 330 nm) and biochemical assay (bicinchonic acid assay BCA; Smith, P.K., et al. (1985) Anal. Biochem. 150 (1): 76-85; using a known-concentration IgG antibody as reference). Antibody-drug conjugates are generally sterile filtered using 0.2 μm filters under aseptic conditions, and stored at +4°C, -20°C or -80°C.

*DAR Determination*

Antibody or ADC (ca. 35 μg in 35 μL) was reduced by addition of 10 μL borate buffer (100 mM, pH 8.4) and 5 μL DTT (0.5 M in water), and heated at 37°C for 15 minutes. The sample was diluted with 1 volume of acetonitrile: water: formic acid (49%: 49%: 2% v/v), and injected onto a Widepore 3.6μ XB-C18 150 x 2.1 mm (P/N 00F-4482-AN) column (Phenomenex Aeris) at 80°C, in a UPLC system (Shimadzu Nexera) with a flow rate of 1 ml/min equilibrated in 75% Buffer A (Water, Trifluoroacetic acid (0.1% v/v) (TFA), 25% buffer B (Acetonitrile: water: TFA 90%: 10%: 0.1% v/v). Bound material was eluted using a gradient from 25% to 55% buffer B in 10 min. Peaks of UV absorption at 214 nm were integrated. The following peaks were identified for each ADC or antibody: native antibody light chain (L0), native antibody heavy chain (H0), and each of these chains with added drug-linkers (labelled L1 for light chain with one drug and H1, H2, H3 for heavy chain with 1, 2 or 3 attached drug-linkers). The UV chromatogram at 330 nm was used for identification of fragments containing drug-linkers (i.e., L1, H1, H2, H3).

A PBD/protein molar ratio was calculated for both light chains and heavy chains:

$$\frac{Drug}{Protein}\,ratio\ on\ light\ chain = \frac{\%Area\ at\ 214nm\ for\ L1}{\%Area\ at\ 214\ nm\ for\ L0\ and\ L1}$$

$$\frac{Drug}{Protein}\,ratio\ on\ heavy\ chain = \frac{\sum_{n=0}^{3} n \times (\%area\ at\ 214\ for\ Hn)}{\sum_{n=0}^{3} \%area\ at\ 214\ for\ Hn}$$

Final DAR is calculated as:

$$DAR = 2 \times \left(\frac{Drug}{Protein}\,ratio\ on\ light\ chain + \frac{Drug}{Protein}\,ratio\ on\ heavy\ chain\right)$$

DAR measurement is carried out at 214 nm because it minimises interference from drug-linker absorbance.

*Generation of ADCs*

Antibody A (comprising a variable domain which is SEQ ID NO. 1 paired with SEQ ID NO. 2) was conjugated with drug linker A in two experiments to give Conj A-A1 and Conj A-A2, and the DAR was measured to be 3.51 (see table below) and 2.52 respectively.

Antibody A (comprising a variable domain which is SEQ ID NO. 1 paired with SEQ ID NO. 2) was conjugated with drug linker B to give Conj A-B, and the DAR was measured to be 2.87.

Antibody A (comprising a variable domain which is SEQ ID NO. 1 paired with SEQ ID NO. 2) was conjugated with drug linker D to give Conj A-D, and the DAR was measured to be 2.19 (see table below).

Antibody A (comprising a variable domain which is SEQ ID NO. 1 paired with SEQ ID NO. 2) was conjugated with drug linker E in two experiments to give Conj A-E1 and Conj A-E2, and the DAR was measured to be 2.24 (see table below) and 2.7 respectively.

Her and Trastuzumab are anti-Her2 antibodies comprising a VH domain having the sequence according to SEQ ID NO. 1 and a VL domain having the sequence according to SEQ ID NO. 2.

As described above, ADCs targeted to Her2 were generated by conjugating Her2 antibodies to the drug linker A, E, D or B. The resulting ADCs are listed in the table below, alongside the measured DAR.

| **ADC** | **DAR** | **Concentration [mg/ml]** | **Yield [%]** |
|---|---|---|---|
| Her2-A | 3.51 | 1.05 | 73 |
| Her2-E | 2.24 | 1.02 | 71 |
| Her2-D | 2.19 | 0.67 | 56 |
| Her2-B | 1.58 | 2.2 | 37 |

**EXAMPLE 12: in vitro cytotoxicity of ADCs**

*Cell Culture*

BT-474 and MDA-MB-468 cells were from the American Type Culture Collection. Cell culture medium was RPMI 1640 supplemented with L-Glutamine and 10% FBS. Cells were grown at 37°C, 5% $CO_2$, in a humidified incubator.

*Cytotoxicity assay*

The concentration and viability of cultures of suspended cells (at up to $1 \times 10^6$/ml) were determined by mixing 1:1 with Trypan blue and counting clear (live)/blue (dead) cells with a haemocytometer. The cell suspension was diluted to the required seeding density (generally $10^5$/ml) and dispensed into 96-well flat bottomed plates. For Alamar blue assay, 100 μl/well was dispensed in black-well plates. For MTS assay, 50 μl/well was dispensed in clear-well plates. A stock solution (1 ml) of ADC (20 μg/ml) was made by dilution of filter-sterile ADC into cell culture medium. A set of 8 x 10-fold dilutions of stock ADC were made in a 24 well plate by serial transfer of 100 μl onto 900 μl of cell culture medium. Each ADC dilution (100 μl/well for Alamar blue, 50 μl/well for MTS) was dispensed into 4 replicate wells of the 96-well

plate, containing cell suspension. Control wells received the same volume of culture medium only. After incubation for 4 days, cell viability was measured by either Alamar blue or MTS assay.

AlamarBlue® (Invitrogen, catalogue number DAL1025) was dispensed (20 μl per well) into each well and incubated for 4 hours at 37°C in the $CO_2$-gassed incubator. Well fluorescence was measured at excitation 570 nm, emission 585 nm. Cell survival (%) was calculated from the ratio of mean fluorescence in the 4 ADC-treated wells compared to the mean fluorescence in the 4 control wells (100%).

MTS (Promega, catalogue number G5421) was dispensed (20 μl per well) into each well and incubated for 4 hours at 37°C in the $CO_2$-gassed incubator. Absorbance was measured at 490 nm. Cell survival (%) was calculated from the mean absorbance in the 4 ADC-treated wells compared to the mean absorbance in the 4 control wells (100%). Dose response curves were generated from the mean data of 3 replicate experiments and the $EC_{50}$ was determined by fitting data to a sigmoidal dose-response curve with variable slope using Prism (GraphPad, San Diego, CA).

*In vitro cytotoxicity*

The efficacy of the above Her ADCs was tested against Her2(+) BT-474 cells. As a Her2-negative control, MDA-MB-468 cells were used.

Figure 1 shows *in vitro* efficacy of Her-A. Serial 10-fold dilutions (μg/mL) of Her-A were incubated with BT-474 cells or MDA-MB-468SU-DHL-1 cells. The Alamar Blue assay was performed at the end of the incubation period and cell survival calculated. Graphs represent the average of three replicate experiments.

Her-A showed significant cytotoxicity against BT-474 cells (Figure 1).

| Her-A | BT474 | MDAMB468 |
|---|---|---|
| EC50 (μg/mL) | 0.02683 | 1.314 |

Figure 2 shows *in vitro* efficacy of Her-E. Serial 10-fold dilutions (μg/mL) of Her-E were incubated with BT-474 cells or MDA-MB-468SU-DHL-1 cells. The Alamar Blue assay was performed at the end of the incubation period and cell survival calculated. Graphs represent the average of three replicate experiments.

Her-E showed significant cytotoxicity against BT-474 cells (Figure 2).

| Her-E | BT474 | MDAMB468 |
|---|---|---|
| EC50 (μg/mL) | 0.06933 | 0.9941 |

Figure 3 shows *in vitro* efficacy of Her-D. Serial 10-fold dilutions (μg/mL) of Her-D were incubated with BT-474 cells or MDA-MB-468SU-DHL-1 cells. The Alamar Blue assay was performed at the end of the incubation period and cell survival calculated. Graphs represent the average of three replicate experiments.

Her-D showed cytotoxicity against BT-474 cells (Figure 3).

| Her-D | BT474 | MDAMB468 |
|---|---|---|
| EC50 (μg/mL) | 0.4302 | 0.6109 |

Figure 4 shows *in vitro* efficacy of Her-B. Serial 10-fold dilutions (μg/mL) of Her-B were incubated with BT-474 cells or MDA-MB-468SU-DHL-1 cells. The Alamar Blue assay was performed at the end of the incubation period and cell survival calculated. Graphs represent the average of three replicate experiments.

Her-B showed cytotoxicity against BT-474 cells (Figure 4).

| Her-B | BT474 | MDAMB468 |
|---|---|---|
| EC50 (μg/mL) | 0.2234 | 1.065 |

**EXAMPLE 13 - in vivo anti-tumour activity of ADCs**

The Her2 +(ve) breast-cancer-derived cell line BT-474 was used to determine the most potent PBD drug linker for Her2 in vivo. The antibody was conjugated to A, E, D, or B warhead-linkers and tested in the BT-474 xenograft model. At the same time, to investigate any potential anti-tumor activity observed with Trastuzumab alone, the antibody without

PBD was tested in the same assay.

*Study design*

Drugs and treatment:

| Group No | Animals per group | ADC | Dose level (mg/kg) | Dose volume (ml/kg) |
|---|---|---|---|---|
| 1 | 10 | [vehicle only] | - | - |
| 2 | 10 | Trastuzumab [no PBD] | 1.0 | 10 |
| 3 | 10 | Her-A | 0.3 | 10 |
| 4 | 10 | Her-A | 1.0 | 10 |
| 5 | 10 | Her-E | 0.3 | 10 |
| 6 | 10 | Her-E | 1.0 | 10 |
| 7 | 10 | Her-D | 0.3 | 10 |
| 8 | 10 | Her-D | 1.0 | 10 |
| 9 | 10 | Her-B | 1.0 | 10 |
| 10 | 10 | Her-B | 1.0 | 10 |

Procedures:

- Set up CR female NCr nu/nu mice with I xl BT474-SPN tumor cells in 0% Matrigel sc in flank.
- Cell Injection Volume is 0.1 mL/mouse.
- Age at Start Date: 8 to 12 weeks.
- Perform a pair match when tumors reach an average size of 100 - 150 $mm^3$ and begin treatment.
- Body Weight: qd x 5 then bi-wk to end
- Caliper Measurement: bi-wk to end
- Report any adverse reactions or death immediately.
- Any individual animal with a single observation of >30% body weight loss or three consecutive measurements of >25% body weight loss will be euthanized.
- Any group with two measurements of mean body weight loss of >20% or > 10% mortality will stop dosing. The group is not euthanized and recovery is allowed. Within a group with >20% weight loss, individuals hitting the individual body weight loss endpoint will be euthanized. If the group treatment related body weight loss is recovered to within 10% of the original weights, dosing may resume at a lower dose or less frequent dosing schedule. Exceptions to non-treatment body weight% recovery may be allowed on a case-by-case basis.
- Endpoint TGD. Animals are to be monitored individually. The endpoint of the experiment is a tumor volume of 2000$mm^3$ or 60 days, whichever comes first. Responders can be followed longer. When the endpoint is reached, the animals are to be euthanized.

*General methodological approach*

For the calculation of group mean tumor volumes the following rule was applied: when an animal exited the study due to tumor size, the final tumor volume recorded for the animal was included with the data used to calculate the mean volume at subsequent time points. Error bars indicate standard error of the mean (SEM). Tumor volumes values were not used to calculate group mean tumor volumes when fewer than 50% of the animals in a group remained in the study. Prism (GraphPad, San Diego, CA) was used for graphical presentations and statistical analyses.

*Results*

Figure 5 shows drug linker selection for Her-ADCs in BT-474 xenograft model. Mice were dosed when the mean tumor volume per experimental group reached 0.1 $cm^3$ and they were treated with a single dose of the ADCs at 0.3 and 1 mg/kg (for binding ADCs) via IV in the tail vein. Data represent the mean tumour volume (+/- SEM) from ten mice in each group.

Her-A and Her-B exhibited the most potent anti-tumor activity (Figure 5); Her-E and Her-D showed strong, but less marked, anti-tumour activity. Trastuzumab without PBD warhead did not show significant anti-tumor activity in this assay.

**Abbreviations**

| | |
|---|---|
| Ac | acetyl |
| Acm | acetamidomethyl |
| Alloc | allyloxycarbonyl |
| Boc | di-*tert*-butyl dicarbonate |
| t-Bu | tert-butyl |
| Bzl | benzyl, where Bzl-OMe is methoxybenzyl and Bzl-Me is methylbenzene |
| Cbz or Z | benzyloxy-carbonyl, where Z-Cl and Z-Br are chloro- and bromobenzyloxy carbonyl respectively |
| DMF | *N*,*N*-dimethylformamide |
| Dnp | dinitrophenyl |
| DTT | dithiothreitol |
| Fmoc | 9*H*-fluoren-9-ylmethoxycarbonyl |
| imp | N-10 imine protecting group: 3-(2-methoxyethoxy)propanoate-Val-Ala-PAB |
| MC-OSu | maleimidocaproyl-O-*N*-succinimide |
| Moc | methoxycarbonyl |
| MP | maleimidopropanamide |
| Mtr | 4-methoxy-2,3,6-trimethtylbenzenesulfonyl |
| PAB | para-aminobenzyloxycarbonyl |
| PEG | ethyleneoxy |
| PNZ | p-nitrobenzyl carbamate |
| Psec | 2-(phenylsulfonyl)ethoxycarbonyl |
| TBDMS | tert-butyldimethylsilyl |
| TBDPS | tert-butyldiphenylsilyl |
| Teoc | 2-(trimethylsilyl)ethoxycarbonyl |
| Tos | tosyl |
| Troc | 2,2,2-trichlorethoxycarbonyl chloride |
| Trt | trityl |
| Xan | xanthyl |

## Claims

**1.** A conjugate of formula ConjA:

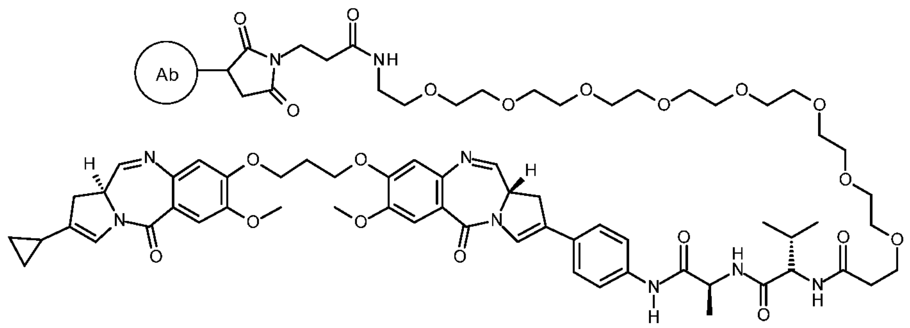

ConjA

ConjB:

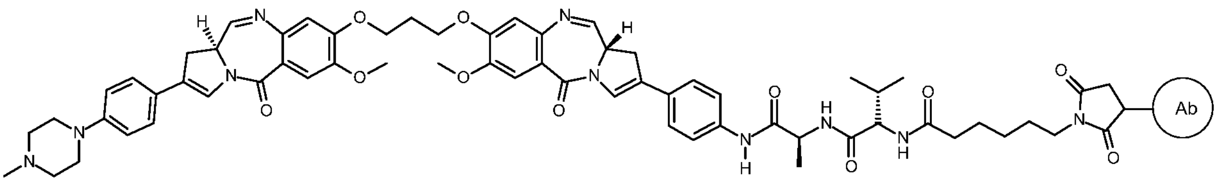

ConjB

ConjC:

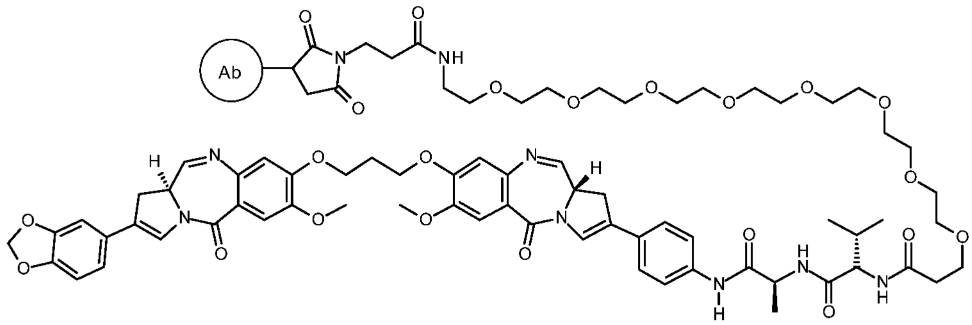

ConjC

ConjD:

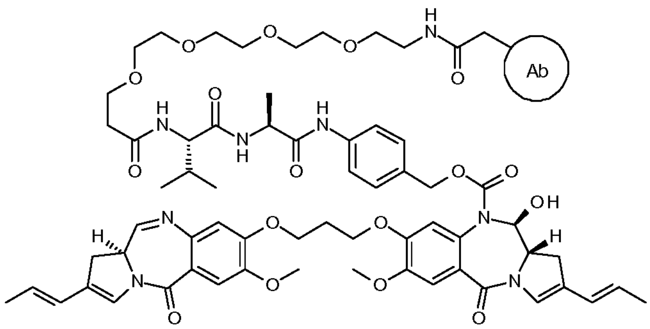

ConjD

Or ConjE:

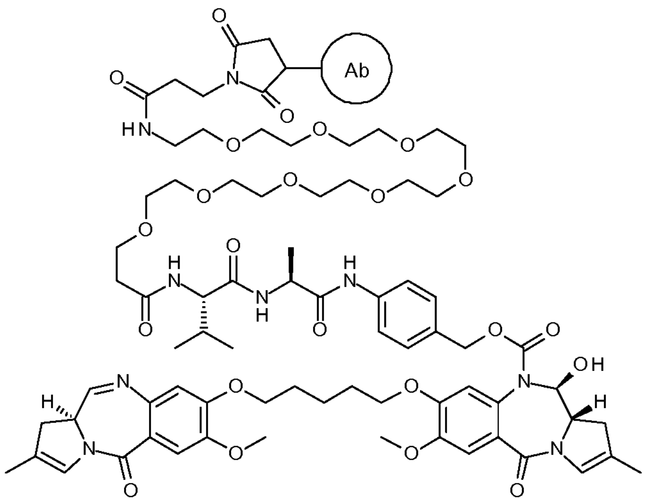

ConjE

Where Ab is an antibody that binds to HER2, the antibody comprising a VH domain having the sequence according to SEQ ID NO. 1; and
wherein the drug loading (p) of drugs (D) to antibody (Ab) is an integer from 1 to about 8.

**2.** The conjugate according to claim 1 wherein the antibody comprises a VH domain paired with a VL domain, the VH and VL domains having sequences of SEQ ID NO. 1 paired with SEQ ID NO. 2.

**3.** The conjugate according to either claim 1 or claim 2 wherein the antibody in an intact antibody.

**4.** The conjugate according to claim 3 wherein the antibody comprises a heavy chain having the sequence of SEQ ID NO. 3 paired with a light chain having the sequence of SEQ ID NO.4.

**5.** The conjugate according to claim 4 wherein the antibody comprises two heavy chains having the sequence of SEQ ID NO. 3, each paired with a light chain having the sequence of SEQ ID NO. 4.

**6.** The conjugate according to any one of claims 1 to 5 wherein the antibody is humanised, deimmunised or resurfaced.

**7.** The conjugate according to claim 1, wherein p is 1, 2, 3, or 4.

**8.** The conjugate according to claim 1 comprising a mixture of the antibody-drug conjugate compounds, wherein the average drug loading per antibody in the mixture of antibody-drug conjugate compounds is about 2 to about 5.

**9.** The conjugate according to any one of claims 1 to 8, for use in therapy.

**10.** The conjugate according to any one of claims 1 to 8, for use in the treatment of a proliferative disease in a subject.

**11.** The conjugate according to claim 10, wherein the disease is cancer.

**12.** The conjugate according to claim 11, wherein the cancer is selected from the group consisting of: breast cancer, gastric cancer, bladder cancer, pancreatic cancer, colorectal cancer, cervical cancer, ovarian cancer, prostate cancer, non-small cell lung cancer, endometrial cancer, head and neck cancer and salivary gland carcinoma.

**13.** A pharmaceutical composition comprising the conjugate of any one of claims 1 to 8 and a pharmaceutically acceptable diluent, carrier or excipient.

**14.** The pharmaceutical composition of claim 13 further comprising a therapeutically effective amount of a chemotherapeutic agent.

**Patentansprüche**

1. Konjugat der Formel ConjA:

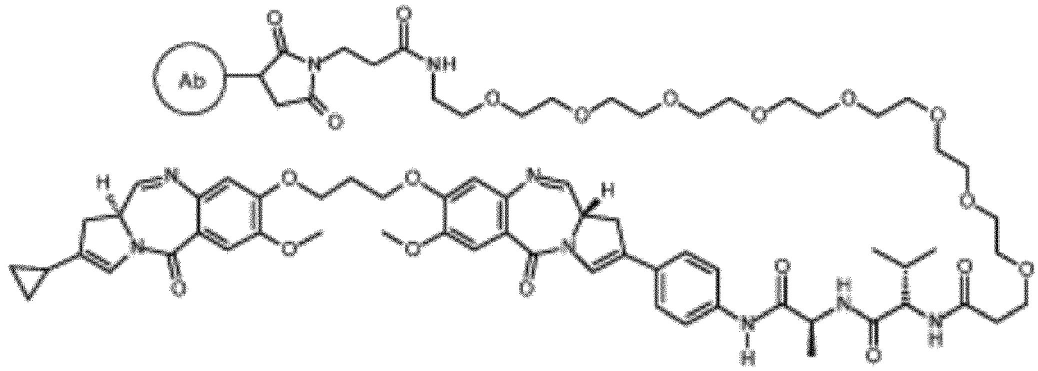

ConjA

ConjB:

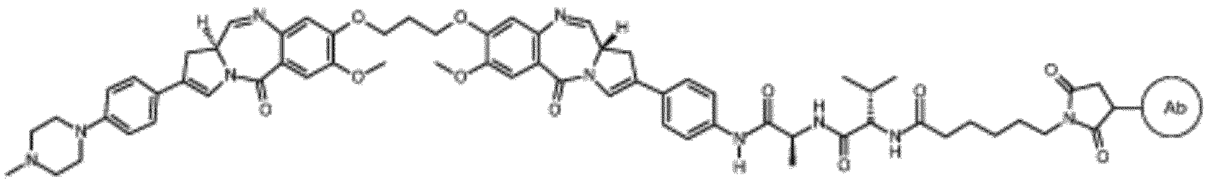

ConjB

ConjC:

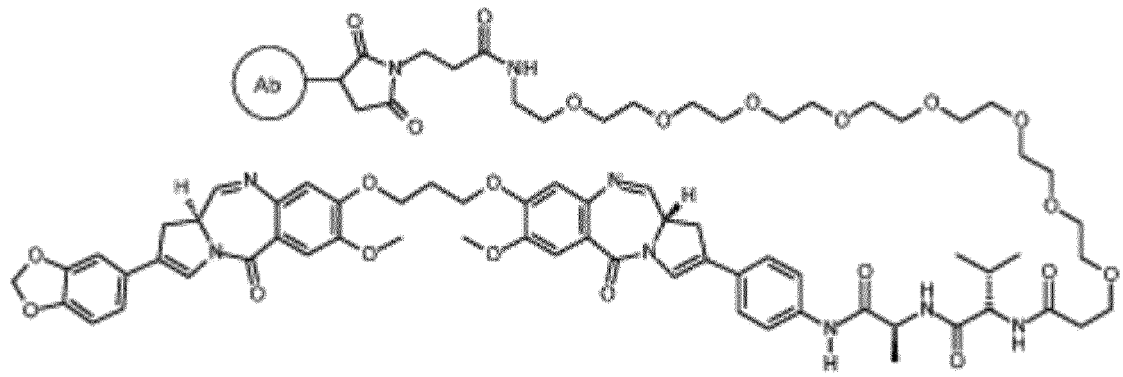

ConjC

ConjD:

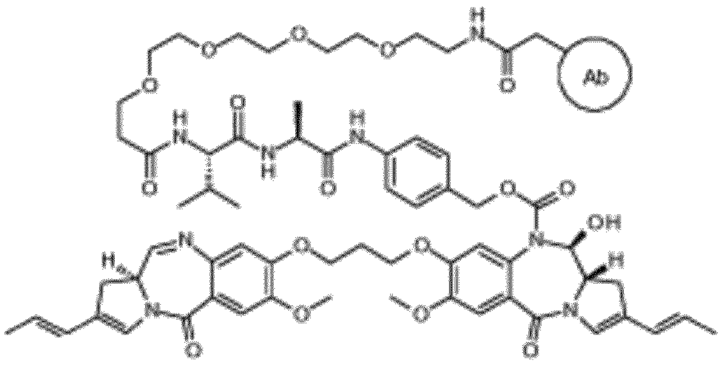

ConjD

oder ConjE:

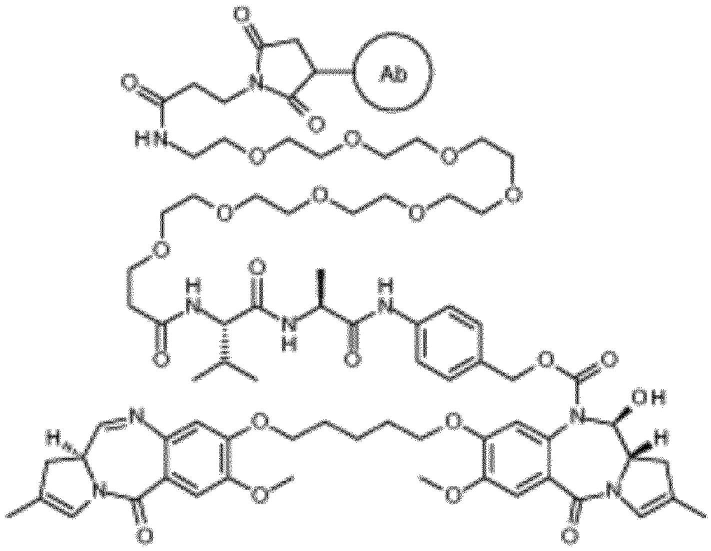

ConjE

worin Ab ein Antikörper ist, der an HER2 bindet, wobei der Antikörper eine VH-Domäne mit einer Sequenz gemäß Seq.-ID Nr. 1 umfasst; und
worin die Arzneimittelbeladung (p) von Arzneimitteln (D) zu Antikörper (Ab) eine ganze Zahl von 1 bis etwa 8 ist.

2. Konjugat nach Anspruch 1, worin der Antikörper eine VH-Domäne umfasst, die mit einer VL-Domäne gepaart ist, wobei die VH- und VL-Domänen Sequenzen aus Seq.-ID Nr. 1, gepaart mit Seq.-ID Nr. 2 aufweisen.

3. Konjugat nach Anspruch 1 oder Anspruch 2, wobei der Antikörper ein intakter Antikörper ist.

4. Konjugat nach Anspruch 3, wobei der Antikörper eine Schwerkette mit der Sequenz von Seq.-ID Nr. 3, gepaart mit einer Leichtkette mit der Sequenz von Seq.-ID Nr. 4 umfasst.

5. Konjugat nach Anspruch 4, wobei der Antikörper zwei Schwerketten mit der Sequenz von Seq.-ID Nr. 3, jeweils gepaart mit einer Leichtkette mit der Sequenz von Seq.-ID Nr. 4 umfasst.

6. Konjugat nach einem der Ansprüche 1 bis 5, wobei der Antikörper humanisiert, deimmunisiert oder oberflächenumgeformt wurde.

7. Konjugat nach Anspruch 1, worin p = 1, 2, 3 oder 4 ist.

**8.** Konjugat nach Anspruch 1 umfassend ein Gemisch der Antikörper-Arzneimit-tel-Konjugat-Verbindungen, wobei die durchschnittliche Arzneimittelbeladung pro Antikörper in dem Gemisch aus Antikörper-Arzneimittel-Konjugat-Verbindungen etwa 2 bis etwa 5 beträgt.

**9.** Konjugat nach einem der Ansprüche 1 bis 8 zur Verwendung in einer Therapie.

**10.** Konjugat nach einem der Ansprüche 1 bis 8 zur Verwendung bei der Behandlung einer Proliferationserkrankung in einem Individuum.

**11.** Konjugat nach Anspruch 10, wobei die Erkrankung Krebs ist.

**12.** Konjugat nach Anspruch 11, wobei der Krebs aus der Gruppe bestehend aus: Brustkrebs, Magenkrebs, Blasenkrebs, Pankreaskrebs, Kolorektalkrebs, Gebärmutterhalskrebs, Eierstockkrebs, Prostatakrebs, nichtkleinzelligem Lungenkrebs, Krebs des Endometriums, Kopf- und Nackenkrebs und Speicheldrüsenkarzinom ausgewählt ist.

**13.** Pharmazeutische Zusammensetzung, umfassend ein Konjugat nach einem der Ansprüche 1 bis 8 und einen pharmazeutisch annehmbaren Verdünner, Träger oder Exzipienten.

**14.** Pharmazeutische Zusammensetzung nach Anspruch 13, weiters umfassend eine therapeutisch wirksame Menge eines Chemotherapeutikums.

## Revendications

**1.** Conjugué de formule ConjA :

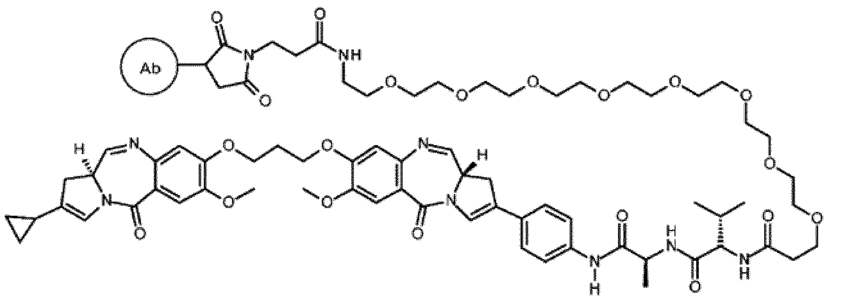

ConjA

ConjB:

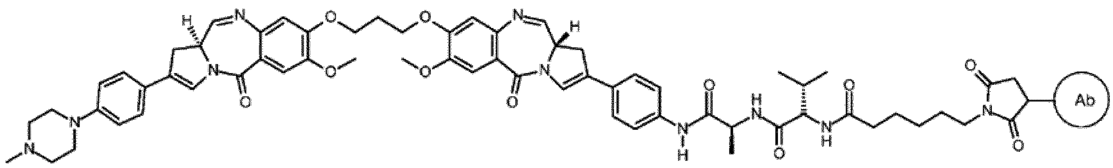

ConjB

ConjC:

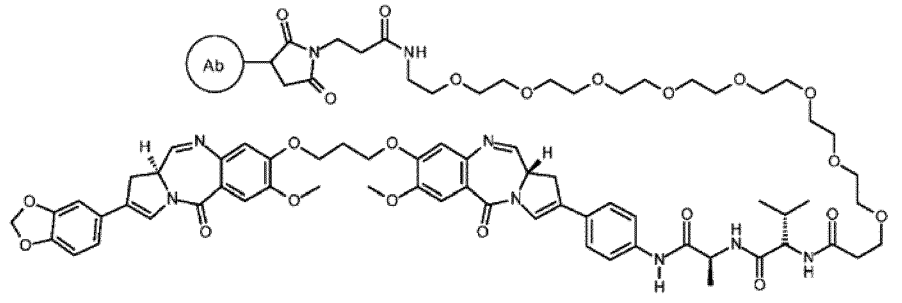

ConjC

ConjD:

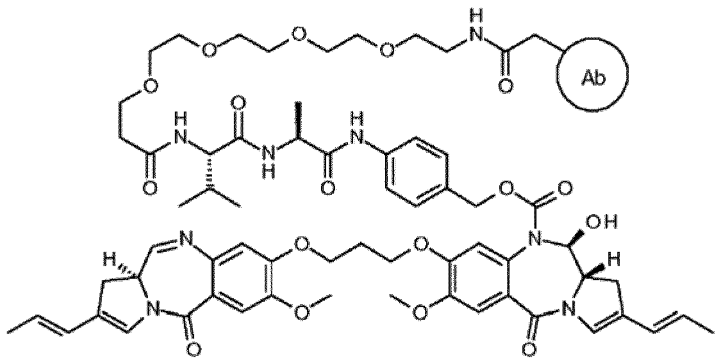

ConjD

ou ConjE :

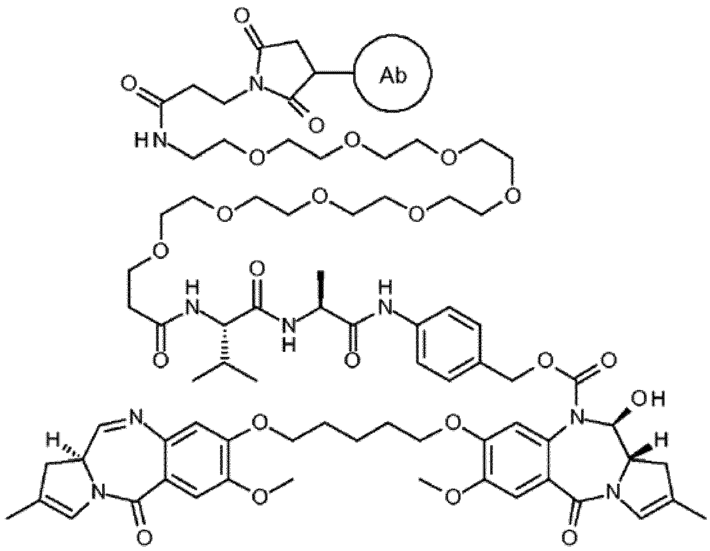

ConjE

dans lequel Ab est un anticorps qui se lie à HER2, l'anticorps comprenant un domaine VH possédant la séquence selon SEQ ID NO: 1 ; et

dans lequel la charge médicamenteuse (p) des médicaments (D) par rapport à l'anticorps (Ab) est un nombre entier compris entre 1 et environ 8.

**2.** Conjugué selon la revendication 1, dans lequel l'anticorps comprend un domaine VH apparié avec un domaine VL, les domaines VH et VL possédant des séquences de SEQ ID NO: 1 appariées avec SEQ ID NO: 2.

3. Conjugué selon l'une quelconque de la revendication 1 ou la revendication 2, dans lequel l'anticorps est un anticorps intact.

4. Conjugué selon la revendication 3, dans lequel l'anticorps comprend une chaîne lourde possédant la séquence de SEQ ID NO: 3 appariée avec une chaîne légère possédant la séquence de SEQ ID NO: 4.

5. Conjugué selon la revendication 4, dans lequel l'anticorps comprend deux chaînes lourdes possédant la séquence de SEQ ID NO: 3, chacune appariée avec une chaîne légère possédant la séquence de SEQ ID NO: 4.

6. Conjugué selon l'une quelconque des revendications 1 à 5, dans lequel l'anticorps est humanisé, désimmunisé ou a subi un resurfaçage.

7. Conjugué selon la revendication 1, dans lequel p est 1, 2, 3, ou 4.

8. Conjugué selon la revendication 1, comprenant un mélange des composés de conjugué anticorps-médicament, où la charge médicamenteuse moyenne par anticorps dans le mélange de composés de conjugué anticorps-médicament est d'environ 2 à environ 5.

9. Conjugué selon l'une quelconque des revendications 1 à 8, destiné à être utilisé en thérapie.

10. Conjugué selon l'une quelconque des revendications 1 à 8, destiné à être utilisé dans le traitement d'une maladie proliférative chez un sujet.

11. Conjugué selon la revendication 10, où la maladie est le cancer.

12. Conjugué selon la revendication 11, où le cancer est sélectionné dans le groupe consistant en : le cancer du sein, le cancer de l'estomac, le cancer de la vessie, le cancer du pancréas, le cancer colorectal, le cancer du col de l'utérus, le cancer de l'ovaire, le cancer de la prostate, le cancer du poumon non à petites cellules, le cancer de l'endomètre, le cancer de la tête et du cou et un carcinome des glandes salivaires.

13. Composition pharmaceutique comprenant le conjugué selon l'une quelconque des revendications 1 à 8 et un diluant, véhicule ou excipient pharmaceutiquement acceptable.

14. Composition pharmaceutique selon la revendication 13 comprenant en outre une quantité thérapeutiquement efficace d'un agent de chimiothérapie.

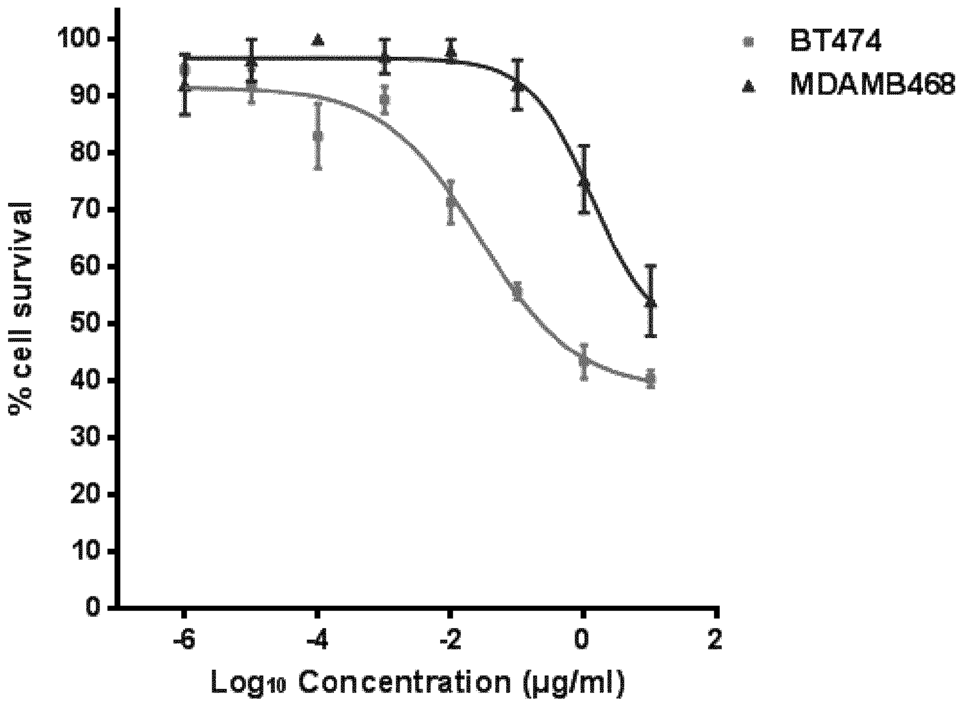


Figure 1

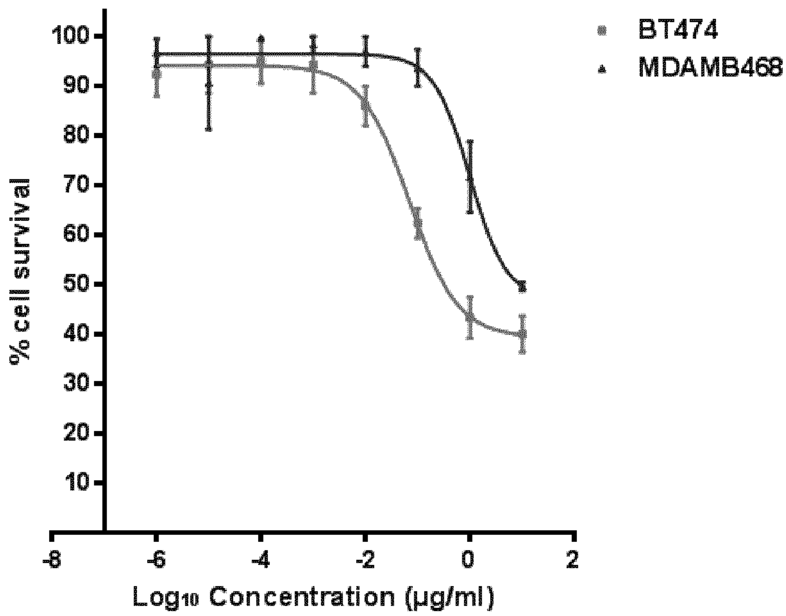


Figure 2

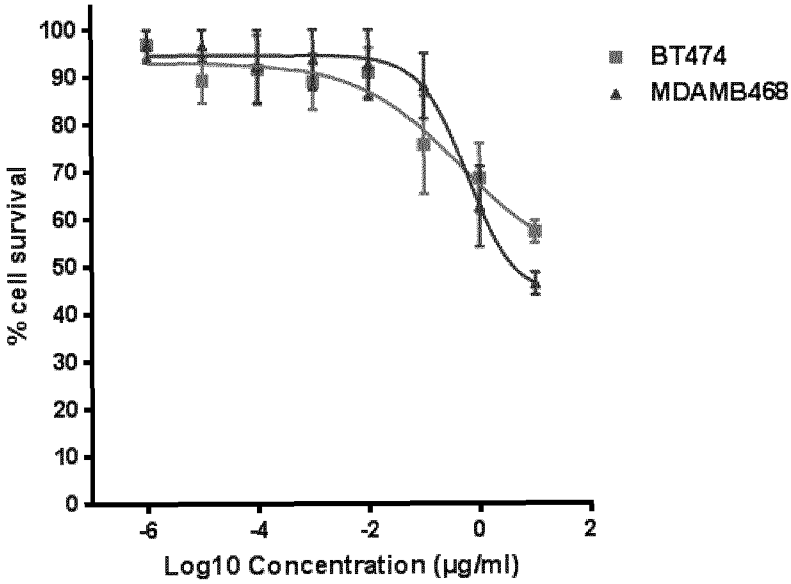


Figure 3

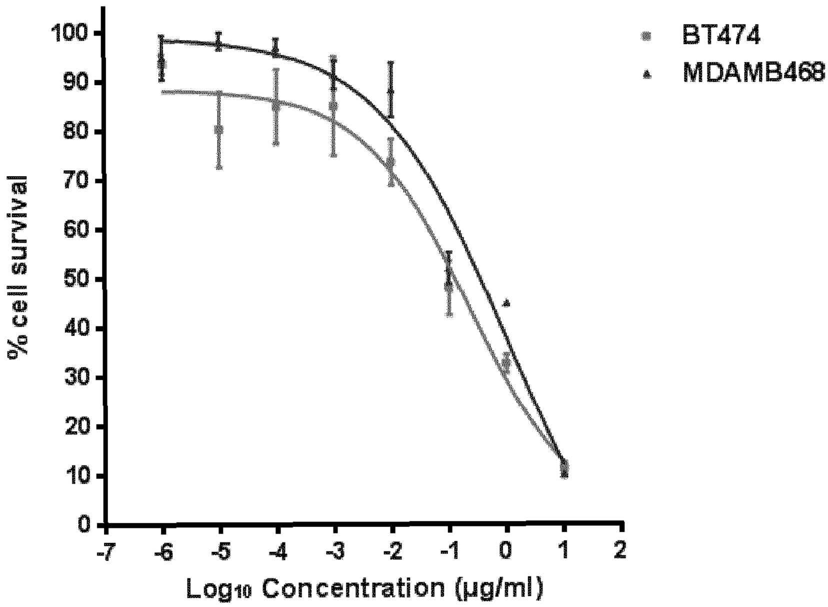


Figure 4

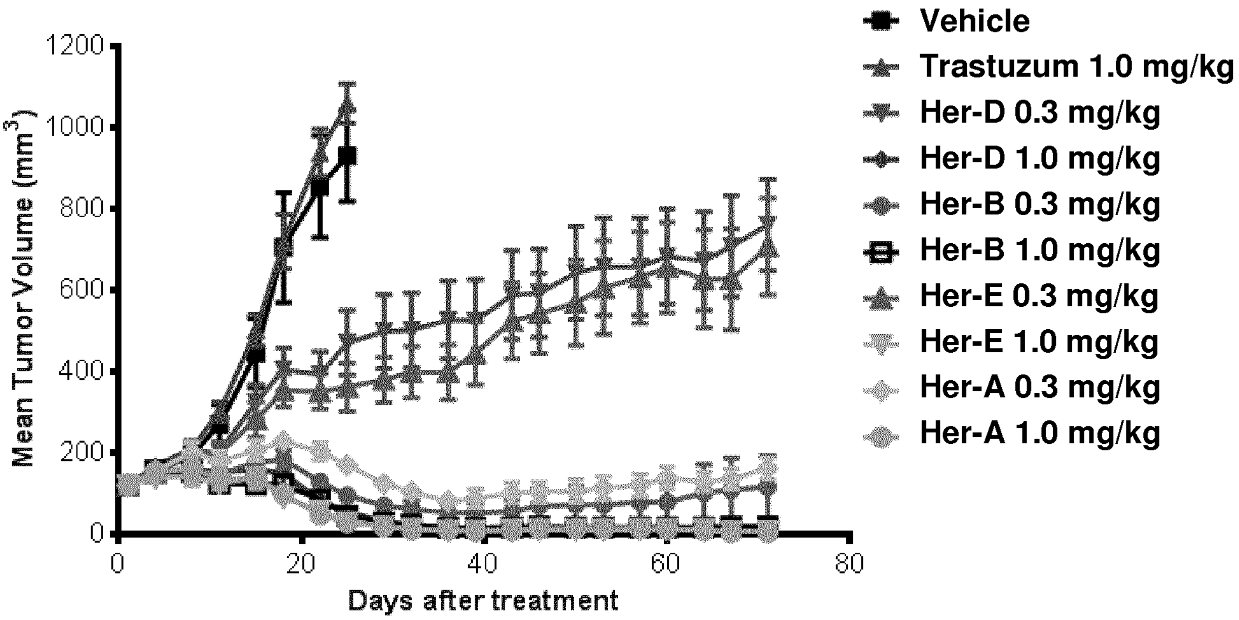
Mean Tumor Volume (mm³)
1200
1000
800
600
400
200
0
0
20
40
60
80
Days after treatment
Vehicle
Trastuzum 1.0 mg/kg
Her-D 0.3 mg/kg
Her-D 1.0 mg/kg
Her-B 0.3 mg/kg
Her-B 1.0 mg/kg
Her-E 0.3 mg/kg
Her-E 1.0 mg/kg
Her-A 0.3 mg/kg
Her-A 1.0 mg/kg

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 58180487 A
- WO 2007085930 A
- WO 2011130613 A
- WO 2011130616 A
- WO 2011130598 A
- US 7521541 B
- US 7723485 B
- WO 2009052249 A
- WO 2011130615 A
- WO 2010043380 A
- US 4816567 A
- US 20080206239 A1
- WO 2005079479 A2
- US 5583024 A
- US 5674713 A
- US 5700670 A
- US 6602677 B
- WO 2007044515 A


### Non-patent literature cited in the description


- **LEIMGRUBER et al.** *J. Am. Chem. Soc.,* 1965, vol. 87, 5793-5795
- **LEIMGRUBER et al.** *J. Am. Chem. Soc.,* 1965, vol. 87, 5791-5793
- **THURSTON et al.** *Chem. Rev.,* 1994, 433-465
- **ANTONOW, D. ; THURSTON, D.E.** *Chem. Rev.,* 2011, vol. 111 (4), 2815-2864
- **HOCHLOWSKI et al.** *J. Antibiotics,* 1987, vol. 40, 145-148
- **KONISHI et al.** *J. Antibiotics,* 1984, vol. 37, 200-206
- **THURSTON et al.** *Chem. Brit.,* 1990, vol. 26, 767-772
- **BOSE et al.** *Tetrahedron,* 1992, vol. 48, 751-758
- **KUMINOTO et al.** *J. Antibiotics,* 1980, vol. 33, 665-667
- **TAKEUCHI et al.** *J. Antibiotics,* 1976, vol. 29, 93-96
- **TSUNAKAWA et al.** *J. Antibiotics,* 1988, vol. 41, 1366-1373
- **SHIMIZU et al.** *J. Antibiotics,* 1982, vol. 29, 2492-2503
- **LANGLEY ; THURSTON.** *J. Org. Chem.,* 1987, vol. 52, 91-97
- **HARA et al.** *J. Antibiotics,* 1988, vol. 41, 702-704
- **ITOH et al.** *J. Antibiotics,* 1988, vol. 41, 1281-1284
- **LEBER et al.** *J. Am. Chem. Soc.,* 1988, vol. 110, 2992-2993
- **ARIMA et al.** *J. Antibiotics,* 1972, vol. 25, 437-444
- **KOHN.** Antibiotics III. Springer-Verlag, 1975, 3-11
- **HURLEY ; NEEDHAM-VANDEVANTER.** *Acc. Chem. Res.,* 1986, vol. 19, 230-237
- **GREGSON et al.** *Chem. Commun.,* 1999, 797-798
- **GREGSON et al.** *J. Med. Chem.,* 2001, vol. 44, 1161-1174
- **CARTER, P.** *Nature Reviews Immunology,* 2006, vol. 6, 343-357
- **XIE et al.** *Expert. Opin. Biol. Ther.,* 2006, vol. 6 (3), 281-291
- **KOVTUN et al.** *Cancer Res.,* 2006, vol. 66 (6), 3214-3121
- **LAW et al.** *Cancer Res.,* 2006, vol. 66 (4), 2328-2337
- **WU et al.** *Nature Biotech.,* 2005, vol. 23 (9), 1137-1145
- **LAMBERT J.** *Current Opin. in Pharmacol.,* 2005, vol. 5, 543-549
- **HAMANN P.** *Expert Opin. Ther. Patents,* 2005, vol. 15 (9), 1087-1103
- **PAYNE, G.** *Cancer Cell,* 2003, vol. 3, 207-212
- **TRAIL et al.** *Cancer Immunol. Immunother.,* 2003, vol. 52, 328-337
- **SYRIGOS ; EPENETOS.** *Anticancer Research,* 1999, vol. 19, 605-614
- **JUNUTULA et al.** *Nature Biotech.,* 2008, vol. 26 (8), 925-932
- **DORNAN et al.** *Blood,* 2009, vol. 114 (13), 2721-2729
- **MCDONAGH.** *Protein Eng. Design & Sel.,* 2006, vol. 19 (7), 299-307
- **DORONINA et al.** *Bioconj. Chem.,* 2006, vol. 17, 114-124
- **ERICKSON et al.** *Cancer Res.,* 2006, vol. 66 (8), 1-8

- **SANDERSON et al.** *Clin. Cancer Res.,* 2005, vol. 11, 843-852
- **JEFFREY et al.** *J. Med. Chem.,* 2005, vol. 48, 1344-1358
- **HAMBLETT et al.** *Cancer Res.,* 2004, vol. 10, 7063-7070
- **MILLER et al.** *Jour. of Immunology,* 2003, vol. 170, 4854-4861
- **JANEWAY, C. ; TRAVERS, P. ; WALPORT, M. ; SHLOMCHIK.** Immuno Biology. Garland Publishing, 2001
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **LONBERG.** *Curr. Opinion,* 2008, vol. 20 (4), 450-459
- **MORRISON et al.** *Proc. Natl. Acad. Sci. USA,* 1984, vol. 81, 6851-6855
- *Nature Biotechnology,* 1994, vol. 12, 899-903
- **ALTUVIA et al.** *J. Mol. Biol.,* 1995, vol. 249, 244-250
- *Molecular Immunology,* 2007, vol. 44, 1986-1998
- *Methods,* 2005, vol. 36, 43-60
- **HAMBLETT et al.** *Clin. Cancer Res.,* 2004, vol. 10, 7063-7070
- **AXUP et al.** *Proc Natl Acad Sci USA.,* 2012, vol. 109 (40), 16101-16116
- **BERGE et al.** *J. Pharm. Sci.,* 1977, vol. 66, 1-19
- McGraw-Hill Dictionary of Chemical Terms. McGraw-Hill Book Company, 1984
- **ELIEL, E. ; WILEN, S.** Stereochemistry of Organic Compounds. John Wiley & Sons, Inc, 1994
- **CROUCH et al.** *J. Immunol.,* 1993, vol. 160, 81-88
- **CREE et al.** *AntiCancer Drugs,* 1995, vol. 6, 398-404
- *Angew Chem. Intl. Ed. Engl.,* 1994, vol. 33, 183-186
- Handbook of Pharmaceutical Additives. Synapse Information Resources, Inc, 2001
- Remington's Pharmaceutical Sciences. Lippincott, Williams & Wilkins, 2000
- Handbook of Pharmaceutical Excipients. 1994
- **GETZ et al.** *Anal. Biochem.,* 1999, vol. 273, 73-80
- **SMITH, P.K. et al.** *Anal. Biochem.,* 1985, vol. 150 (1), 76-85